# EUROPEAN PATENT APPLICATION

(11) **EP 4 600 349 A1**
(43) Date of publication of application: **13.08.2025**
(21) Application number: 23868583.8
(22) Date of filing: 20.09.2023
(51) Int. Cl.: C12N 5/077, C12N 5/071, A61K 35/28, A61K 35/38, A61P 1/04, A61K 47/42, A61K 47/34

(54) **STROMAL CELL LAYER AROUND INTESTINAL ORGANOID FOR ENHANCING ENGRAFTMENT AND REGENERATION EFFICACY, AND USE THEREOF**

(30) Priority: 21.09.2022 KR 20220119252
(71) Applicant: Korea Research Institute of Bioscience and Biotechnology, Daejeon 34141 (KR)
(72) Inventor: SON, Mi-Young, Daejeon 34141 (KR); LEE, Hana, Daejeon 34141 (KR); HAN, Tae-Su, Daejeon 34141 (KR)
(74) Representative: Cabinet Becker et Associés
(86) International application number: PCT/KR2023/014264
(87) International publication number: WO 2024/063531

(57) **Abstract**

The present disclosure relates to mature intestinal organoid-derived mesenchymal stromal cells, and a use thereof. The mature intestinal organoid-derived mesenchymal stromal cells according to the present disclosure can act directly on a lesion site to significantly enhance regeneration and engraftment efficacy, and thus can significantly enhance the efficiency of transplantation. Further, the mature intestinal organoid-derived mesenchymal stromal cells according to the present disclosure have excellent efficacy due to being capable of regenerating damaged regions immediately and directly, unlike conventional mesenchymal stem cell therapeutic agents, allow for the application of a simplified subculture method, can maintain the characteristics thereof even when frozen and thawed, and can be utilized in allogeneic transplantation, thus having the advantage of a high likelihood of universal applicability.

## Description

### [Technical Field]

The present disclosure relates to a stromal cell layer around an intestinal organoid for enhancing engraftment and regeneration efficacy, a mesenchymal stromal cell around an intestinal organoid, a method for producing the same, and use thereof.

### [Background Art]

Stromal cells are connective tissue cells that support the function of the parenchymal cells forming a particular organ or tissue. The stromal cells can be composed of cells with different characteristics, forming a heterogeneous cell population and differentiating in different ways depending on their environment.

Recently, a number of transplantation therapies have been developed using tissue engineering methods, including mesenchymal stromal cells. However, a major limitation of these transplantation techniques is the difficulty in developing stable cell therapeutic agents that are both therapeutically effective and do not cause an immune response in the human body. Several attempts have been made to utilize various mesenchymal stromal cells as cell therapeutic agents. However, development has been largely limited to the use of autologous tissues, and progress remains very slow in the development of universally applicable cell sources.

In addition, even aside from the difficulty in securing universally usable cell sources, the progress of stromal cell-related research requires a smooth supply of stromal cells, but there are major limitations in obtaining cells for use. For example, invasive methods must be used to obtain mesenchymal stromal cells derived from umbilical cord blood or adipose tissue. The most non-invasive cells to obtain are mesenchymal stromal cells, which can be obtained through bone marrow harvest, but these cells require anesthesia and cause pain, which limits their use. In addition, methods for obtaining cells using peripheral blood are required, but the number of mesenchymal stem cells capable of being isolated from adults using only peripheral blood is too small, the isolation method is not cost-effective, and even when cells are successfully isolated, most do not proliferate in quantities sufficient for cell therapy. Therefore, an alternative method that enhances practicality is needed.

Further, adult-derived mesenchymal stromal cells, etc., are often very limited in their utilization since their cell function decreases significantly with each passage, and their secretion of various factors and stem cell migration abilities are reduced. Furthermore, the utility of randomly adult-derived mesenchymal stromal cells is often very low due to significant variations in their properties depending on their origin.

Against this backdrop, there is an urgent need to develop novel stromal cells capable of exhibiting improved properties through established differentiation protocols and maintaining their properties in the long term, a method for producing the same, and use thereof.

The present inventors confirmed that intestinal organoids differentiated from pluripotent stem cells, particularly intestinal organoids matured *in vitro,* have a stable structure more similar to the human intestine due to a cell layer composed of stromal cells in the tissue around the intestinal organoid, similar to the human intestinal microenvironment, unlike adult stem cell-derived intestinal organoids or patient (tissue)-derived intestinal organoids. Against this backdrop, the present disclosure was completed by obtaining a cell layer composed of stromal cells in the tissue around the intestinal organoid and confirming that this stromal cell layer has an excellent effect on enhancing engraftment and regeneration efficacy, unlike previously known stromal cells.

### [Disclosure]

### [Technical Problem]

An object of the present disclosure is to provide a stromal cell layer around an intestinal organoid differentiated from a pluripotent stem cell (PSC).

Another object of the present disclosure is to provide a mesenchymal stromal cell derived from an intestinal organoid differentiated from a pluripotent stem cell (PSC).

Another object of the present disclosure is to provide a pharmaceutical composition comprising: the mesenchymal stromal cell derived from the intestinal organoid differentiated from the pluripotent stem cell (PSC).

Another object of the present disclosure is to provide a pharmaceutical composition for assisting transplantation, comprising the mesenchymal stromal cell derived from the intestinal organoid differentiated from the pluripotent stem cell (PSC).

Still another object of the present disclosure is to provide a pharmaceutical composition for preventing or treating an intestinal disease, comprising: the mesenchymal stromal cell derived from the intestinal organoid differentiated from the pluripotent stem cell (PSC).

### [Technical Solution]

Various modifications can be made and various embodiments may be implemented in the present disclosure, and specific embodiments are illustrated in the drawings and described in detail. However, it should be understood that this is not intended to limit the present disclosure to specific embodiments, and comprises all modifications, equivalents, and substitutes included in the spirit and scope of the present disclosure.

Terms used in the present application are only used to describe specific embodiments and are not intended to limit the present disclosure. Singular expressions include plural expressions unless the context clearly indicates otherwise. In the present specification, terms such as "comprise" or "have" are intended to designate the presence of features, numbers, steps, operations, components, parts, or combinations thereof described in the specification and it should not be understood as precluding the possibility of the presence or addition of one or more other features, numbers, steps, operations, components, parts, or combinations thereof.

When amounts, concentrations, or other values or parameters herein are given as ranges, preferred ranges, or lists of upper desirable values and lower desirable values, it should be understood as specifically disclosing all ranges formed by any pair of any upper range limit or preferred value and any lower range limit or preferred value, regardless of whether the scope is separately disclosed.

When ranges of numerical values are stated herein, unless otherwise stated, it is intended that the endpoints of the range and the scope of the parent invention within the range are not limited to the specific values stated when defining the range.

### Pluripotent stem cell

As used herein, the term "pluripotent stem cell (PSC)" is commonly known as a PS cell, and includes any cell that can be differentiated into almost all cells, i.e., a cell derived from any of three germ layers (reproductive epithelium) including endoderm (internal gastric wall, gastrointestinal tract, lung), mesoderm (muscle, bone, blood, genitourinary), and ectoderm (epithelial tissue and nervous system). The PSC may be derived from embryonic stem cells (including embryonic germ cells) or may be a descendant of pluripotent cells obtained by inducing non-pluripotent cells, such as adult somatic cells, by forcing expression of specific genes.

As used herein, the term "induced pluripotent stem cell (iPSC)" is commonly abbreviated as an iPS cell, and refers to a type of pluripotent stem cells that are artificially induced from normally non-pluripotent cells, such as adult somatic cells, by inducing "forced" expression of specific genes.

As used herein, the term "embryonic stem cell (ESC)" is commonly abbreviated as an ES cell, and refers to a cell derived from the inner cell mass of blastocyst, which is a pluripotent early-stage embryo. For purposes of the present disclosure, the term "ESC" is sometimes used broadly, including embryonic germ cells.

### Basal medium

A differentiation medium used in the method of the present disclosure comprises a basal medium. The basal medium is any basal medium suitable for animal or human cells, subject to the limitations provided herein.

The basal medium for animal or human cell culture typically contains a number of components necessary to support maintenance of cultured cells. Combinations of suitable components may be easily formulated by a skilled person in consideration of the following contents. The basal medium for use in the present disclosure will generally include a nutrient solution containing standard cell culture components as described in more detail above and in the document, for example, amino acids, vitamins, lipid supplements, mineral salts, carbon energy sources, and buffers. In some embodiments, the culture medium is further supplemented with one or more standard cell culture components selected from, for example, amino acids, vitamins, liquid supplements, inorganic salts, carbon energy sources, and buffers.

The skilled person will understand, from the general knowledge of the art, the type of culture medium that may be used as the basal medium in the differentiation medium of the present disclosure. The potentially suitable cell culture medium is commercially available, and include, but is not limited to, Dulbecco's modified eagle medium (DMEM), minimum essential medium (MEM), knockout-DMEM (KO-DMEM), Glasgow's minimum essential medium (G-MEM), a basal medium eagle (BME), DMEM/Ham's F12, Advanced DMEM/Ham's F12, Iscove's modified dulbecco's medium, minimum essential medium (MEM), Ham's F-10, Ham's F-12, Medium 199, and RPMI 1640 medium.

For example, the basal medium may be DMEM/F12 and/or RPMI 1640.

If needed, Advanced DMEM/F12 or Advanced RPMI that is optimized for serum-free culture and already contains insulin is used.

### Differentiation into intestinal organoid

The differentiation into intestinal organoid from pluripotent stem cells may include 1) a differentiation process from pluripotent stem cells into definitive endoderm (DE) cells, 2) a differentiation process from definitive endoderm (DE) cells into hindgut (HG), and 3) a differentiation process from hindgut (HG) into (mature) intestinal organoids.

### 1) Differentiation from pluripotent stem cells into definitive endoderm (DE) cells

One or more growth factors are used in a differentiation process from pluripotent stem cells into definitive endoderm (DE) cells.

Specifically, growth factors that may affect Activin A/nodal signaling activation or phosphatidylinositol 3-kinase (PI3K) signaling inhibition may be used.

For example, one or more growth factors used in the differentiation process may include a growth factor in TGF-β superfamily. One or more growth factors may include Nodal/Activin and/or BMP subgroup of growth factors in TGF-β superfamily.

In some aspects, one or more growth factors are selected from the group consisting of Nodal, Activin A, Activin B, BMP4, Wnt3a, bFGF or any combination of these growth factors. In some aspects, the pluripotent stem cells are treated with one or more growth factors for at least 6 hours; at least 12 hours; at least 18 hours; at least 24 hours; at least 36 hours; at least 48 hours; at least 60 hours; at least 72 hours; at least 84 hours; at least 96 hours; at least 120 hours; at least 150 hours; at least 180 hours; or at least 240 hours. In some aspects, the embryonic stem cells or iPSCs are treated with one or more growth factors at a concentration of 10 ng/ml or more; 20 ng/ml or more; 50 ng/ml or more; 75 ng/ml or more; 100 ng/ml or more; 120 ng/ml or more; 150 ng/ml or more; 200 ng/ml or more; 500 ng/ml or more; 1,000 ng/ml or more; 1,200 ng/ml or more; 1,500 ng/ml or more; 2,000 ng/ml or more; 5,000 ng/ml or more; 7,000 ng/ml or more; 10,000 ng/ml or more; or 15,000 ng/ml or more. In some aspects, the concentration of the growth factor is maintained at a constant level during the treatment process. The concentration of the growth factor may be varied during the treatment process.

That is, the method may include a step of treating pluripotent stem cells with any one or more selected from the group consisting of Nodal, Activin A, Activin B, BMP4, bFGF, and Wnt3a to perform differentiation into definitive endoderm cells. More specifically, the method may include a step of treating embryonic stem cells or induced pluripotent stem cells with Activin A to perform differentiation into definitive endoderm cells.

According to one embodiment of the present disclosure, Activin A may be used to differentiate pluripotent stem cells into definitive endoderm (DE) cells. In the differentiation, for example, 10 ng/ml to 500 ng/ml of Activin A may be used, but the present disclosure is not limited thereto. More specifically, 10, 20, 30, 40, 50, 60, 70 80, 90, 100, 200, 300, 400, or 500 ng/ml of Activin A may be used. More specifically, the culturing may be performed for 6 hours to 120 hours, or 12 hours to 100 hours, but the present disclosure is not limited thereto.

According to an embodiment of the present disclosure, the growth factor is suspended in a medium containing fetal bovine serum (FBS) with varying concentrations, as needed. For example, the medium may contain any fetal bovine serum (FBS or FCS) suitable for growth, such as 0.1%, 0.2%, 0.3%, 0.5%, 1.0%, 2.0%, 3.0%, 4.0%, or 5.0%, as well as 1x B-27^{™} Supplement, serum free.

If necessary, the culturing may be performed on micropatterned substrates/plates (e.g., Aggrewell, EZSPHERE) to produce aggregates (e.g., spheroids) and may be performed in contact with the extracellular matrix (ECM). The extracellular matrix may be applied to the present differentiation method, including all of the matters described herein.

### 2) Differentiation from definitive endoderm (DE) cells to hindgut (HG)

Definitive endoderm (DE) induced by activin, preferably activin A, may be subject to a differentiation step into hindgut (HG) by FGF/Wnt signaling.

FGF and Wnt ligands may induce differentiation from DE developed in iPSC or ESC, into the hindgut through direct differentiation.

The differentiation into Hindgut or Mid-hindgut may be performed on a micropatterned substrate/plate (e.g., Aggrewell, EZSPHERE) and formed into an aggregation form or may be performed through three-dimensional culture (e.g., suspension) within a bioreactor. As for serum-free differentiation, B-27 may be added instead of fetal bovine serum (FBS or FCS), and Activin A, FGF, bFGF, BMP-4, LY294002(PI3K inhibitor), CHIR99021(GSK3 inhibitor), and Retinoic acid may be additionally added.

Altering the expression of any Wnt signaling protein in combination with any FGF ligand may result in direct differentiation as described herein.

Modulators/activators of the Wnt signaling pathway include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. In some aspects, the pathway may be modulated through the use of a small molecule modulator or a protein modulator that activates the above-described pathway or a protein that activates the pathway. Examples of the small molecule modulator of the Wnt pathway include, but are not limited to, lithium chloride; 2-amino-4,6-disubstituted pyrimidine(hetero)arylpyrimidine; IQ1; QS11; NSC668036; DCA-beta catenin; and 2-amino-4-[3,4-(methylenedioxy)-benzyl-amino]-6-(3-methoxyphenyl) pyrimidine. Exemplary natural inhibitors of Wnt signaling include, but are not limited to, Dkk1, SFRP protein, and FrzB. In some aspects, exogenous molecules include, but are not limited to, small molecules such as WAY-316606; SB-216763; or BIO (6-bromoindirubin-3'-oxime).

In addition, the exogenous molecules include GSK3-inhibiting molecules or proteins that activate the Wnt signaling pathway.

Exemplary GSK3 inhibitors include, but are not limited to, LY2090314, BIO(6-bromoindirubin-3'-oxime), SB216763 (3-(2,4-dichlorophenyl)-4-(1-methyl-1H-indol-3-yl)-1H-pyrrole-2,5-dione), GSK-3β inhibitor VII (α,4-dibromoacetophenone), L803-mts (Myr-N-GKEAPPAPPQSpP-NH₂), and CHIR99021.

Specifically, the GSK3 inhibitors include Chiron/CHIR99021 that inhibits GSK3. The GSK3 inhibitor may be treated in an amount of about 0.1 uM to about 100 uM, or about 0.2 uM to about 50 uM, or about 0.3 uM to about 10 uM.

A Fibroblast growth factor (FGF) is a group of growth factors associated with vascularization, wound healing, and embryo development. In some aspects, it will be understood by one of ordinary skill in the art that any FGF may be used together with a protein derived from the Wnt signal pathway.

In some embodiments, the FGF signaling pathway is activated in contact with cells with at least one molecule selected from the group consisting of FGF1, FGF2, FGF3, FGF4, FGF10, FGF11, FGF12, FGF13, FGF14, FGF15, FGF16, FGF17, FGF18, FGF19, FGF20, FGF21, FGF22, and FGF23.

In some aspects, the DE cells are treated with one or more modulators of the signaling pathways described herein for at least 6 hours; at least 12 hours; at least 18 hours; at least 24 hours; at least 36 hours; at least 48 hours; at least 60 hours; at least 72 hours; at least 84 hours; at least 96 hours; at least 120 hours; at least 150 hours; at least 180 hours; at least 200 hours; at least 240 hours; at least 270 hours; at least 300 hours; at least 350 hours; at least 400 hours; at least 500 hours; at least 600 hours; at least 700 hours; at least 800 hours; at least 900 hours; at least 1,000 hours; at least 1,200 hours; or at least 1,500 hours.

In some aspects, the DE cells are treated with one or more molecules of the FGF signaling pathway described herein at a concentration of 10 ng/ml or more; 20 ng/ml or more; 50 ng/ml or more; 75 ng/ml or more; 100 ng/ml or more; 120 ng/ml or more; 150 ng/ml or more; 200 ng/ml or more; 500 ng/ml or more; 1,000 ng/ml or more; 1,200 ng/ml or more; 1,500 ng/ml or more; 2,000 ng/ml or more; 5,000 ng/ml or more; 7,000 ng/ml or more; 10,000 ng/ml or more; or 15,000 ng/ml or more.

In some aspects, the concentration of signaling molecules remains constant during the course of treatment. In another aspect, the concentration of molecules in the signaling pathway is varied during the course of the treatment.

In some aspects, the signaling molecules according to the invention are suspended in a DMEM medium.

In some aspects, the signaling molecules according to the invention are suspended in a medium containing fetal bovine serum (fetal bovine serum, FBS or FCS).

Any known molecules of the signal pathway described herein, including any molecules of the Wnt and FGF signaling pathways, are applicable alone or in combination.

That is, the method includes a step of differentiating the definitive endoderm cells into hindgut by treatment with FGF and Wnt ligands. More specifically, the method includes a step of differentiating the definitive endoderm cells into hindgut by treatment with FGF and CHIR99021.

According to an embodiment of the present disclosure, the differentiation from the definitive endoderm (DE) into the hindgut may be performed through the GSK3 inhibitor and the fibroblast growth factor. More specifically, the differentiation into the hindgut may be performed through three-dimensional culture by treatment with CHIR99021 and FGF4.

In the differentiation, for example, 100 ng/ml, 120 ng/ml, 150 ng/ml, 200 ng/ml, 500 ng/ml, 1,000 ng/ml; or 1,200 ng/ml of FGF4 may be used, but the present disclosure is not limited thereto. In addition, CHIR99021 may be used in an amount of about 0.3 uM to about 10 uM. As needed, it is suspended in a medium containing fetal bovine serum (FBS or FCS). For example, the medium may contain any fetal bovine serum (FBS or FCS) suitable for growth, such as 0.1, 0.2, 0.3, 0.5, 1.0, 2.0, 3.0, 4.0, or 5.0%.

More specifically, the medium may be cultured for 24 hours or longer; 36 hours or longer; 48 hours or longer; 60 hours or longer; 72 hours or longer; 84 hours or longer; 96 hours or longer; 120 hours or longer; 150 hours or longer; 180 hours or longer, but the present disclosure is not limited thereto.

### 3-1) Differentiation from hindgut (HG) into intestinal organoid

Differentiation from hindgut (HG) to intestinal organoid essentially includes BMP inhibitors, Wnt agonists, and receptor tyrosine kinase ligands. In addition, one or more additional components selected from the group consisting of ROCK inhibitors, B27, N-acetylcysteine, N2, nicotinamide, Gastrin, IGF-1, heregulin-1β, bFGF, A-83-01, and/or SB202190 may be included.

The BMP inhibitor is an agent that binds to a BMP molecule to form a complex. The inhibitor may be an agent that binds to the BMP receptor and prevents binding of the BMP ligand to the receptor, such as an antibody that binds to the receptor. The BMP inhibitor may be a protein or a small molecule and may be natural, modified, and/or partially or entirely synthetic. The BMP inhibitor may be Noggin, DAN, or a DAN-like protein including Cerberus and Gremlin (R&D systems). A preferred BMP inhibitor is Noggin. Noggin may be used at any suitable concentration. The culture medium may include about 10 ng/ml to about 100 ng/ml of Noggin. For example, the culture medium may include about 10 ng/ml, 20 ng/ml 30 ng/ml, 40 ng/ml, or 50 ng/ml or more of Noggin. Preferably, the culture medium may include approximately 10 to 100 ng/ml of Noggin.

The Wnt agonist may preferably be R-spondin 1, R-spondin 2, R-spondin 3 or R-spondin 4. The culture medium may include the Wnt agonist at a concentration of 50 ng/ml, 100 ng/ml, 200 ng/ml, 300 ng/ml, 500 ng/ml, 600 ng/ml, 700 ng/ml, 800 ng/ml, 900 ng/ml, 1 ug/ml, 1.5 ug/ml, or 2 ug/ml or more. Preferably, the culture medium may contain approximately 50 to 800 ng/ml of R-spondin 1.

The receptor tyrosine kinase ligand is, for example, a mitogenic growth factor selected from the group consisting of an epidermal growth factor (EGF), a transforming growth factor-alpha (TGF-alpha), a basal fibroblast growth factor (bFGF), a brain-derived neurotrophic factor (BDNF), neuregulin (NRG 1-4), a hepatocyte growth factor (HGF), and keratinocyte growth factor (KGF).

Preferably, the receptor tyrosine kinase ligand is the EGF. The EGF is a potent mitogenic factor for a variety of cultured ectodermal and mesodermal cells and has sufficient effects on differentiation of certain cells in vivo and in vitro and some fibroblasts in cell cultures. Preferred concentrations thereof are at least 10 ng/ml, 20 ng/ml, 25 ng/ml, 30 ng/ml, 40 ng/ml, 45 ng/ml, or 50 ng/ml, 100 ng/ml, 200 ng/ml, 300 ng/ml, 500 ng/ml, 600 ng/ml, and 700 ng/ml. More preferred concentrations are more than or equal to 50 ng/ml and less than or equal to 300 ng/ml.

In addition to the essential composition of the BMP inhibitor, the WNT agonist, and the receptor tyrosine kinase ligand as described above, additional factors may be interpreted as further including addition of any known factor of the intestinal organoid. Preferably, depending on the purpose of differentiation, one or more additional components selected from ROCK inhibitors, B27, N-acetylcysteine, N2, nicotinamide, Gastrin, IGF-1, heregulin-1β, bFGF, A-83-01, and/or SB202190 may be further included.

The ROCK inhibitor is preferably selected from R-(+)-trans-4-(1-aminoethyl)-N-(4-pyridyl)cyclohexanecarboxamide dihydrochloride monohydrate (Y-27632, Sigma-Aldrich), 5-(1,4-diazepan-1-ylsulfonyl)isoquinoline (fasudil or HA1077, Cayman Chemical), and (S)-(+)-2-methyl 1-[(4-methyl-5-isoquinolinyl)sulfonyl]-hexahydro-1H-1,4-diazepinedihydrochloride (H-1152, Tocris Bioscience). B27, N-acetylcysteine, N2, nicotinamide, Gastrin, IGF-1, heregulin-1β, bFGF, A-83-01, and/or SB202190 may be added as needed, for example, to improve the culture efficiency and lifespan of organoids, to modulate the proliferation of cells, to aid in the stability of DNA, etc.

Preferably, B27 may be further included. More specifically, the B27 supplement is referred to as "B27 supplement minus vitamin A" (also referred to herein as "B27 without vitamin A" or "B27 wo VitA"; and it may be obtained in Invitrogen (Located in Carlsbad, California, USA); www.invitrogen.com; current catalog number 12587010; PAA Laboratories GmbH (Located in Pasching, Austria); www.paa.com; catalog number F01-002; and document [Brewer et al. (1993) J Neurosci Res.35(5):567-76]). In some embodiments, the B27 supplement may be replaced with a generic formulation including one or more of the components selected from the following list: biotin, cholesterol, linoleic acid, linolenic acid, progesterone, putrescine, retinyl acetate, sodium selenite, tri-iodothyronine (T3), DL-alpha-tocopherol (vitamin E), albumin, insulin, and transferrin.

If necessary, the culturing may be performed in contact with the extracellular matrix (ECM) during differentiation from the Hindgut into the intestinal organoid.

In some embodiments, the extracellular matrix is a three-dimensional matrix. In some embodiments, the hindgut is embedded in the extracellular matrix. The culture medium of the present disclosure may diffuse into the three-dimensional extracellular matrix.

Matters regarding the ECM disclosed in documents [Polymer Hydrogels to Guide Organotypic and Organoid Cultures (Adv Funct Mater, 2020, Valentina Magno et al.) and Engineering the Extracellular Matrix for Organoid Culture (Int J Stem Cells. 2022 Feb 28; 15(1): 60-69)] are incorporated herein by reference.

The extracellular matrix includes fibrin, laminin, collagen, and/or alginate, but is not limited thereto. Examples of extracellular matrix-producing cells include chondrocytes, which mainly produce collagen and proteoglycans; fibroblasts, which mainly produce type IV collagen, laminin, interstitial procollagen, and fibronectin; and colon myofibroblasts, which mainly produce collagen (types I, III, and V), chondroitin sulfate proteoglycans, hyaluronic acid, fibronectin, and tenascin-C. These are "naturally produced extracellular matrices (ECM)". The naturally produced ECM may be commercially provided. Examples of commercially available extracellular matrices include extracellular matrix proteins (Invitrogen) and basement membrane preparations from Engelbreth-Holm-Swarm (EHS) murine sarcoma cells (e.g., Cultrex^{®} basement membrane extracts (Trevigen, Inc.), type I collagen (Invitrogen), VitroGel^{®} (TheWell Bioscience Inc.), Regenix^{®} (Cellartgen Inc.), Geltrex (ThermoFisher), or Matrigel^{®} (BD Biosciences)) .

### 3-2) Differentiation from Hindgut (HG) into mature intestinal organoid (mature hIO)

As used herein, the term "maturation" or "mature" intestinal organoid (mature hIO), as opposed to an immature intestinal organoid, refers to an intestinal organoid in which genes necessary for digestive functions, transport systems, immune functions, and host defense of the small intestine of an adult are expressed. Specifically, the mature small intestine has unique characteristics including enhancement of expression of genes necessary for small intestine stem cell marker genes, digestive functions, transport systems, a wide range of immune functions, and host defense. In particular, proper expression and activity of transporters involved in physiological and pharmacokinetic roles is a prerequisite for normal small intestinal functions, such as absorption, distribution and excretion of drugs. Particularly, the mature intestinal organoid according to the present disclosure has improved efficacy in terms of engraftment. More specifically, this may be due to high expression characteristics of vascular endothelium-related factors in the stromal cell layer present around the intestinal epithelial cell layer constituting the mature intestinal organoid, and various secretory factors that are characteristics of stromal cells.

In addition, the mature intestinal organoid may exhibit characteristics of budding structure development present on the intestinal organoid. Further, the organoid exhibits higher development of goblet cells, thereby exhibiting high efficiency in terms of differentiation ability of intestinal stem cells.

The differentiation from hindgut (HG) into mature intestinal organoid is used in co-culture to mimic the *in vivo* intestinal environment, or essentially involves treatment with cytokines secreted by T-lymphocyte co-culture, that is, treatment with cytokines secreted by T-lymphocyte, and/or treatment with STAT3 and mTOR signaling pathway activators, in addition to the above-described factors for differentiation into the intestinal organoid.

More specifically, the cytokine secreted by T-lymphocyte may be one or more selected from the group consisting of IL-2 (interleukin-2), IL-22 (interleukin-22), IL-6 (interleukin-6), IL-1β (interleukin-1β), IL-11 (interleukin-11), EGF (epidermal growth factor), OSM (oncostatin M), and IL-10 (interleukin-10), and more specifically, may be IL-2.

Further, the differentiation from hindgut to mature intestinal organoid may be performed to enable maturation of intestinal organoid by treatment with STAT3 and mTOR signaling pathway activators, in addition to the above-described factors for differentiation into the intestinal organoid. For example, the maturation of intestinal organoid may be performed by treatment with colivelin.

In addition, the maturation of intestinal organoid may be performed by treatment with NRG-1 (Neuregulin-1).

In other words, any one or more cytokines selected from the group consisting of IL-2, IL-22, IL-6, IL-1β, IL-11, EGF, OSM, NRG-1, and IL-10, NRG-1, and/or colivelin may be included as a medium component.

As used herein, the term immature intestinal organoid refers to an intestinal organoid without the use in co-culture to mimic the in vivo intestinal environment or without the treatment with cytokines secreted by T-lymphocyte co-culture, or without the treatment with STAT3 and mTOR signaling pathway activators. Preferred concentrations thereof are at least 0.1, 0.2, 0.3, 0.4, 0.5, 0.6, 0.7, 0.8, 0.9, 1.0, 2.0, and 3.0 ng/ml. More preferably, the concentration may be 0.3 to 2.0 ng/ml.

In other words, the present disclosure may include a step of maturing the intestinal organoid by treating the hindgut with a BMP inhibitor, a Wnt agonist, a receptor tyrosine kinase ligand, and cytokine secreted by T-lymphocyte. More specifically, the present disclosure may include a step of maturing the intestinal organoid by treating the hindgut with Noggin as a BMP inhibitor;
at least any one Wnt agonist selected from the group consisting of R-spondin 1, R-spondin 2, R-spondin 3, and R-spondin 4;
an epidermal growth factor (EGF) as a receptor tyrosine kinase ligand; and
at least any one cytokine secreted by T lymphocytes, selected from the group consisting of IL-2, IL-22, IL-6, IL-1β, IL-11, EGF, OSM, and IL-10, NRG-1 and/or colivelin.

According to an embodiment of the present disclosure, the differentiation from hindgut into mature intestinal organoid may essentially include Noggin, EGF and R-spondin 1, and the culturing may be performed by adding cytokine and/or colivelin, for differentiation into the mature intestinal organoid. The addition of these cytokines and/or colivelin may be achieved within 24 hours, within 20 hours, within 16 hours, within 12 hours, within 8 hours, within 4 hours, within 2 hours, within 1 hour, or simultaneously, at the time of medium exchange in the culture stage of intestinal organoid after culturing the hindgut. The rapid addition of cytokines, as described above, may have greater benefits for maturation of intestinal organoids, particularly the enhancement of vascular endothelial factor expression to promote engraftment.

Further, one or more additional components selected from the group consisting of ROCK inhibitors, B27, N-acetylcysteine, N2, nicotinamide, and Gastrin as described above, may be further included.

### Mature human intestinal organoid, mature hIO; Mat-hIO

As used herein, the term "maturation" or "mature" intestinal organoid (mature hIO) is an intestinal organoid in which genes necessary for digestive functions, transport systems, immune functions, and host defense of the small intestine of an adult are expressed. Specifically, the mature small intestine has unique characteristics including small intestine stem cell marker genes, digestive functions, transport systems, a wide range of immune functions, and enhancement of expression of genes necessary for host defense. In particular, proper expression and activity of transporters involved in physiological and pharmacokinetic roles is a prerequisite for normal small intestinal functions, such as absorption, distribution and excretion of drugs.

The mature intestinal organoid according to the present disclosure is produced from an intestinal organoid derived from a pluripotent stem cell (also referred to as pluripotent stem cell-derived intestinal organoid).

The pluripotent stem cell-derived intestinal organoid according to the present disclosure is characterized by having a stromal cell layer around the intestinal organoid epithelial layer. Therefore, the intestinal organoid has high expression levels of markers of mesenchymal stromal cells, including but not limited to αSMA, VIM, DESMIN, etc. The αSMA is able to function not only as a marker of mesenchymal stromal cells but also as a marker of pericytes.

Conventional adult stem cell-derived intestinal organoids or patient (tissue)-derived intestinal organoids do not have a peristromal cell layer when inducing differentiation into intestinal organoids. On the other hand, the pluripotent stem cell-derived intestinal organoid according to the present disclosure exhibits properties that differ from previously known adult-derived intestinal organoids by having the stromal cell layer around the intestinal organoid.

The mature intestinal organoid differentiated by treatment with cytokines or colivelin for maturation according to the present disclosure has improved efficacy in terms of engraftment compared to immature intestinal organoids derived from non-mature pluripotent stem cells. More specifically, the stromal cell layer around the mature intestinal organoid may exhibit high expression of vascular endothelial-related factors.

More specifically, the mature intestinal organoid is a mature intestinal organoid comprising a peristromal cell layer (i.e., a stromal cell layer around an intestinal organoid), wherein the peristromal cell layer comprises intestine-derived mesenchymal stromal cells (MSC).

The mature intestinal organoid is derived from pluripotent stem cells (PSCs).

The mature intestinal organoid is characterized by higher expression of any one or more factors selected from the group consisting of VIL1, KRT20, CHGA, MUC13, LYZ, OLFM4, SI, LCT, and DPP4, compared to pluripotent stem cell-derived immature intestinal organoids. More specifically, the mature intestinal organoid may exhibit a 2-fold or greater difference in the level expression of genes selected from the group consisting of KRT20, CHGA, MUC13, LYZ, and OLFM4 compared to pluripotent stem cell-derived immature intestinal organoids.

The mature intestinal organoid according to the present disclosure may have good maintenance and differentiation of intestinal organoid through cell spheroid formation and low cell-matrix interaction, especially under xeno-free conditions, thereby providing an intestinal organoid suitable for clinical applications.

The mature intestinal organoid according to the present disclosure may exhibit high expression of vascular endothelial-related factors or vascular endothelial cell-related factors. For example, the mature intestinal organoid according to the present disclosure may have high expression levels of any one or more vascular endothelial cell-related factors selected from the group consisting of hVE-Cad, PECAM-1, and VEGF.

The mature intestinal organoid according to the present disclosure is characterized in that the expression of any one or more selected from the group consisting of vascular markers hCD31, ANGPT1 and KDR is higher than that of adult stem cell-derived organoids or pluripotent stem cell-derived immature intestinal organoids.

The mature intestinal organoid according to the present disclosure may have high expression levels of small intestine-specific markers. The small intestine-specific markers may include, but are not limited to, VIL1, KRT20, CHGA, LYZ, DEFA5, OLFM4, MUC2, MUC13, DPP4, LCT, SLC5A1, CREB3L3, etc.

The mature intestinal organoid according to the present disclosure comprises mesenchymal stromal cells in the stromal cell layer around the intestinal organoid, and particularly comprises a large number of cells exhibiting characteristics of vascular cells (hCD31⁺). In addition, compared to adult stem cell-derived organoids or pluripotent stem cell-derived immature intestinal organoids, the mature intestinal organoid according to the present disclosure has very high expression levels of groups related to vascular development, mesenchyme development, epithelial cell proliferation, and muscle tissue development.

In particular, it is characterized by a high expression level of hCD31⁺ together with high expression levels of markers related to mesenchymal stromal cells.

In addition, the mature intestinal organoid is characterized in that the stromal cell layer around the intestinal organoid and/or the mesenchymal stromal cells present therein may have endothelial cell type characteristics.

In addition, in the mature intestinal organoid, the stromal cell layer around the intestinal organoid and/or the mesenchymal stromal cells present therein may exhibit high expression levels of angiogenic factors, vascular endothelial-related factors, and/or vascular secretory factors, compared to adult stem cell-derived intestinal organoids or pluripotent stem cell-derived immature intestinal organoids.

More specifically, the angiogenic factor or vascular endothelial-related factor may be at least any one selected from the group consisting of CD31, CDH5, ECSCR, LYVE1, and IGF2. The vascular secretory factor may be at least any one selected from the group consisting of VEGF, Angiogenin, DKK1, EGF, Osteopontin, ICAM-1, VCAM-1, IGFBP3, MCP-1, and Activin A. The mature intestinal organoid according to the present disclosure may greatly assist in engraftment by comprising high levels of angiogenic factors, vascular endothelial-related factors, and/or vascular secretory factors.

As described above, the mature intestinal organoid having the stromal cell layer around the intestinal organoid may exhibit excellent therapeutic efficacy through high engraftment efficacy and excellent regeneration efficacy of intestinal stem cells within the organoid by transplanting the peripheral cell layer around the organoid together.

### Mature intestinal organoid-derived peristromal cell layer

Existing intestinal organoid technologies have not adequately addressed the necessity of the surrounding environment of intestinal organoids, interactions with stromal cells/immune cells, etc.

In particular, in adult stem cell-derived organoid technologies, where significant research and development are ongoing, the peristromal stromal cell layer is not generated or is insufficient in the environment around the organoid. In addition, even when utilizing pluripotent stem cells, techniques that aims to control this surrounding microenvironment, especially the peristromal cell layer have not been identified or reported.

The present disclosure may exhibit high engraftment ability by controlling the function and characteristics of the peristromal cell layer derived from the mature intestinal organoid through the production process of the above-described mature intestinal organoid.

The peristromal cell layer comprises the above-described mesenchymal stromal cells. Further, the peristromal cell layer, including the mesenchymal stromal cells, may have high expression levels of vascular endothelial- or vascular endothelial cell-related factors.

Further, the peristromal cell layer may further comprise at least any one cell selected from the group consisting of smooth muscle cells, fibroblasts, myofibroblasts, telocytes, and pericytes.

This peristromal cell layer may support the engraftment of intestinal organoid and angiogenesis, and exhibit rapid recovery and therapeutic efficacy through interactions with immune cells, stromal cells, etc.

### Mature intestinal organoid-derived mesenchymal stromal cells and isolation and culture thereof

The present disclosure provides mesenchymal stromal cells isolated (derived) from the mature intestinal organoid-derived peristromal cell layer and a method for isolating and/or culturing the same.

The mesenchymal stromal cells according to the present disclosure are mesenchymal stromal cells derived from the peristromal cell layer of mature intestinal organoids differentiated from pluripotent stem cells (PSCs).

A method for culturing mesenchymal stromal cells derived from the peristromal cell layer of mature intestinal organoids differentiated from pluripotent stem cells (PSCs) may be performed through the following steps:
(a) isolating a mature intestinal organoid into single cells or small cell clumps;
(b) seeding the isolated single cells or the small cell clumps in a culture medium coated with any one selected from the group consisting of gelatin, fibrin, and collagen I; and
(c) obtaining only cells that are adhered and cultured from the seeded cells.

In Step (a) above, the mature intestinal organoid refers to an intestinal organoid exhibiting the above-described mature characteristics. The intestinal organoid is isolated into single or small cell clumps by commonly known single cell isolation methods. According to an embodiment of the present disclosure, the mature intestinal organoid is isolated from an extracellular matrix, and cells are obtained from a cell layer existing around the intestinal organoid, followed by treatment with Trypsin-EDTA, etc., to isolate single cells.

In Step (b) above, the isolated single cells or small cell clumps may be seeded on a coating medium selected from the group consisting of gelatin, fibrin, and collagen I, and then cultured.

In Step (c) above, when single cells or small cell clumps are cultured in a coating medium selected from the group consisting of gelatin, fibrin, and collagen I, only cells exhibiting the characteristics of mesenchymal stromal cells are adhered to and cultured in the coating medium.

These adherent cultured cells exhibit characteristics as mesenchymal stromal cells, and the above-described properties of mature intestinal organoid-derived mesenchymal stromal cells are maintained and expressed. A commonly known culture medium may be used for culturing the mature intestinal organoid-derived mesenchymal stromal cells. Preferably, the culturing may be performed in a medium containing B27, EGF, IL-2, or any combination thereof.

In particular, the medium composition according to the present disclosure exhibits excellent effects on the growth and maintenance of mesenchymal stromal cells even under xeno-free conditions.

Further, the method for culturing mesenchymal stromal cells derived from the peristromal cell layer of the mature intestinal organoid differentiated from the pluripotent stem cell (PSC) may further comprise, after Step (c), (d) subculturing the adhered and cultured cells.

The above mature intestinal organoid may be produced, for example, through the following steps:
1) differentiating pluripotent stem cells into definitive endoderm cells by treatment with at least any one agent selected from the group consisting of Nodal, Activin A, Activin B, BMP4, and Wnt3a;
2) differentiating the definitive endoderm cells into a hindgut by treatment with FGF and Wnt ligands; and
3) maturing the intestinal organoid by treating the hindgut with a BMP inhibitor, a Wnt agonist, a receptor tyrosine kinase ligand, and cytokine secreted by T-lymphocyte.

More specifically, Step 1) above may include a step of differentiating embryonic stem cells or induced pluripotent stem cells into definitive endoderm cells by treatment with Activin A.

More specifically, Step 2) above may include a step of differentiating the definitive endoderm cells to the hindgut by treatment with FGF and CHIR99021.

More specifically, Step 3) above may include a step of maturing the intestinal organoid by treating the hindgut with Noggin as a BMP inhibitor, at least any one selected from the group consisting of R-spondin 1, R-spondin 2, R-spondin 3, and R-spondin 4 as Wnt agonists, an epidermal growth factor (EGF) as a receptor tyrosine kinase ligand, and at least any one cytokine selected from the group consisting of IL-2, IL-22, IL-6, IL-1β, IL-11, EGF, OSM, and IL-10 as cytokines secreted by T-lymphocytes.

More specifically, the cytokine secreted by T-lymphocyte may be IL-2.

The present disclosure also provides mesenchymal stromal cells derived from the stromal cell layer around the mature intestinal organoid, produced through the above culture process.

The present disclosure also provides mesenchymal stromal cells derived from the stromal cell layer around the mature intestinal organoid. More preferably, the present disclosure provides mesenchymal stromal cells derived from the stromal cell layer around the mature intestinal organoid, obtained through the above culture process.

The mesenchymal stromal cells derived from the stromal cell layer around the mature intestinal organoid exhibit at least one or more characteristics selected from the following:
(a) an enhanced expression level of hCD31 (PECAM1) compared to adult-derived mesenchymal stem cells, pluripotent stem cell-derived mesenchymal stem cells and/or immature intestinal organoid-derived mesenchymal stromal cells;
(b) enhanced expression levels of factors related to vascular development, mesenchyme development, epithelial cell proliferation, and/or muscle tissue development, compared to adult-derived mesenchymal stem cells, pluripotent stem cell-derived mesenchymal stem cells, and/or immature intestinal organoid-derived mesenchymal stromal cells;
(c) enhanced expression levels of endothelial cell types, compared to adult-derived mesenchymal stem cells, pluripotent stem cell-derived mesenchymal stem cells, and/or immature intestinal organoid-derived mesenchymal stromal cells;
(d) enhanced expression levels of endothelial cell characteristic factors in gene ontology (GO) term enrichment, compared to adult-derived mesenchymal stem cells, pluripotent stem cell-derived mesenchymal stem cells, and/or immature intestinal organoid-derived mesenchymal stromal cells;
(e) enhanced expression levels of angiogenic factors, vascular endothelial-related factors and/or vascular secretory factors, compared to adult-derived mesenchymal stem cells, pluripotent stem cell-derived mesenchymal stem cells, and/or immature intestinal organoid-derived mesenchymal stromal cells; and
   wherein the angiogenic factor and/or vascular endothelial-related factor is any one or more selected from the group consisting of hCD31(PECAMl), CDH5, ECSCR, LYVE1, and IGF2, and the vascular secretory factor is any one or more selected from the group consisting of Angiogenin DKK1, EGF, Osteopontin, ICAM-1, IGFBP3, VEGF, VCAM-1, MCP-1, and Activin A,
(f) maintained expression levels of any one or more selected from the group consisting of αSMA, VIM and DESMIN at the time of subculture.

The mature intestinal organoid-derived mesenchymal stromal cells that constitute the stromal cell layer around the mature intestinal organoid may additionally exhibit at least one of the following characteristics:
enhanced expression levels of any one or more (considered as an example of intestinal-related promoting factors) selected from the group consisting of CD34, RSPO3, DLL1, WNT2B, HGF, FOXL1, NOG, GLI1, WNT9A, WNT3, GLI2, and DLL3, compared to adult-derived mesenchymal stem cells;
enhanced expression levels of any one or more (considered as an example of vascular-related promoting factors) selected from the group consisting of VWF, PECAM1, ANGPTL1, CD34, ICAM1, and FLT1, compared to adult-derived mesenchymal stem cells;
enhanced expression levels of NOG and/or WNT9A (considered as an example of intestinal-related promoting factors), compared to immature intestinal organoid-derived mesenchymal stem cells; and/or
enhanced expression levels of any one or more selected from the group consisting of CD31, CDH5, ECSCR, LYVE1, IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1 (considered as an example of vascular-related promoting factors), compared to immature intestinal organoid-derived mesenchymal stem cells.

The mature intestinal organoid-derived mesenchymal stromal cells according to the present disclosure exhibit excellent paracrine effects. More specifically, the mature intestinal organoid-derived mesenchymal stromal cells according to the present disclosure may provide high expression levels of vascular development, mesenchyme development, epithelial cell proliferation, and muscle tissue development-related factors, thereby exhibiting high expression levels of related secretomes. Through this high paracrine efficacy, the present disclosure may 1) increase cell proliferation efficacy, 2) have an excellent ability to transform surrounding cells into cells capable of synthesizing large amounts of biogenic building blocks and ATP, and 3) have excellent angiogenic ability, to thereby be directly used for transplantation or be utilized for assisting transplantation.

On the other hand, the immature intestinal organoid-derived mesenchymal stromal cells are cells derived from intestinal organoids without the use in co-culture to mimic the *in vivo* intestinal environment or without the treatment with cytokines secreted by T-lymphocyte co-culture, or without the treatment with NRG-1 and/or with STAT3 and mTOR signaling pathway activator, colivelin. More specifically, unlike mature intestinal organoid-derived mesenchymal stromal cells, the immature intestinal organoid-derived mesenchymal stromal cells refer to mesenchymal stromal cells derived from intestinal organoids to which neither any one nor more selected from the group consisting of IL-2, IL-22, IL-6, IL-1β, IL-11, EGF, OSM, and IL-10 are added, nor are NRG-1 or colivelin added, as medium components.

Adult-derived mesenchymal stem cells refer to mesenchymal stem cells that exist in any tissue of an adult, such as bone marrow, umbilical cord blood, and adipose-derived mesenchymal stem cells.

Mesenchymal stem cells derived from pluripotent stem cells refer to cells that are differentiated into mesenchymal stem cells through the differentiation process of pluripotent stem cells.

As described above, the mesenchymal stromal cells derived from the stromal cell layer around the mature intestinal organoid have the following advantages and thus have high potential for use as a therapeutic agent for various diseases.

The mesenchymal stromal cells of the present disclosure correspond to mesenchymal stromal cells isolated from pluripotent stem cell-derived intestinal organoids, which can be used for allogeneic transplantation. Specifically, the mesenchymal stromal cells isolated from pluripotent stem cell-derived intestinal organoids with a reduced expression level of human leukocyte antigen (HLA), which are responsible for immune rejection during cell, tissue, and organ transplantation, may be utilized for therapeutic purposes as a cell source that does not cause immune rejection.

The mesenchymal stromal cells of the present disclosure may have high expression levels of angiogenic factors, vascular endothelial-related factors, and/or vascular secretory factors and exhibit gene ontology of endothelial cells, especially vascular endothelial cells, thereby enhancing an early engraftment rate including vascularization during intestinal transplantation.

The mesenchymal stromal cells of the present disclosure may continuously show equivalent characteristics without losing the characteristics of mesenchymal stromal cells during passage culture, and thus they can be maintained for a long period of time without changing cell characteristics and can be subjected to passage culture. Further, the mesenchymal stromal cells of the present disclosure may be used under xeno-free medium conditions, and thus they can also be utilized as cell therapeutic agents.

For use as a therapeutic agent, it is preferable to use the mesenchymal stromal cells at a passage level of 1 to 10, and more preferably at a passage level of 2 to 6.

In particular, the present disclosure has a high potential for use as a therapeutic agent by continuously maintaining the characteristics of stem cells that are usually changed or lost under freezing and thawing conditions.

Under the above advantages, the mesenchymal stromal cells according to the present disclosure may have a high potential for use as an excellent regenerative therapeutic agent in combination with mesenchymal stromal cells themselves, other intestinal organoids or intestinal stem cells based on their high engraftment capacity.

### Medicinal Use

The present disclosure provides a pharmaceutical composition comprising: the mesenchymal stromal cell derived from the stromal cell layer around the mature intestinal organoid.

The present disclosure provides a pharmaceutical composition for preventing or treating an intestinal disease, comprising: the mesenchymal stromal cell derived from the stromal cell layer around the mature intestinal organoid.

The present disclosure provides a pharmaceutical composition for assisting intestinal transplantation, comprising: the mesenchymal stromal cell derived from the stromal cell layer around the mature intestinal organoid.

The present disclosure provides a pharmaceutical composition for preventing or treating an intestinal disease, comprising: the mesenchymal stromal cell derived from the stromal cell layer around the mature intestinal organoid; and a small intestinal organoid, a large intestinal organoid, or an intestinal stem cell. The mesenchymal stromal cells derived from the stromal cell layer around the mature intestinal organoid may be differentiated from pluripotent stem cells (PSCs).

The mesenchymal stromal cells derived from the stromal cell layer around the mature intestinal organoid may exhibit the characteristics of mesenchymal stromal cells and show improved characteristics such as angiogenesis and enhanced engraftment as described above, thereby being utilized alone or in combination with other components for various diseases requiring the administration of mesenchymal stromal cells.

In particular, the present disclosure may be utilized for therapeutic purposes as a bioengineering technology to restore the function of cells or tissues.

For example, the pharmaceutical composition may be utilized as a transplant material and may be applied to treatment of various intestinal diseases. In particular, the pharmaceutical composition may be considered for use as a material for regeneration and reconstruction of injured (including dysfunction) intestinal tissue.

Thus, the pharmaceutical composition according to the present disclosure may be a tissue therapeutic agent.

In the present disclosure, the intestinal disease may be any one or more intestinal diseases selected from the group consisting of intestinal leakage syndrome, short bowel syndrome, irritable bowel syndrome, Crohn's disease, ulcerative colitis, intestinal Behcet's disease, infectious enteritis, ischemic bowel disease, and radiation enteritis.

The mesenchymal stromal cell derived from the stromal cell layer around the mature intestinal organoid differentiated from the pluripotent stem cell (PSC) of the present disclosure may be used as a pharmaceutical composition for assisting intestinal transplantation to perform intestinal transplantation of a small intestinal organoid, a large intestinal organoid (colon organoid) or an intestinal stem cell.

A prerequisite for transplantation efficiency and regenerative treatment efficiency is that blood is supplied to transplanted cells or tissues through angiogenesis by rapidly engrafting on a transplanted site. Conventional small intestinal organoids, large intestinal organoids (colon organoids) or intestinal stem cells have a low engraftment rate and have insufficient vascularization at the time of intestinal transplantation, so that an initial transplantation efficiency is very low. The mesenchymal stromal cell derived from the stromal cell layer around the mature intestinal organoid differentiated from the pluripotent stem cell (PSC) of the present disclosure may be utilized as an adjuvant to improve the efficacy of intestinal transplantation by improving engraftment rates and vascularization efficiency in such intestinal transplantation.

In addition, the present disclosure may be utilized as a composition for treating an intestinal disease, comprising: the mesenchymal stromal cell derived from the stromal cell layer around the mature intestinal organoid differentiated from the pluripotent stem cell (PSC); and a small intestinal organoid, a large intestinal organoid, or an intestinal stem cell.

In such transplantation, the mesenchymal stromal cell derived from the stromal cell layer around the mature intestinal organoid differentiated from the pluripotent stem cell (PSC); and a small intestinal organoid, a large intestinal organoid, and/or an intestinal stem cell may be used in transplantation together with at least any one biodegradable support selected from the group consisting of fibrin, laminin, collagen, gelatin, chitosan, alginate, hyaluronic acid, dextran, polylactic acid, poly(glycolic acid) (PGA), poly(lactic acid-co-glycolic acid) (poly(PLGA)), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide) copolymer, a copolymer thereof, and a mixture thereof.

In other words, the cells or organoids that are the target of the above transplant material may be utilized for transplantation as it is or by being embedded in the support described above.

In addition, dimethylsulfoxide (DMSO) and the like may be added for the purpose of protecting cells, an antibiotic substance and the like may be added for the purpose of blocking incorporation of bacteria, and various components (vitamins, cytokines, growth factors, steroids, and the like) may be added to the transplant material of the present disclosure for the purpose of activating cells, proliferation, or inducing differentiation.

The transplant material of the present disclosure is also available for the construction of *in vivo* test system. For example, the above-described transplant material may be transplanted into experimental animals such as mice, rats, guinea pigs, hamsters, pigs, cynomolgus monkeys, rhesus monkeys, chimpanzees, and the like, to produce humanized animals (human intestinal models). Such humanized animals are particularly useful for experiments such as pharmacokinetics and toxicity tests, and are expected to contribute to studies such as the effect of the first-pass effect on oral drugs or pharmaceutical enteritis.

The term "prevention" herein means all actions that inhibit or delay the onset of intestinal diseases by administration of the composition, and the term "treatment" herein means all actions that improve or benefit the symptoms of intestinal diseases by administration of the composition.

The composition of the present disclosure may include 1.0 x 10⁵ to 1.0 x 10¹⁰, preferably, 1.0 x 10⁶ to 1.0 x 10⁹ cells per 1 ml.

The pharmaceutical composition of the present disclosure may be formulated into various formulations such as a liquid formulation, a suspension, and the like, according to the conventional method.

The pharmaceutical composition of the present disclosure may be administered while being formulated into a unit dosage form of a pharmaceutical formulation suitable for administration into the body of a patient according to the conventional method in the pharmaceutical field, and the formulation includes an effective dosage by one or several administrations. The preferred formulations suitable for this purpose include parenteral formulations such as injections, infusions, and transplants. In addition, the pharmaceutical composition may include a pharmaceutically acceptable common inert carrier and diluent. The pharmaceutically acceptable carrier and diluent may be biologically and physiologically friendly to the mesenchymal stromal cells and recipients to be transplanted with the mesenchymal stromal cell. The diluent may include saline, a water-soluble buffer, a solvent and/or a dispersion medium, but the present disclosure is not limited thereto. In addition, for example, the injection may further include a preservative, a painless agent, a solubilizer, a stabilizer, or the like, and the preparation for topical administration may further include a base, an excipient, a lubricant, a preservative, or the like.

The composition of the present disclosure may be used unfrozen or may be frozen for later use. When the composition is to be frozen, a standard cryopreservative (e.g., DMSO, glycerol, and Epilife^{®} cell freezing medium (Cascade Biologics)) may be added to the cell population prior to freezing.

In addition, the pluripotent stem cell-derived intestinal organoid of the present disclosure may be transplanted and administered using an administration method commonly used in the art, and preferably, may be directly engrafted or transplanted into a disease site of the patient in need of treatment, but the present disclosure is not limited thereto. In addition, the administration may be performed by both non-surgical administrations using a catheter and surgical administration such as injection or transplantation after incision in the disease site. The dosage may be 5 x 10⁵ ~ 10⁸/60 kg adults or 5 x 10⁵ ~ 10⁸/1 dose. However, it should be understood that an actual dosage of an active component should be determined in light of various relevant factors such as a disease to be treated, severity of the disease, a route of administration, a weight, age and sex of the patient, and the like, and thus the dosage does not limit the scope of the present disclosure in any way.

The present disclosure provides a pharmaceutical composition comprising: the mesenchymal stromal cell derived from the stromal cell layer around the mature intestinal organoid for use in preventing or treating an intestinal disease.

The present disclosure provides use of the mesenchymal stromal cell derived from the stromal cell layer around the mature intestinal organoid in the manufacture of a medicament for use in preventing or treating an intestinal disease.

The present disclosure provides a method for treating an intestinal disease, comprising: administering to a subject in need thereof a mesenchymal stromal cell derived from the stromal cell layer around the mature intestinal organoid.

The term "subject" refers to any animal that has developed or may develop an intestinal disease, including humans, monkeys, cows, horses, sheep, pigs, chickens, turkeys, quails, cats, dogs, mice, rats, rabbits, or guinea pigs.

The present disclosure provides a pharmaceutical composition comprising: the mesenchymal stromal cell derived from the stromal cell layer around the mature intestinal organoid for use in assisting intestinal transplantation.

The present disclosure provides use of the mesenchymal stromal cell derived from the stromal cell layer around the mature intestinal organoid in the manufacture of a medicament for use in assisting intestinal transplantation.

The present disclosure provides a method for assisting intestinal transplantation, comprising: administering to a subject in need thereof a mesenchymal stromal cell derived from a stromal cell layer around a mature intestinal organoid.

The present disclosure provides a pharmaceutical composition comprising: the mesenchymal stromal cell derived from the stromal cell layer around the mature intestinal organoid for use in assisting intestinal transplantation.

The present disclosure provides use of the mesenchymal stromal cell derived from the stromal cell layer around the mature intestinal organoid in the manufacture of a medicament for use in assisting intestinal transplantation.

The present disclosure provides a method for assisting intestinal transplantation, comprising: administering to a subject in need thereof a mesenchymal stromal cell derived from a stromal cell layer around a mature intestinal organoid.

The present disclosure provides a pharmaceutical composition for preventing or treating an intestinal disease, comprising: the mesenchymal stromal cell derived from the stromal cell layer around the mature intestinal organoid; and a small intestinal organoid, a large intestinal organoid, or an intestinal stem cell.

The present disclosure provides use of the mesenchymal stromal cell derived from the stromal cell layer around the mature intestinal organoid; and a small intestinal organoid, a large intestinal organoid, or an intestinal stem cell, in the manufacture of a medicament for use in preventing or treating an intestinal disease.

The present disclosure provides a method for treating an intestinal disease, comprising: administering to a subject in need thereof the mesenchymal stromal cell derived from the stromal cell layer around the mature intestinal organoid; and a small intestinal organoid, a large intestinal organoid, or an intestinal stem cell.

Unless otherwise defined, all technical terms used in the present disclosure are used in the same meaning as commonly understood by those skilled in the art in the related field of the present disclosure. In addition, a preferred method or sample is described in the present specification, but those similar or equivalent thereto are also included in the scope of the present disclosure. The contents of all referenced publications herein are incorporated by reference into the present disclosure.

### [Advantageous Effects]

The mature intestinal organoid-derived mesenchymal stromal cells according to the present disclosure can act directly on a lesion site to significantly enhance regeneration and engraftment efficacy, and thus can significantly enhance the efficiency of transplantation. Further, the mature intestinal organoid-derived mesenchymal stromal cells according to the present disclosure have excellent efficacy due to being capable of regenerating damaged regions immediately and directly, unlike conventional mesenchymal stem cell therapeutic agents, allow for the application of a simplified subculture method, can maintain the characteristics thereof even when frozen and thawed, and can be utilized in allogeneic transplantation, thus having the advantage of a high likelihood of universal applicability. Furthermore, when combined with small intestinal organoids, large intestinal organoids (colon organoids) or intestinal stem cells, the present disclosure exhibits universally improved intestinal disease treatment effects, providing high potential for use as a therapeutic adjuvant for intestinal transplantation. In addition, the present disclosure significantly increases the depth of the crypt in the affected area, relieves inflammation, and shows excellent effects in histological recovery and functional recovery of the epithelium.

### [Description of Drawings]

FIG. 1 shows the isolation, culture, and freeze-thaw techniques of mesenchymal stromal cells from transplantable mature intestinal organoids derived from pluripotent stem cells.

FIG. 1a is a schematic diagram of the isolation, culture, and freeze-thaw protocol of mesenchymal stromal cells from the intestinal organoids, which shows maturation of the differentiated human intestinal organoids (hIOs) derived from the pluripotent stem cells through IL-2 treatment from P0 for maturation, wherein mesenchymal stromal cells (MSC^{hIO}) isolated from intestinal organoids P0-P2 are able to be cultured for 3-7 days, followed by subculture, freezing, and thawing.

FIG. 1b shows the phenotypes of immature intestinal organoids (Cont-hIO), mature intestinal organoids (Mat-hIO), differentiated from pluripotent stem cells, and mesenchymal stromal cells isolated therefrom (MSC^{Cont-hIO} and MSC^{Mat-hIO}), which shows representative images of subculture from P0 to P10 after isolation.

FIG. 1c shows the results of qPCR analysis by passage (P2, P3, P4, and P8) of markers (αSMA (ACTA2), Desmin, and Vimentin) of representative cell types of mesenchymal stromal cells, including smooth muscle cells or pericytes, intestinal subepithelial myofibroblasts (ISEMF), and fibroblasts, which indicates that markers of intestinal mesenchymal stromal cells are still maintained across passages.

FIG. 2 shows the genetic level characterization of immature intestinal organoid-derived mesenchymal stromal cells (MSC^{Cont-hIO}), mature intestinal organoid-derived mesenchymal stromal cells (MSC^{Mat-hIO}), mesenchymal stem cells differentiated from pluripotent stem cells (MSC^{PSC}), and commercially available human mesenchymal stromal cells.

FIG. 2a shows immunofluorescence staining results of mesenchymal stromal cell marker and pericyte marker (αSMA), and vascular marker (CD31), which present high expression of αSMA and CD31 in MSC^{Cont-hIO}.

FIG. 2b is a heatmap showing the significant correlation between each sample through Spearman correlation, which indicates that the higher the similarity between samples, the closer it is to red, and that the closer it is to blue, the lower the similarity.

FIG. 2c shows the MDS plot confirmed through gene expression analysis of Fold >2 or higher from RNAseq data, which indicates that MSC^{Mat-hIO} also has different characteristics from MSC^{Cont-hIO}.

FIG. 2d shows EnrichR and Ontology analysis confirming the Endothelial cell type in MSC^{Mat-hIO} compared to MSC^{Cont-hIO}.

FIG. 2e shows the gene expression of vascular endothelial-related factors (CD31 (PECAM1), CDH5, ECSCR, and LYVE1) in Fibroblast, iPSC, MSC^{Cont-hIO}, MSC^{Mat-hIO}, and hMSC, confirmed through qPCR, which presents a significant increase in the isolated MSC^{Mat-hIO}.

FIG. 2f shows the vascular endothelial-related factors (VEGF) secreted from MSC^{Cont-hIO} and MSC^{Mat-hIO} in the culture medium, confirmed through ELISA.

FIG. 3 shows identification of the constituent cells of intestinal organoids through single cell RNAseq of Cont-hIO and Mat-hIO, respectively.

FIG. 3a shows the UMAP of Cont-hIO and Mat-hIO, wherein the left panels (2 panels) show the UMAP of the entire intestinal organoid, and the right panel is a zoomed-in view of a group of mesenchymal stromal cells, which presents the grouping of mesenchymal stromal cells from Cont-hIO and mesenchymal stromal cells from Mat-hIO.

FIG. 3b shows the correlation between pseudo-bulk samples.

FIG. 3c shows the analysis of clusters in gene ontology biological process (GOBP) by selecting differentially expressed genes (DEGs) in Mat-hIO compared to Cont-hIO in the mesenchymal stromal cell group based on scRNAseq, which presents terms related to epithelial cell adhesion and cellular vascular endothelial stimulation.

FIG. 3d shows GOTERM_BP analysis of DEG in DAVID (https://david.ncifcrf.gov/summary.jsp), confirming increases in cell adhesion, angiogenesis, and differentiation in Mat-hIO compared to Cont-hIO.

FIG. 3e shows a detailed analysis of GOTERM of DEG in the mesenchymal stromal cells of Mat-hIO, confirming increases in epithelial cell proliferation, mesenchyme development, vascular endothelial growth factor stimulus, and muscle tissue development.

FIG. 3f shows a volcano plot of DEGs in the mesenchymal stromal cell cluster of Mat-hIO, which indicates that the up-regulated genes in the mesenchymal stromal cells of Mat-hIO include IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, etc.

FIG. 4 shows significant increases in cytokines and vascular-related factors in the conditioned media (CM) in which control group and high-performance mesenchymal stromal cells around the intestinal organoid are isolated and cultured.

FIG. 4a shows dot blot analysis of the secretory factors of the conditioned media of the control group and high-performance mesenchymal stromal cells around the intestinal organoid, confirmed from the Human XL Cytokine Array kit, which presents remarkable increases in the secretion of Angiogenin, DKK1, EGF, Osteopontin, ICAM-1, IGFBP-3, and VCAM-1.

FIG. 4b shows dot blot analysis of the secretory factors of the conditioned media of the control group and high-performance mesenchymal stromal cells around the intestinal organoid, confirmed from the Human Angiogenesis Array kit, which presents remarkable increases in the secretion of angiogenin, Activin A, and MCP-1.

FIG. 5 shows the effect of the culture media of the control group and high-performance mesenchymal stromal cells around the intestinal organoid on the growth and morphology of intestinal stem cells (ISCs).

FIG. 5a is a schematic diagram showing the culture of intestinal stem cells in ISC medium, MSC^{Cont-hIO} CM (Cont-CM), MSC^{Mat-hIO} CM (Mat-CM), hMSC-CM, MSC medium (early Cont-MSC medium), and MSC medium+IL-2 (early Mat-MSC medium) culture media for 3 days.

FIG. 5b shows cell morphology on Day 0, 1, and 3. On Day 1, Mat-CM showed a marked change in ISC morphology.

FIG. 5c shows the distribution of cells under Mat-CM conditions on Day 1 using Crystal Violet (CV) staining, quantitatively confirming a significant increase in cell distribution in Mat-CM.

FIG. 5d shows the selection of representative genes (DEGs) showing significant differences in Mat-MSCs compared to Cont-MSCs based on scRNAseq to identify factors that significantly change the morphology and distribution of ISCs in Mat-CMs, and the changes observed by treating the selected genes with growth factors (BMP4) and each MSC-CM, which presents changes on days 1 and 3 after BMP4 single treatment, ISC medium, Cont-CM, Mat-CM, hMSC-CM, MSC medium, and MSC medium+IL-2 treatment.

FIG. 5e shows Cont-Cm, Cont-CM+BMP4, and Mat-CM treated for 1 day to verify the effect of BMP4 on ISC morphology and distribution changes, which indicates that cell distribution in Cont-CM+BMP4 treatment shows changes in morphology and distribution expansion at a similar rate to Mat-CM treatment, compared to Cont-CM.

FIG. 6 shows real-time cell metabolism analysis by culturing intestinal stem cells for 12 hours with ISC medium, Cont-CM, Mat-CM, and hMSC-CM.

FIG. 6a shows cell morphology at 0 hour and 12 hour before observing real-time cell metabolism with the XF analyzer.

FIG. 6b shows that ISC medium, MSC^{Cont-hIO} CM and MSC^{Mat-hIO} CM have no differences in glycolytic process and OXPHOS, by confirming the difference in Basal ECAR/Basal OCR.

FIG. 6c shows sequential treatment with oligomycin, FCCP, and antimycin A to measure the oxygen consumption rate (OCR), indicating a significant increase in maximum respiration in MSC^{Mat-hIO} CM.

FIG. 6d shows sequential treatment with oligomycin, glucose, and 2-D-glucose to measure extracellular acidity rate (ECAR), indicating that MSC^{Mat-hIO} CM has significantly higher glycolytic capacity and glycolytic reserve, and has more glycolytic process ability.

FIG. 7 shows tubule formation confirmed by treating the representative genes having significant difference in Cont-CM and Mat-CM of HUVEC with growth factor (IGF2) and each MSC-CM.

FIG. 7a shows observation at 4 hours and 8 hours in the group seeded under the condition of 25,000 cells per well, which presents HUVEC tubule formation by treatment with growth factor (IGF2) and EBM-2+bFGF (Basal medium), IGF2, Cont-CM, Cont-CM+IGF2, Mat-CM, EGM-2 (Growth medium). Tubules are formed faster when treated with IGF2 than with EBM-2+bFGF (Basal medium) or when IGF2 is added to Cont-CM than when treated with Cont-CM single treatment. The Cont-CM+IGF2 and Mat-CM conditions at 8 hours show high rates of complete tubule formations, good branching angles, and low cell aggregates.

FIG. 7b shows quantitative evaluation of tubule formation performed using ImageJ's Angiogenesis Analyzer. It shows that addition of IGF2 to Cont-CM results in a significant increase in the number of junctions and segments compared to Cont-CM single treatment. For total length, Cont-CM, Cont-CM+IGF2, and Mat-CM conditions show a significant increase compared to EBM-2+bFGF (Basal medium).

FIG. 8 shows wound healing (migration) confirmed by treating the representative genes having significant difference in Cont-CM and Mat-CM of HUVEC with growth factor (IGF2) and each MSC-CM.

FIG. 8a shows wound formation in HUVEC cells (0 hr), and cell morphology (bright field) of wound healing (migration) of cells at 36 hours, respectively.

FIG. 8b shows quantitative evaluation results of the wound size using the wound healing size tool of ImageJ, which indicates that migration proceeds rapidly in Cont-CM+IGF2, Mat-CM, and EGM-2 (Growth medium).

FIG. 9 shows cell engraftment and regeneration efficacy verified after transplantation of pluripotent stem cells (embryonic stem cells, induced pluripotent stem cells)-derived immature intestinal organoid (Cont-hIO) and mature intestinal organoid-derived mesenchymal stromal cells (MSC^{Mat-hIO}) into immunodeficient mice with EDTA-induced intestinal epithelial injury.

FIG. 9a is a schematic diagram illustrating the transplantation of pluripotent stem cells (embryonic stem cells, induced pluripotent stem cells)-derived immature intestinal organoid (Cont-hIO) and mesenchymal stromal cells isolated from mature intestinal organoid (MSC^{Mat-hIO}) into EDTA-induced intestinal epithelial injury model.

FIG. 9b shows cell morphology of immature intestinal organoid (Cont-hIO) before transplantation and mature intestinal organoid-derived mesenchymal stromal cells (MSC^{Mat-hIO}).

FIG. 9c shows the colonic environment of each group observed before transplantation, immediately after epithelial injury, and 2 weeks after transplantation, using a colonoscope for experimental animals. In addition, it shows fluorescence dissecting microscope images confirming the status of the excised intestine and the engraftment confirmed by fluorescence expression using reporter intestinal organoids derived from induced pluripotent stem cells.

FIG. 9d shows the histological characteristics through H&E staining, and the functional properties of Goblet cells through AB-PAS staining, in the transplanted rectum area. Compared to the Cont-hIO transplantation group, the Cont-hIO+MSC^{Mat-hIO} co-transplantation (simultaneous transplantation) group shows structurally higher crypt depth and formation (restoration) rate, particularly, functionally active secretion of Mucin.

FIG. 10 shows the results of weight analysis and colonoscopic examination confirmed after co-transplantation of pluripotent stem cell-derived immature intestinal organoid (Cont-hIO) or immature intestinal organoid and mature intestinal organoid-derived mesenchymal stem cells (MSC^{Mat-hIO}) into immunodeficient mice with DSS-induced inflammatory bowel disease, respectively.

FIG. 10a is a schematic diagram illustrating the transplantation of immature intestinal organoid or immature intestinal organoid-derived and mature intestinal organoid-derived mesenchymal stem cells into the DSS-induced inflammatory bowel disease model.

FIG. 10b shows the change in body weight of each group from the time of transplantation into the inflammatory bowel disease model until the excision time after transplantation. Compared to the Cont-hIO single transplantation group, the Cont-hIO+MSC^{Mat-hIO} co-transplantation group recovers and gains weight rapidly, and the Cont-hIO transplantation group shows a decrease in initial disease severity compared to the Fibrin transplantation group or DSS-NT (disease control), but a slower recovery compared to the Cont-hIO+MSC^{Mat-hIO} transplantation group.

FIG. 10c shows the extent of inflammation and recovery in the intestine of each group on Day 0 and 14 after epithelial injury transplantation, observed using a colonoscope for experimental animals. Redness and some areas of inflammation remain in the MSC^{Mat-hIO} single transplantation group or Cont-hIO transplantation group, but the Cont-hIO+MSC^{Mat-hIO} transplantation group shows no redness and inflammation due to intestinal epithelial injury.

FIG. 10d shows the histological characteristics through H&E staining, and the functional properties of Goblet cells through AB-PAS staining, from longitudinal sections of the cell-transplanted intestine. Compared to the Cont-hIO transplantation group, the Cont-hIO+MSC^{Mat-hIO} co-transplantation group shows structurally higher crypt depth and formation (restoration) rate, particularly, functionally active secretion of Mucin.

FIG. 10e shows crypt depth quantification, confirming a significant increase in Cont-hIO+MSC^{Mat-hIO} co-transplantation group.

FIG. 11 shows histological/functional analysis by AB-PAS of mature intestinal organoid (P7) without MSCs and mature intestinal organoid (P4) with MSCs on Day 14 after transplantation. The mature intestinal organoid-transplantation group with MSCs (Mat-hIO P4) shows a high crypt depth throughout the structure through Swiss-roll section.

FIG. 11a is a schematic diagram illustrating transplantation of induced pluripotent stem cell-derived mature intestinal organoids (P4, P7) into an EDTA-induced intestinal epithelial injury model.

FIG. 11b shows cell morphology of mature intestinal organoid without MSCs (P7) and with MSCs (P4) before transplantation.

FIG. 11c shows the histological structure and functional characteristics of Goblet cells through AB-PAS staining of Swiss-roll sections to confirm the structure of the entire intestine. Compared to the mature intestinal organoid-transplantation group without MSCs (P7), the mature intestinal organoid-transplantation group with MSCs (P4) structurally has significantly high crypt depth and formation (recovery) rate.

FIG. 12 shows the differentiation process of induced pluripotent stem cell-derived large intestinal (colonic) organoid and characteristics thereof compared to conventional small intestinal organoid, and shows the engraftment and regeneration efficacy confirmed 2 weeks after co-transplantation of induced pluripotent stem cell-derived colon organoid or MSC^{Mat-hIO}, and single transplantation of MSC^{Mat-hIO} into immunodeficient mice with DSS-induced inflammatory bowel disease to confirm the utilization of MSC^{Mat-hIO}.

FIG. 12a is a schematic diagram showing the differentiation process of induced pluripotent stem cell-derived large intestinal (colonic) organoid. Unlike small intestinal organoid differentiation, hindgut may be differentiated into large intestinal organoid by additional treatment with Activin A and treatment with BMP2 for 3 days in the early stage (P0) of the colon organoid (hCO).

FIG. 12b shows morphology of organoids of Hindgut and hCO by passage (P0, P1, P2, P6, and P10) during the differentiation process of colon organoid (hCO).

FIG. 12c shows qPCR results, confirming that representative markers of the intestine (VIL1, KRT20, LYZ, SI, LCT, and OLFM4) are more highly expressed in small intestinal organoid, and CHGA, MUC5B, SATB2, and CAII are highly expressed in colon organoids.

FIG. 12d is a schematic diagram of transplanting hCO, hCO+MSC^{Mat-hIO}, and MSC^{Mat-hIO} into a DSS-induced inflammatory bowel disease model.

FIG. 12e shows the extent of inflammation and recovery in the intestine of each group before and 2 weeks after transplantation, observed using a colonoscope for experimental animals, which presents a reduction in redness in the hCO+MSC^{Mat-hIO} co-transplantation group compared to the hCO and MSC^{Mat-hIO} transplantation groups.

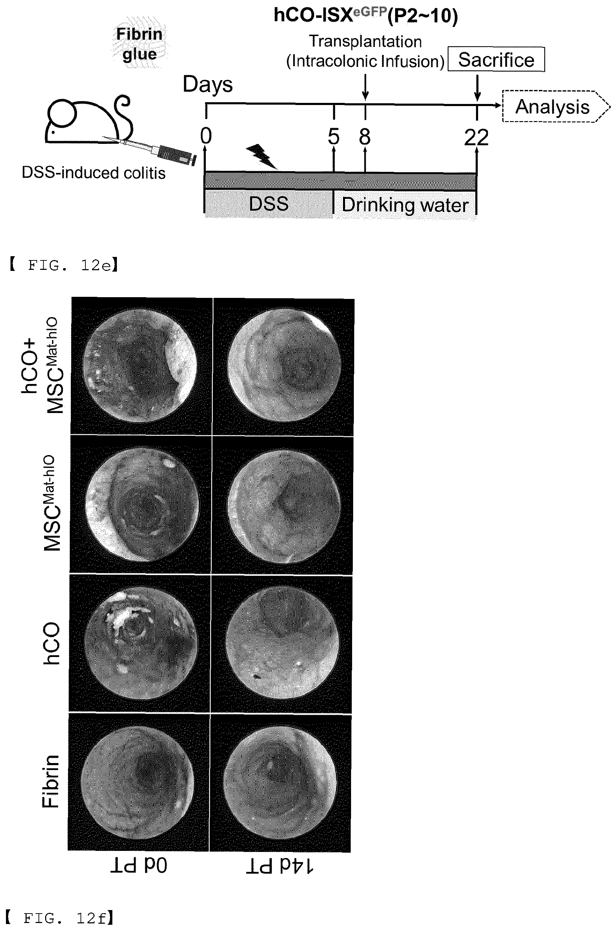

FIG. 12f shows the histological characteristics through H&E staining, and the functional properties of Goblet cells through AB-PAS staining, in the rectum area, which presents the results of crypt structure and mucin staining confirming that the control group transplanted with fibrin (ECM only) still had poor histological recovery after 14 days, while the hCO or MSC^{Mat-hIO} single transplantation group showed recovery of intestinal epithelial injury caused by inflammation compared to Fibrin (control group). In particular, the hCO+MSC^{Mat-hIO} co-transplantation group exhibits structurally significantly high crypt depth and functionally active secretion of mucin.

FIG. 13 shows early transplantation (Day 5) observations of colon organoids produced from adult colonic stem cells from human colonic tissue and MSC^{Mat-hIO} transplanted into immunodeficient mice with EDTA-induced intestinal epithelial injury to confirm the utilization of MSC^{Mat-hIO}.

FIG. 13a is a schematic diagram of transplantation of fibrin, hCN, and hCN+MSC^{Mat-hIO} into the EDTA-induced intestinal epithelial injury model.

FIG. 13b shows the extent of inflammation and recovery in the intestine of each group before and 2 weeks after transplantation, observed using a colonoscope for experimental animals. Compared to the hCN single transplantation group, the hCN+MSC^{Mat-hIO} co-transplantation group shows a reduction in intestinal epithelium injury in the rectal area.

FIG. 13c shows the histological characteristics through H&E staining, and the functional properties of Goblet cells through AB-PAS staining, in the rectum area. Compared to the fibrin-transplanted control group (ECM only) and hCN transplantation group, the hCN+MSC^{Mat-hIO} co-transplantation group shows rapid engraftment in the rectal area and formation of structure, and functionally active secretion of mucin.

FIG. 13d shows the fluorescence expression of human-specific antibodies (hECAD) confirmed by immunofluorescence staining (IHC), which presents quantification of the hECAD+ area relative to nuclei (DAPI), indicating that the hCN+MSC^{Mat-hIO} xenograft group on day 5 has better engraftment efficacy than hCN.

FIG. 14 shows transplantation of a colon organoid derived from adult colon stem cells of human colon tissue and MSC^{Mat-hIO} into immunodeficient mice with EDTA-induced intestinal epithelial injury, confirming the engraftment and regeneration efficacy 2 weeks after transplantation.

FIG. 14a is a schematic diagram illustrating the transplantation of adult colon stem cell-derived colon organoid and MSC^{Mat-hIO} into an EDTA-induced intestinal epithelial injury model.

FIG. 14b shows the change in body weight of each group from the time of transplantation into the intestinal epithelial injury model until the excision time after transplantation. Compared to the hCN single transplantation group, the hCN+MSC^{Mat-hIO} co-transplantation group shows weight gain from day 3, a large difference between the groups on days 5 to 7, and on day 14, the hCN transplantation group also shows rapid weight recovery.

FIG. 14c shows the colonic environment of each group observed before transplantation, immediately after epithelial injury, and 2 weeks after transplantation, using a colonoscope for experimental animals. At 2 weeks after transplantation, it shows small difference in the degree of epithelial injury between the groups.

FIG. 14d shows histological characteristics through H&E staining in the cell-transplanted rectum area. On day 14, the Fibrin group still shows epithelial injury in the rectum area, but the hCN, hCN+MSC^{Mat-hIO}, and Mat-hIO transplantation groups show recovery of the epithelial structure at the entrance.

FIG. 14e shows functional characteristics of goblet cells using AB-PAS staining. On day 14, the Fibrin group still shows epithelial injury in the rectum area, resulting in a lack of mucin secretion, while the hCN, hCN+MSC^{Mat-hIO}, and Mat-hIO transplantation groups show recovery of epithelial structure at the entrance and active mucin secretion.

FIG. 15 shows the results of transplanting a colon organoid derived from adult colon stem cells of human colon tissue and MSC^{Mat-hIO} into immunodeficient mice with DSS-induced intestinal epithelial injury, confirming the engraftment and regeneration efficacy 2 weeks after transplantation.

FIG. 15a is a schematic diagram illustrating the transplantation of adult colon stem cell-derived colon organoid and MSC^{Mat-hIO} into an DSS-induced intestinal epithelial injury model.

FIG. 15b shows cell morphology of adult colon stem cell-derived colon organoid (hCN) and MSC^{Mat-hIO} before transplantation.

FIG. 15c shows colonoscopy for experimental animals to observe the colonic environment of each group before transplantation, 1 week after transplantation, and 2 weeks after transplantation, which presents that at 2 weeks after transplantation, the difference in the degree of epithelial injury between the hCN and hCN+ MSC^{Mat-hIO} groups is small.

FIG. 15d shows histological characteristics through H&E staining in the cell-transplanted rectum area. After 2 weeks, the Fibrin group still shows residual epithelial injury due to inflammation from the rectum to the middle of the intestinal epithelium, whereas the hCN, hCN+MSC^{Mat-hIO}, and Mat-hIO transplantation groups show recovery of the epithelial structure throughout the intestine.

FIG. 15e shows functional characteristics of goblet cells using AB-PAS staining. After 2 weeks, the Fibrin group still shows epithelial injury caused by inflammation throughout the intestine, resulting in a lack of mucin secretion, while the hCN, hCN+MSC^{Mat-hIO}, and Mat-hIO transplantation groups show recovery of intestinal epithelial structure and active mucin secretion.

FIG. 16 shows the transplantation efficacy of spherical intestinal stem cells (InS^{hIO}) isolated from induced pluripotent stem cell-derived immature or mature intestinal organoid in immunodeficient mice with EDTA-induced intestinal epithelial injury.

FIG. 16a is a schematic diagram of transplantation of immature intestinal organoid-derived intestinal stem cells (InS^{Cont-hIO}), mature intestinal organoid-derived intestinal stem cells (InS^{Mat-hIO}), and MSC^{Mat-hIO} into an EDTA-induced intestinal epithelial injury model. Further, it shows the cell morphology of mature intestinal organoid-derived intestinal stem cells (InS^{Mat-hIO}) before transplantation.

FIG. 16b shows that on Day 14 after transplantation, the InS^{Mat-hIO}+MSC^{Mat-hIO}-transplantation group shows recovery of the mucin secretion functionality of goblet cells, as confirmed by AB-PAS staining of Swiss-roll sections. In addition, the rectum area of the longitudinal section sample shows structurally rapid recovery, indicating a high crypt depth and improved mucin functionality, as confirmed by H&E and AB-PAS staining.

FIG. 17 shows the co-transplantation efficacy of MSC^{Mat-hIO} and spherical intestinal stem cells (InS^{hIO}) isolated from pluripotent stem cell-derived mature intestinal organoid in immunodeficient mice with DSS-induced inflammatory bowel disease to confirm the utilization of MSC^{Mat-hIO}.

FIG. 17a is a schematic diagram illustrating the transplantation of intestinal stem cells or mature intestinal organoid-derived mesenchymal stem cells into a DSS-induced inflammatory bowel disease model.

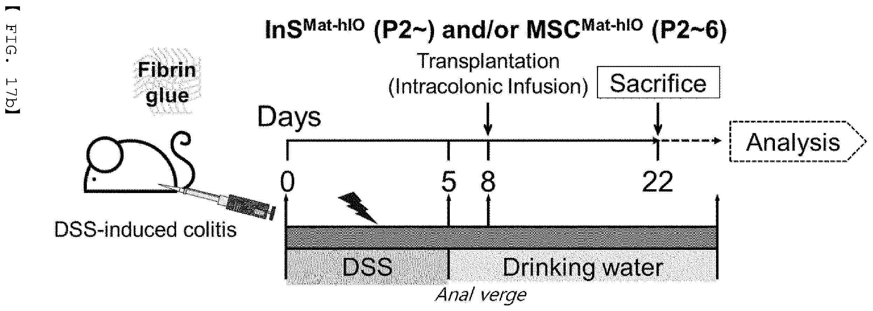

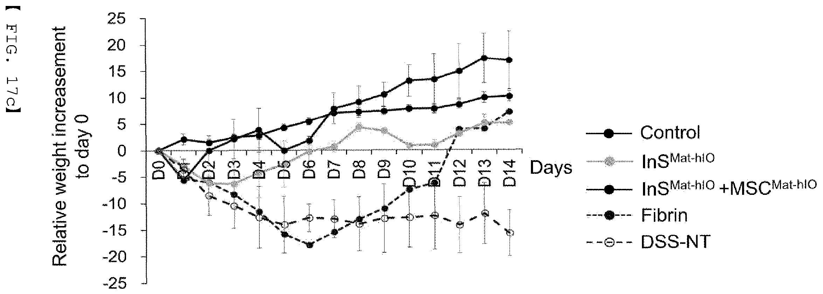

FIG. 17b shows the change in body weight of each group from the time of transplantation into the inflammatory bowel disease model until the excision time after transplantation. Compared to the InS^{Mat-hIO} single transplantation group, the InS^{Mat-hIO}+MSC^{Mat-hIO} transplantation group shows weight gain at a faster rate than the untreated control group. The InS^{Mat-hIO} single transplantation group shows faster recovery from the initial disease and no worsening of symptoms compared to the Fibrin transplantation group or DSS-NT (disease control group), but shows slower recovery than the InS^{Mat-hIO}+MSC^{Mat-hIO} transplantation group.

FIG. 17c shows the extent of inflammation and recovery in the intestine of each group on Day 0 and 14 after epithelial injury transplantation, observed using a colonoscope for experimental animals.

FIG. 17d shows the histological characteristics through H&E staining, and the functional properties of Goblet cells through AB-PAS staining, in the rectum area. Compared to the fibrin-transplanted control group (ECM only) and InS^{Mat-hIO} transplantation group, the InS^{Mat-hIO}+MSC^{Mat-hIO} co-transplantation group shows rapid recovery throughout the intestine and functionally active secretion of mucin.

FIG. 18 shows a technique for differentiation and culture of high-performance mature intestinal organoids for transplantation derived from pluripotent stem cells.

FIG. 18a is a schematic diagram of a protocol for differentiation and subculture into mature intestinal organoids from pluripotent stem cells, which shows a protocol for maturation of human intestinal organoids (hIO) derived and differentiated from pluripotent stem cells, through daily treatment with 1 ng/ml rhIL-2 from P0 for maturation, wherein mature intestinal organoids are subcultured every 10 to 14 days, and especially from P1, a blade or chopper is used for subculture.

FIG. 18b shows cell morphology of immature or mature intestinal organoids (P2) differentiated from pluripotent stem cells (embryonic stem cells and induced pluripotent stem cells).

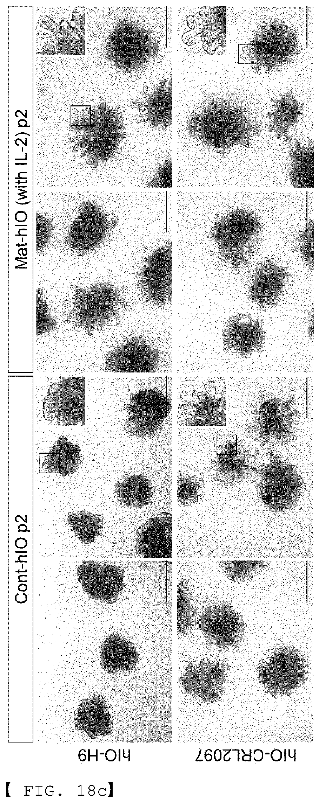

FIG. 18c shows qPCR analysis results of comparison between organoids (conventional) produced by the conventional method for producing mature organoids (treatment with rhIL-2 from P1, subculture with a surgical blade, and containing 80-90 ng/ml Noggin and 400-450 ng/ml R-spondin1 in hIO medium) and organoids (transplantable) produced by a method for producing transplantable mature intestinal organoids (treatment with rhIL-2 from P0, subculture with a surgical blade or chopper from P1, and containing 40-50 ng/ml Noggin and 200-250 ng/ml R-spondin1 in hIO medium), which presents expression of intestinal cell type-specific markers (VIL1, KRT20, CHGA, MUC2, and LYZ) and intestinal maturation markers (MUC13, OLFM4, SI, LCT, and DPP4 and LCT).

FIG. 18d shows comparison of transplantable immature and mature organoids through qPCR analysis, which presents the expression of intestinal stem cell markers ASCL2, AXIN2, and OLFM4.

FIG. 18e shows comparison of transplantable immature and mature organoids through qPCR analysis, which presents the expression of vascular-specific markers CD31, CDH5, ECSCR, LYVE1, and IGF2.

FIG. 18f shows comparison of conventional mature organoids and transplantable mature intestinal organoids through qPCR analysis, which presents that expression of intestinal stem cell-specific markers (ASCL2, AXIN2, LGR5, and OLFM4) and vascular endothelium-related factors (CD31, CDH5, ECSCR, and LYVE1) is highly expressed in transplantable mature intestinal organoids.

FIG. 19 shows organoids differentiated into intestinal organoids from pluripotent stem cells in ECMatrix^{™}-511, cultured using Matrigel and clinically applicable Xeno-free ECM (Vitrogel #2, #3).

FIG. 19a shows the maintenance of the intestinal epithelial structure in Vitrogel #2, #3, and the histological analysis through H&E staining and cell morphology after 5 days of culture.

FIG. 19b shows expression of small intestine-specific markers (CDX2, VIL1, LGR5, ASCL2, LYZ, MUC2, and MUC13) through qPCR.
shows xenograft intestinal organoids observed 8 weeks after transplantation of immature or high-performance mature organoids into each kidney of the same immunodeficient mouse

FIG. 20a shows a process of transplanting immature intestinal organoids (Cont-hIO) or mature intestinal organoids (Mat-hIO) derived from induced pluripotent stem cells into each kidney of the same immunodeficient mouse, followed by excision after 8 weeks.

FIG. 20b shows the cell morphology of immature or mature intestinal organoids derived from induced pluripotent stem cells before transplantation (P1).

FIG. 20c shows the kidneys excised from mice 8 weeks after transplantation. Even when transplanted into the same mouse, vascularization is observed around the Mat-hIO xenograft (n=6).

FIG. 20d (left panel) shows sections of isolated kidneys and xenografts, indicating histological analysis of structures such as adult small intestine and vascularization by H&E staining, which shows the vascularization in Mat-hIO. Right panel shows a graph showing the measurement of Villus length of Cont-hIO and Mat-hIO xenografts.

FIG. 20e shows immunofluorescence staining of xenografts obtained from the kidneys of mice excised 8 weeks after transplantation, confirming the small intestinal epithelial structure (hECAD), mesenchymal stromal cell and pericyte markers (αSMA), and vascular markers hVE-Cad, PECAM-1 (hCD31), and VEGF. Mat-hIO xenografts exhibit expression patterns similar to human small intestinal tissue.

FIG. 21 shows comparison of the genomes of *in vitro* cultured immature or high-performance mature organoids (P2) and immature intestinal organoids (Cont-hIO) and mature intestinal organoids (Mat-hIO) transplanted as a renal xenograft at 4 or 8 weeks from RNAseq data.

FIG. 21a shows 3D MSC plot using Euclidean distance to determine the migration path of in vitro organoids 4 and 8 weeks after transplantation.

FIG. 21b is a heatmap showing the data comparing the gene expression levels of each sample, wherein 8-week-old xenograft Mat-hIO and *in vitro* mature organoids exhibit gene expression more similar to human small intestinal tissue.

FIG. 21c shows visualization of the functionally grouped annotation network of selected terms of 538 genes in ClueGO of Cytoscape plugin. The size of the nodes (circles) reflects the statistical significance of the terms. Edges represent the degree of connectivity between terms. Each color represents a group of GO terms. The most important term showing the difference in Mat-hIO xenograft group compared to the Cont-hIO xenografted for 8 weeks is Vascular development.

FIG. 21d shows GOTERM_BP analysis of DEG in DAVID (https://david.ncifcrf.gov/summary.jsp), confirming increases in angiogenesis, blood vessel development, vasculogenesis, cell differentiation, etc., in Mat-hIO xenografts (8 weeks) compared to Cont-hIO.

FIG. 21e shows the mRNA levels, verified by qPCR, based on the RNAseq results, of vascular markers (hCD31, ANGPT1, and KDR), mesenchymal stromal cell markers (αSMA, VIM, and DESMIN), and intestine-specific markers (VIL1, KRT20, CHGA, LYZ, DEFA5, OLFM4, MUC2, MUC13, DPP4, LCT, SLC5A1, and CREB3L3). Vascular markers (hCD31, ANGPT1, and KDR) are increased in the 8-week Mat-hIO xenograft group, and mesenchymal stromal cell marker and pericyte marker (αSMA) is increased in the 4-week Mat-hIO xenograft group.

### [Best Mode]

The following exemplary embodiments are presented to help understanding of the present disclosure. These Examples and Preparation Examples are only provided to more easily understand the present disclosure, but the content of the present disclosure is not limited by these Examples.

### Experimental Example 1. Cell culture and iPSC production

Human pluripotent stem cells (hPSCs) including human embryonic stem cells (hESCs) and human induced pluripotent stem cells (hiPSCs) were cultured by the known method (Molecular carcinogenesis 55, 387-396 (2016), Proteomics 15, 2220-2229 (2015)). Non-integrating-hiPSCs were transfected by electroporation and reprogrammed using Episomal iPSC reprogramming vector (Cat. No. A14703. Invitrogen, Carlsbad, CA, USA) according to the known method.

After 5 days of electroporation, fibroblasts were plated in 1 x 10⁵/well on Matrigel (BD Biosciences, San Diego, CA, USA)-coated 6-well plates, and cultured in E8 medium (Stem Cell Technologies, Vancouver, Canada). After 3 weeks, hiPSC colonies were selected, and the number of cells was increased for passage and subsequent characterization.

### Experimental Example 2. Differentiation from hPSC into intestinal organoid (hIO) for producing immature intestinal organoid (Cont-hIO)

Human intestinal organoids (hIOs) were produced using the known method (Nature 470, 105-109 (2011)). To induce definitive endoderm, hPSCs were plated on Matrigel or dishes coated with ECMatrix^{™}-511 and treated with 100 ng/ml of Activin A (R&D Systems, Minneapolis, MN, USA) in an RPMI 1640 medium having dialyzed fetal bovine serum (dFBS, HyClone, Thermo Fisher Scientific Inc., Waltham, MA, USA) at concentrations of 0%, 0.2% and 2% for 3 days. In addition, in order to perform differentiation into 3D hindgut spheroids, 500 ng/ml of FGF4 (R&D Systems) and 3 µM of CHIR99021 (TOCRIS) were treated together with the RPMI 1640 medium containing 2% of dFBS for 4 to 6 days. From day 4, which was induced to the hindgut, spheroids were inserted into Matrigel (BD Biosciences), cultured in a hIO medium (2 mM of L-glutamine, 1% of penicillin-streptomycin, and 15 mM of HEPES buffer in Advanced DMEM F12) including 1X B27 (Invitrogen), 200 to 250 ng/ml of R-spondin 1 (R&D Systems), 100 ng/ml of EGF (R&D Systems), and 40 to 50 ng/ml of Noggin (R&D Systems), and subcultured every 10 to 14 days. In particular, from P1, the cells were cut using a McIlwain Tissue Chopper during subculture, embedded in Matrigel or Vitrogel, and cultured in hIO medium.

### Experimental Example 3. Culture technique for producing mature intestinal organoid (Mat-hIO)

After the differentiation into the hindgut, interleukin-2 (hereinafter, rhIL-2 (R&D Systems)) at a concentration of 1 ng/ml (approximately 13 U/ml) was added to the hIO medium daily at the hIO (P0) inserted into Matrigel. The hIO medium was treated with 1 ng/ml rhIL-2 every day until hIO P2, and subsequently, starting from hIO P3, the culture medium could be replaced every two days, along with treatment with rhIL-2. In particular, in order to maintain the peripheral stromal cells, the cells were cut to a certain size level (500 um x 500 um) or more using a chopper, etc., and subcultured. Since the peripheral stromal cells were not isolated arbitrarily, the characteristics of high-performance mature intestinal organoids could be maintained.

### Experimental Example 4. Isolation and culture of mesenchymal stromal cells from immature (Cont-hIO) and mature intestinal organoid (Mat-hIO)

For mesenchymal stromal cell culture, 0.2% gelatin was coated on the culture dish for 1 hour. When subculturing intestinal organoids from immature (Cont-hIO) and mature intestinal organoids (Mat-hIO) P0~P2, the organoids were isolated from the Matrigel dome, and the mesenchymal stromal cells present in the Matrigel dome and the bottom were washed with ice-Advanced DMEM/F12. Afterwards, the cells were treated with Trypsin-EDTA (TE) at 37°C for 3-5 minutes to be isolated into single cells, followed by washing once with hIO medium. Cells collected by centrifugation were cultured in a pre-gelatin-coated culture dish with hIO medium containing 1x B27 and 100 ng/ml EGF. Mature intestinal organoid-derived mesenchymal stromal cells could be treated with rhIL-2 (1 ng/ml) while changing the culture medium every two days. Subculture was performed in the same manner at a ratio of 1 : 3 after 3-5 days.

### Experimental Example 5. Differentiation and culture techniques for producing colon organoid (hCO)

Human colon organoids (hCOs) were produced using the known method (see Cell Stem Cell 21(1): 51-64 (2017)). As previously described, to induce complete endoderm, hPSCs were treated with 100 ng/ml Activin A for 3 days in RPMI 1640 medium with 0%, 0.2%, and 2% dialyzed fetal bovine serum (dFBS). In addition, in order to differentiate the hPSCs into 3D hindgut spheroids, 100 ng/ml Activin A, 500 ng/ml FGF4, and 3 µM CHIR99021 were treated together with the RPMI 1640 medium containing 2% of dFBS for 4 to 6 days. From 4 days after induction into the hindgut, at least 30 spheroids were embedded in Matrigel and patterned for 3 days in hIO medium (2 mM L-glutamine, 1% Penicillin-Streptomycin, and 15 mM HEPES buffer in Advanced DMEM F12) containing 1X B27, 100 ng/ml EGF, and 100 ng/ml BMP2 (R&D Systems). Then, the resulting product was cultured in hIO medium containing 1X B27 and 100 ng/ml EGF, and subcultured every 14 days. In particular, from P1, the cells were cut into 500 um x 500 um pieces using a McIlwain Tissue Chopper, embedded in Matrigel, and cultured in hIO medium containing 1X B27 and 100 ng/ml EGF.

### Experimental Example 6. Culture of adult stem cell-derived colon organoid (hCN) obtained from human colon tissue

Human adult stem cell-derived colon organoids (hCNs) were produced and cultured using the known method (Methods Mol Biol. 1576:327-337 (2019)). Human tissues were washed with cold saline, the intestinal mucosa and epithelium (Mucosa) were isolated from the intestinal submucosa, and dissociated with Trypsin-EDTA (TE). The isolated crypt was washed 4 to 6 times and passed through a 150 um filter mesh to collect the crypts or gland fractions. The collected crypts were pelleted by centrifugation (150 g, 5 min), washed repeatedly, and then mixed with Matrigel and human colonoid medium (50% v/v Wnt-3A-conditioned medium, 10% v/v R-Spondin-1-conditioned medium, 100 ng/ml recombinant human Noggin (Peprotech, cat no. 120-10C), 50 ng/ml recombinant murine EGF (Peprotech, cat no. 315-09), 500 nM A-83-01 (Sigma-Aldrich, cat no. SML0788), 10 µM SB202190 (Sigma-Aldrich, cat no. S7067), 10 nM [Leu]15-Gastrin-1 human, SB202190 (Sigma-Aldrich, cat no. G9145), 10 mM nicotinamide (Sigma-Aldrich, cat no. N0636), 1 mM N-acetylcysteine (Sigma-Aldrich, cat no. A9165), 2 mM GlutaMAX (Life technologies, cat no. 35050-061), 10 mM HEPES (Life technologies, cat no. 15630-080), 1x penicillin-streptomycin (Life technologies, cat no. 15140-148), 1x N2 supplement (Life technologies, cat no. 17502-048), 1x B27 supplement (Life technologies, cat no. 17504-044), and 1% w/v BSA in advanced DMEM/F12 medium (Life technologies, cat no. 12634-028)) in a 1:1 ratio and seeded so that 100 crypts were cultured in a 20 ul dome in a 48-well plate. Matrigel dome was solidified in a 37°C CO₂ incubator for 10 minutes, and then cultured by adding 2.5 µM CHIR99021 and 2.5 µM thiazovivin to 300 µl of culture medium. Human colonoid (hCN) medium was replaced every 2 days and passaging was performed every 6-8 days at a 1:3 to 4 ratio. During the passaging, 10 µM Y-27632 was added to the hCN medium for the first 2 days and the hCN medium was changed every 2 days.

### Experimental Example 7. Production and culture techniques of immature or mature organoid-derived spherical intestinal stem cells (InS-hIO)

3D Intestinal stem cells derived from human intestinal organoids (hIO) were produced using the known method (Int J Stem Cells. 28;15(1):104-111 (2022)). As previously described, immature (Cont-hIO) or mature intestinal organoids (Mat-hIO) were isolated into single cells using TE at 37°C for 5 minutes, and centrifuged at 1500 rpm for 5 minutes. The cell pellets were suspended in Matrigel, and the InS-Matrigel mixture was formed in a dome and cultured in a CO₂ cell incubator for 30 minutes or more to solidify Matrigel. For the first 2 days, the cells were cultured by treatment with 10 µM Y-27632 (Tocris) and 1 µM Jagged-1(AnaSpec, Fremont, CA, USA) in InS^{exp} medium (2mM l-glutamine, 15 mM Hepes buffer, 1X B27 supplement, 10 nM [Leu-15]-gastrin I (Sigma-Aldrich, St. Louis, MO, USA), human recombinant WNT3A (100 ng/ml) (R&D Systems), EGF (100 ng/ml), Noggin (100 ng/ml), R-spondin1 (100 ng/ml), 500 nM A-83-01 (Tocris), 500 uM SB202190 (Sigma-Aldrich), 10 nM prostaglandin E2 (SigmaAldrich), 1 mM N-acetylcysteine (Sigma-Aldrich), 10 mM nicotinamide (Sigma-Aldrich) in Advanced DMEM/F12), and the medium was replaced with InS^{exp} medium every two days. The method of subculture was also performed in the same manner after dissociating the cells inside Matrigel into single cells using TE. InS^{Mat-hIO} isolated from Mat-hIO was cultured by treatment with 1 ng/ml rhIL-2 every day.

### Experimental Example 8. Freezing and thawing of mesenchymal stromal cells

For freezing mesenchymal stromal cells, at 3-5 days after subculture, the cells were treated with TE at 37°C for 3 to 5 minutes in the same manner as the subculture process to be isolated into single cells, followed by washing once with hIO medium. Then, the cells were frozen in freezing medium (Recovery^{™} Cell Culture Freezing Medium, Gibco) and stored long-term in an LN₂ Tank. A warm hIO medium (37°C) was prepared in advance to thaw frozen intestinal stem cells. After rapid thawing of the frozen cells, the cells were washed once with hIO medium, and cultured in hIO medium containing 1x B27 and 100 ng/ml EGF in a pre-gelatin-coated culture dish. Mature intestinal organoid-derived mesenchymal stromal cells could be treated with rhIL-2 (1 ng/ml) while changing the culture medium every two days. Depending on the condition of the cells upon initial thawing, the cells were cultured for 3~7 days and then subcultured in the same manner.

### Experimental Example 9. Quantitative real-time RT-PCR (qRT-PCR)

Total RNA was extracted from cells using the RNeasy kit (Qiagen) and reverse transcribed using the Superscript III cDNA synthesis kit (Invitrogen). qRT-PCR was performed on the 7500 Fast Real-time PCR system (Applied Biosystems, Foster City, CA, USA) according to the known method (Cho et al., Oncotarget 6, 23837-23844, 2015). All experiments were repeated three times, and the CT value of each target gene was calculated using the software provided by the manufacturer. The base sequences of the primers used are shown in Table 1.

**[Table 1]**

| **Gene** | **Primer (Forward)** | **SEQ ID NO:** | **Primer (Reverse)** | **SEQ ID NO:** |
|---|---|---|---|---|
| *GAPDH* | GAA GGT GAA GGT CGG AGT C | 1 | GAA GAT GGT GAT GGG ATT TC | 2 |
| *ACTA2 (αSMA)* | TTC AAT GTC CCA GCC ATG TA | 3 | GAA GGA ATA GCC ACG CTC AG | 4 |
| *VIM* | AGA ACG TGC AGG AGG CAG AAG AAT | 5 | TTC CAT TTC ACG CAT CTG GCG TTC | 6 |
| *DES* | CAG TGG CTA CCA GGA CAA CA | 7 | CTC AGA ACC CCT TTG CTC AG | 8 |
| *PECAM1 ( CD31)* | TGC GGC CAA ATG TTA TTT TC | 9 | TGC ATG GTT TCT GAC ATC GT | 10 |
| *CDH5* | CAG CCC AAA GTG TGT GAG AA | 11 | CGG TCA AAC TGC CCA TAC TT | 12 |
| *ECSCR* | GAG CAT GAG GGA AGA TGC GAC | 13 | TGA CCA CAC CAA CTA GGA TGA | 14 |
| *LYVE1* | GCT TTC CAT CCA GGT GTC AT | 15 | ACC ACG AAT CCA TCT CCA AC | 16 |
| *VIL1* | AGC CAG ATC ACT GCT GAG GT | 17 | TGG ACA GGT GTT CCT CCT TC | 18 |
| *KRT20* | TGG CCT ACA CAA GCA TCT GG | 19 | TAA CTG GCT GCT GTA ACG GG | 20 |
| *LYZ* | AAA   ACC   CCA   GGA GCA GTT AAT | 21 | CAA CCC TCT TTG CAC AAG CT | 22 |
| *SI* | GGT AAG GAG AAA CCG GGA AG | 23 | GCA CGT CGA CCT ATG GAA AT | 24 |
| *LCT* | TCC TCG CCT ACA ACC TCA AC | 25 | GCT GGG ACA CTC CAT TCT CT | 26 |
| *OLFM4* | ACC   TTT   CCC   GTG GAC AGA GT | 27 | TGG ACA TAT TCC CTC ACT TTG GA | 28 |
| *CHGA* | TGA CCT CAA CGA TGC ATT TC | 29 | CTG TCC TGG CTC TTC TGC TC | 30 |
| *MUC5B* | GGG CAC CAA AGA GCA TAG AG | 31 | AAA GCA CAC GCA CGT TGT AG | 32 |
| *SATB2* | CCT CCT CCG ACT GAA GAC AG | 33 | TGG TCT GGG TAC AGG CCT AC | 34 |
| *CA ±* | TGC TTT CAA CGT GGA GTT TG | 35 | CCC CAT ATT TGG TGT TCC AG | 36 |
| *MUC2* | TGT AGG CAT CGC TCT TCT CA | 37 | GAC ACC ATC TAC CTC ACC CG | 38 |
| *MUC13* | CGG ATG ACT GCC TCA ATG GT | 39 | AAA GAC GCT CCC TTC TGC TC | 40 |
| *DPP4* | CAG AAG ACT GGG AAC ATT TGA | 41 | CAC ACT TGA ACA CGC CAC TT | 42 |
| *ASCL2* | CGT GAA GCT GGT GAA CTT GG | 43 | GGA TGT ACT CCA CGG CTG AG | 44 |
| *AXIN2* | GAG TGG ACT TGT GCC GAC TTC A | 45 | GGT GGC TGG TGC AAA GAC ATA G | 46 |
| *OCT4* | GCT CGA GAA GGA TGT GGT CC | 47 | CGT TGT GCA TAG TCG CTG CT | 48 |
| *NANOG* | GCA GAA GGC CTC AGC ACC TA | 49 | AGG TTC CCA GTC GGG TTC A | 50 |
| *FOXA2* | TGG GAG CGG TGA AGA TGG AAG GGC AC | 51 | TCA TGC CAG CGC CCA CGT ACG ACG AC | 52 |
| *SOX17* | CGC TTT CAT GGT GTG GGC TAA GGA CG | 53 | TAG TTG GGG TGG TCC TGC ATG TGC TG | 54 |
| *CDX2* | CTG GAG CTG GAG AAG GAG TTT C | 55 | ATT TTA ACC TGC CTC TCA GAG AGC | 56 |
| *LGR5* | TGC TCT TCA CCA ACT GCA TC | 57 | CTC AGG CTC ACC AGA TCC TC | 58 |
| *ANGPT1* | GGG GGA GGT TGG ACT GTA AT | 59 | GAA TAG GCT CGG TTC CCT TC | 60 |
| *KDR* | CCC ACG TTT TCA GAG TTG GT | 61 | CGG CTC TTT CGC TTA CTG TT | 62 |
| *DEFA5* | CCT TTG CAG GAA ATG GAC TC | 63 | GGA CTC ACG GGT AGC ACA AC | 64 |
| *SLC5A1* | GTG CAG TCA GCA CAA AGT GG | 65 | ATG CAC ATC CGG AAT GGG TT | 66 |
| *CREB3L3* | ATC TCC TGT TTG ACC GGC AG | 67 | GTC GTC AGA GTC GGG GTT TG | 68 |
| *IGF2* | GTG GCA TCG TTG AGG AGT G | 69 | CAC GTC CCT CTC GGA CTT G | 70 |

### Experimental Example 10. Cell and tissue immunofluorescence staining

Immunofluorescence was performed according to the known method (Kwak et al., Biochemical and biophysical research communications 457, 554-560, 2015). Specifically, hPSCs and definitive endoderm cells were fixed with 4% paraformaldehyde (PFA) and permeabilized with PBS containing 0.1% Triton X-100.

The hIO and tissues were fixed, cryoprotected with sucrose, and frozen using optimal cutting temperature (OCT) compound (Sakura Finetek, Tokyo, Japan). Then, frozen sections were cut into 10 µm using a cryostat microtome at -20°C and permeabilized with PBS containing 0.1% Triton X-100 for immunofluorescence analysis.

Specifically, after blocking with 4% BSA, the cells were reacted with the primary antibodies overnight at 4°C. Then, the cells were reacted with the secondary antibodies for 1 hour at room temperature. The primary antibodies used are listed in Table 2. DAPI was added to visualize nuclei. The slides were observed using an EVOS FL Auto2 (ThermoFisher) and an Axiovert 200M microscope (Carl Zeiss, Gottingen, Germany) or a fluorescence microscope (IX51, Olympus, Japan).

**[Table 2]**

| **Antibody** | **Catalog No.** | **Manufacturer** | **Dilution Ratio** |
|---|---|---|---|
| anti-α-SMA | A5228 | Sigma | 1:500 for IHC |
| anti-hCD31 | MA5-15336 | Thermo Scientific | 1:400 for IHC |
| anti-PECAM-1 | sc-376764 | Santa Cruz | 1:50 for IHC |
| anti-VE-Cadherin | AF938 | R&D systems | 5-15 µg/mL |
| anti-VEGF | sc-7269 | Santa Cruz | 1:50 for IHC |
| anti-E-Cadherin | 610182 | BD Biosciences | 1:200 for IHC |
| anti-E-Cadherin | AF648 | R&D systems | 1:500 for IHC |
| anti-E-Cadherin | Ab1416 | Abcam | 1:100 for IHC |

| | | | |
|---|---|---|---|
| *IHC: Immunohistochemistry | | | |

### Experimental Example 11. RNA sequencing and RNA quantification

For RNA sequence determination and quantification, first, RNA samples were prepared with an RNA Integrity Number (RIN) value of 7.5 or more through the Agilent 2100 Bioanalyzer system (Agilent Biotechnologies, Palo Alto, USA), and mRNA libraries were prepared through the Illumina TruSeq kit, followed by sequencing using Illumina HiSeq2500 machines (Illumina, San Diego, CA, USA). A sequencing quality was determined through a FastQC package, and a trimmed read length of 50 bases or less was excluded. Then, mapping was performed through HISAT2 (v2.0.5), with reference to the hg19 for the human genome information. Differentially expressed genes (DEGs) between samples were analyzed using Cuffquant and Cuffnorm (Cufflinks v2.2.1).

### Experimental Example 12. Bioinformatic analysis

Bioinformatic analysis was performed using IPA analysis software (Ingenuity systems, Redwood City, CA, USA), protein analysis through evolutionary relationships (PANTHER, http://www.pantherdb.org) database, and DAVID bioinformatics resource 6.7 (http://david.abcc.ncifcrf.gov). Additionally, Gene Ontology (GO)/pathway to identify cell types was analyzed using EnrichR (https://maayanlab.cloud/Enrichr/). Functionally grouped Gene Ontology (GO)/pathways were analyzed using the Cytoscape software platform (version 3.3.0, http://www.cytoscape.org/what_is_cytoscape.html) in conjunction with ClueGO plug-in (Version 2.2.5, http://apps.cytoscape.org/apps/cluego) .

### Experimental Example 13. Single cell RNA sequencing and analysis

Sequencing data were processed using the 10x Genomics pipeline CellRanger (version 7.0.0) and a filtered gene-barcode matrix was generated using default parameters. The filtered gene-barcode matrix was used as input for downstream analysis using the R package Seurat (version 4.1.1). Cells with at least 200 genes and 9000 or more genes and cells with a mitochondrial fraction greater than 25% were removed to eliminate potential low-quality cells and doublets. Afterwards, SCtransform (version 0.3.3) was performed to normalize each dataset, and sample integration was prepared via standard correlation analysis to identify the largest source of variation preserved across all samples. Highly variable genes (HVGs) were identified using the FindVariableFeatures function, and then multiple sample data were integrated using the FindIntegrationAnchors and Integrateddata functions. After integrating the samples, primary components (PCs) were determined, and the first 50 PCs were used for cell clustering using the K-nearest neighbor graph with a clustering resolution of 0.8. Cell clusters were visualized using UMAP. To determine differentially expressed genes between conditions in each cluster, the FindMarkers function was used with the Wilcoxon Rank Sum test (min.pct=0.25, logfc.threshold=0.25) and p-value adjustment by Bonferroni correction.

### Experimental Example 14. Enzyme-linked immunosorbent assay (ELISA)

Mesenchymal stromal cells isolated from immature or mature intestinal organoids were cultured for 5 days, and the conditioned medium was collected to measure factors secreted from the cells. The level of VEGF, a vascular secretory factor, was measured according to the published experimental method using the VEGF Human ELISA Kit (R&D Systems), and the measured value was normalized to the total protein concentration of the total cells.

### Experimental Example 15. Human XL cytokine array

Mesenchymal stromal cells isolated from immature or mature intestinal organoids were cultured for 5 days, and the culture medium was collected to measure factors secreted from the cells. The measurement was performed according to the published experimental method using the Human XL Cytokine Array Kit (RY022B, R&D Systems), and the measured values were analyzed by measuring the density of the Dot blot using FujiFilm LAS-3000.

### Experimental Example 16. Human Angiogenesis Array

Mesenchymal stromal cells isolated from immature or mature intestinal organoids were cultured for 5 days, and the culture medium was collected to measure factors secreted from the cells. The measurement was performed according to the published experimental method using the Human Angiogenesis Array Kit (ARY007, R&D Systems), and the measured values were analyzed by measuring the density of the Dot blot using FujiFilm LAS-3000.

### Experimental Example 17. Histological (Hematoxylin & Eosin, H&E) staining experiment

For histopathological analysis, colon tissues were fixed with 10% formalin in transverse, Swiss-roll, and longitudinal forms, and cultured in 10%, 20%, and 30% sucrose solutions in sequence for cryopreservation. Then, the tissues were embedded in Tissue-Tek^{®} O.C.T compound (Sakura^{®} Finetek, Tokyo, Japan), cryosectioned at 5 - 10 µm thickness, and adhered to slide glasses, followed by H&E staining according to the known method. The slides were observed through an optical microscope (BX53F, Olympus, Japan).

### Experimental Example 18. PAS/Mucicarmine staining experiment

Mature functional goblet cells were identified in mature intestinal organoids using a technique that stains mucin secreted by mature goblet cells. First, the control group and mature intestinal organoids were fixed with 4% paraformaldehyde and then further fixed with 10%, 20%, and 30% sucrose solutions. Then, the sample tissues were frozen using OCT, cryosectioned at 10 µm thickness using a cryostat microtome, and adhered to slide glasses, followed by staining using the Alcian Blue PAS Stain Kit. The slides were observed through an optical microscope (BX53F, Olympus, Japan).

### Experimental Example 19. EDTA-induced intestinal epithelial injury model

EDTA-induced intestinal epithelial injury model was created using the knkown method (Cell Stem Cell 22(2):171-176 (2018). NSG or NIG mice (male, 6-12 weeks old) were fed a standard diet without fasting, and maintained at a constant temperature (20-22°C) on a 12-hour day/night cycle. The mice were anesthetized with an intraperitoneal injection of 250-500 mg/kg Tribromoethanol (Avertin). The colon of mice was washed with PBS by inserting a thin catheter to remove the contents in the intestine. Then, 200 ul of 50°C 250 mM EDTA/PBS was slowly injected into the intestinal lumen for 2 minutes to injure the anal margin, rectum. After 2 minutes of exposure to EDTA/PBS, the intestinal lumen was washed with PBS for 1 minute. Then, an electric toothbrush equipped with a soft interdental brush (EW-DL22, EW0945, Panasonic Holdings Corp., Japan) was used to create an epithelial abrasion for 1 minute. Here, the brush head was inserted 1.5 cm into the colon to scrape the intestinal epithelial surface in a circular motion, and after 1 minute, success was confirmed if an isolated crypt was observed when washing the brush with PBS. The intestinal lumen was then washed again with PBS.

### Experimental Example 20. Inflammatory bowel disease model (DSS-induced colitis model)

NSG or NIG mice (male, 6-12 weeks old) were fed a standard diet without fasting, and maintained at a constant temperature (20-22°C) on a 12-hour day/night cycle. The DSS-induced colitis model was administered drinking water containing 5% (w/v) DSS (36-50 kDa; MP Biomedicals, Hampton, NH, USA) for 5-7 days. Mice were administered drinking water to remove DSS during the 3-day digestion period and prepared for transplantation. The DSS-induced inflammatory bowel model was subjected to daily measurements of body weight, stool consistency, and bleeding parameters to assess disease activity index (DAI).

### Experimental Example 21. Colonic transplantation

For xenotransplantation, organoids grown in Matrigel for 10 days were isolated from Matrigel and washed with cold hIO medium. 3D cells, hIO, hCO InS^{hIO} were cut into a certain size, 200 um x 200 um, using a chopper before transplantation, and suspended in Matrigel/Advanced DMEM/F12 (1 : 10) or Fibrin. A 100 ul suspension contained 1 to 2 x 10⁶ cells. In addition, organoid-derived mesenchymal stromal cells were isolated into single cells using TE in the same manner as the passage culture method, and then prepared so that the 100 ul suspension contained 1 to 2 x 10⁶ cells. When co-transplanting organoids or 3D intestinal stem cells and mesenchymal stromal cells, the cells were transplanted at a 1 : 1 ratio, resulting in a total of 2 x 10⁶ cells/100 ul. Prior to transplantation, mice were not fasted, fed a standard diet, and anesthetized with an intraperitoneal injection of 250-500 mg/kg Tribromoethanol (Avertin). The colon of mice was washed with PBS by inserting a thin catheter to remove the contents in the intestine. A 200 ul pipette, a soft catheter, or an endoscopic catheter was used to conduct injection into the lumen of the colon of the diseased mouse. When the cells were suspended in Matrigel and injected, the anal verge was attached with adhesive (Vetbond Tissue Adhesive) (3M, USA) for 8-12 hours to promote the retention of transplanted cells in the intestine. Note that the Anal verge step is not required when utilizing Cell + Fibrin / Thrombin ECM. At each observation period, the mice were humanely euthanized and the xenografts were isolated for analysis. The xenograft of colon tissue was confirmed by observation with an inverted fluorescence dissecting microscope (SZX16, Olympus).

### Experimental Example 22. Colonoscopy

To determine the inflammation level and environment in the intestine, mice were anesthetized with intraperitoneal injection of Avertin before endoscopy. The colon of mice was washed with PBS by inserting a thin catheter to remove the contents in the intestine. Medical gas was administered using an air pump, and a rigid HOPKINS endoscope (Karl Storz) was carefully inserted into the rectum, allowing for the recording of endoscopic images.

### Experimental Example 23. Renal capsule transplantation

NSG mice (8-12 weeks old) were housed in a standard animal maintenance facility. For xenotransplantation, organoids (P1) cultured for 10 days post passage in hIO medium containing 1X B27, 100 ng/ml EGF were isolated from matrigel and washed with cold hIO medium. The organoids were stored in cold hIO medium. Before transplantation, mice were fed a standard diet without fasting and were anesthetized with 2% isoflurane (Butler Schein) by inhalation. The posterior ribs on both sides of the mouse were cleaned with isopropyl alcohol and povidine-iodine, and then incised. The kidneys were exposed and one Cont-hIO and one Mat-hIO were transplanted subrenally in each kidney. The kidney was then returned to the abdominal cavity, and the double layer of skin was closed with suture. After 8 weeks of engraftment, the mice were humanely euthanized, and the renal tissue xenograft was observed using an inverted fluorescence dissecting microscope (SZX16, Olympus).

### Experimental Example 24. Analysis of cell morphological changes of intestinal stem cells depending on culture medium and growth factor

Intestinal stem cells derived from human intestinal organoids (hIO) were isolated using the known method (Int J Stem Cells. 28;15(1):104-111 (2022)). Before cell isolation, cells were coated with 1% Matrigel in Advanced DMEM/F12 (Gibco) for 1 hour, and as previously described, immature (Cont-hIO) intestinal organoids were isolated into single cells using TE for 5 min at 37°C and centrifuged at 1500 rpm for 5 minutes. For the first 2 days, the cell pellets were cultured by treatment with 10 µM Y-27632 (Tocris) and 1 µM Jagged-1(AnaSpec, Fremont, CA, USA), and then cultured in ISC^{3D-hIO} medium (which is the same as InS^{exp} medium (2 mM l-glutamine, 15 mM Hepes buffer, 1X B27 supplement, 10 nM [Leu-15]-gastrin I (Sigma-Aldrich, St. Louis, MO, USA), human recombinant WNT3A (100 ng/ml) (R&D Systems), EGF (100 ng/ml), Noggin (100 ng/ml), R-spondin1 (100 ng/ml), 500 nM A-83-01(Tocris), 500 uM SB202190 (Sigma-Aldrich), 10 nM prostaglandin E2 (SigmaAldrich), 1 mM N-acetylcysteine (Sigma-Aldrich), 10 mM nicotinamide (Sigma-Aldrich) in Advanced DMEM/F12). After that, the medium was replaced with ISC^{3D-hIO} medium every two days. The method of subculture was also performed in the same manner after dissociating the attached cells on the surface into single cells using TE. To confirm the cell morphological changes depending on culture medium (MSC medium: MSC^{3D-hIO} medium, ISC medium: ISC^{3D-hIO} medium) and growth factor (100 ng/ml, BMP4) in Cont-CM, Mat-CM, and hMSC-CM, the cells were treated and observed for 1 to 3 days.

### Experimental Example 25. Cresyl violet (CV) staining experiment

Intestinal stem cells (ISC) were washed twice with PBS and fixed with 10% formalin for 10 minutes. After removing the fixative, 0.5% cresyl violet solution was added and the cells were incubated for 10 minutes. Then, the cells were washed with PBS until no dye was released and observed.

### Experimental Example 26. Real-time cell metabolism analysis

To measure the oxygen consumption rate (OCR) and extracellular acidity rate (ECAR), 20,000 ISC cells were cultured in a Seahorse XF Pro M Cell Culture Microplate for 2 days prior to measurement. The day before the analysis, the cells were exposed to the specific medium for 12 hours. On the day of the analysis, the culture medium was removed and the cells were washed once with warm assay medium (Agilent Seahorse XF DMEM Medium pH 7.4, 103575-100). To the assay medium, 1 mM glutamine, 1 mM pyruvate, and 2 mM glucose were added to measure oxygen consumption rate, and 1 mM glutamine was added to measure the extracellular acidity rate. The culture plate was placed in a 37°C CO₂ incubator for 1 hour, and the oxygen consumption rate and extracellular acidity rate were measured using a Seahorse XFe96 Pro Analyzer according to the manufacturer's instructions. For oxygen consumption rate measurement, 1.5 µM oligomycin, 5 µM FCCP, 1 µM rotenone + 1 µM antimycin A were sequentially added at each time point, and for extracellular acidity rate measurement, 1.5 µM oligomycin, 100 mM glucose, and 50 mM 2-D-glucose were sequentially added at each indicated time point. The measured values were normalized through protein quantification and analyzed.

### Experimental Example 27. HUVEC tubule formation assay

50 ul Matrigel (minimum protein concentration 10 mg/ml) was coated on a 96-well culture plate and the cells were cultured in a CO₂ incubator at 37°C for 30 minutes. HUVECs (C2517A, Lonza) grown to 70-80% in EGM-2 Growth medium (CC-3162, Lonza) were isolated into single cells by treatment with TE, and the cells were diluted with growth factors (100 ng/ml, IGF2) and 2% CM (Cont-CM, Mat-CM) for each condition in EBM-2 (CC-3156, Lonza) medium supplemented with 2% FBS and 35 ng/ml bFGF, seeded on Matrigel-coated plates at 2-2.5 X 10⁴ cells/well, 200 ul, and observed at 0 and 6 hours.

### Experimental Example 28. HUVEC wound healing assay

24-well culture plates were coated with 0.2% gelatin and the cells were cultured in a CO₂ incubator at 37°C for 1 hour. HUVEC (C2517A, Lonza) grown to 70-80% in EGM-2 Growth medium (CC-3162, Lonza) were isolated into single cells by treatment with TE, and the cells were diluted with EGM-2 growth medium, seeded at 0.5-1 X 10⁵ cells/well, and cultured for 1 day. On day 2, the cells were cultured for 1 day (70-80% growth) in EBM-2 (CC-3156, Lonza) medium supplemented with 2% FBS and 35 ng/ml bFGF, and observed at 0 and 36 hours after scratching using a 200 µl tip on day 3 and adding growth factors for each condition (100 ng/ml, IGF2) and 2% CM (Cont-CM and Mat-CM).

### Experimental Example 29. Statistical analysis

All in vitro experimental results are expressed as mean ± standard error of the mean (s.e.m.), with at least three replicates. Quantitative analysis was performed using ImageJ software. P-values were determined using two-tailed Student's t-test or one-way ANOVA. Analysis of the length of crypt depth was determined using Welch's t-test. All analyses of statistical significance were calculated relative to the control group unless otherwise noted.

### Example 1. Isolation and culture of mesenchymal stromal cells from hPSC-derived hIO for transplantation

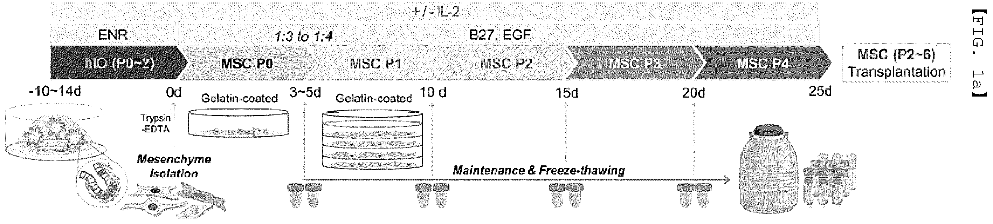

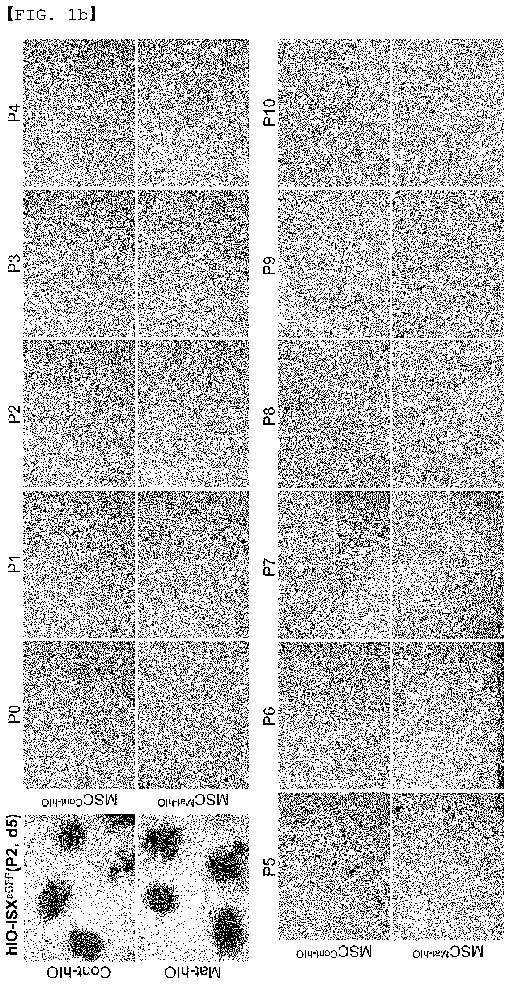

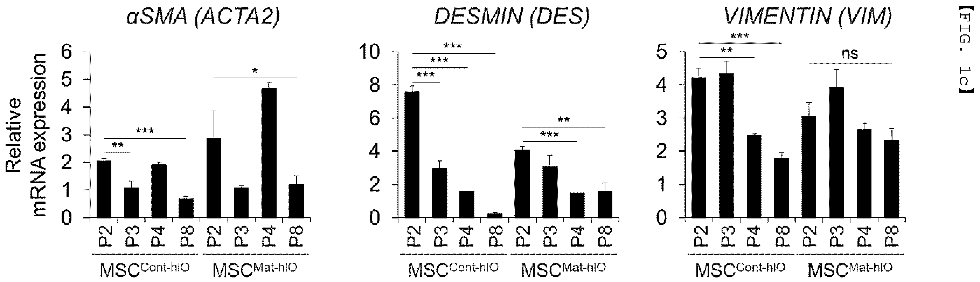

As described above, hIOs were produced from hPSCs containing hESC lines or non-integrating-hiPSC lines, using the hIO differentiation protocol. Then, mesenchymal stromal cells were isolated from mature organoids co-cultured with rhIL-2 (1 ng/ml) from P0 and conventional immature organoids P0 to P2, followed by freezing and thawing process during passage culture, which was shown as a schematic diagram (FIG. 1a). Mesenchymal stromal cells (P0) isolated from Cont-hIO and Mat-hIO (P2) were culturable in hIO medium containing 1X B27 and 100 ng/ml EGF and were maintained until P10 (FIG. 1b). In addition, it was confirmed that representative markers of mesenchymal stromal cells (αSMA, VIM, and DES) were expressed during passage culture (FIG. 1c).

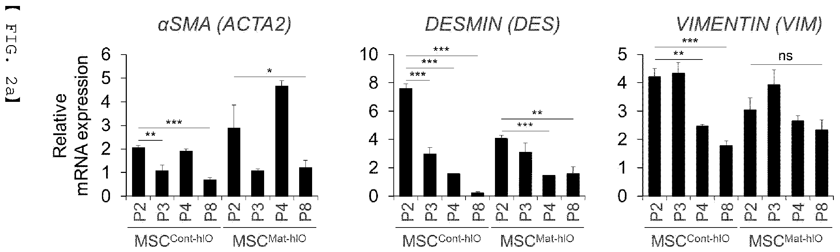

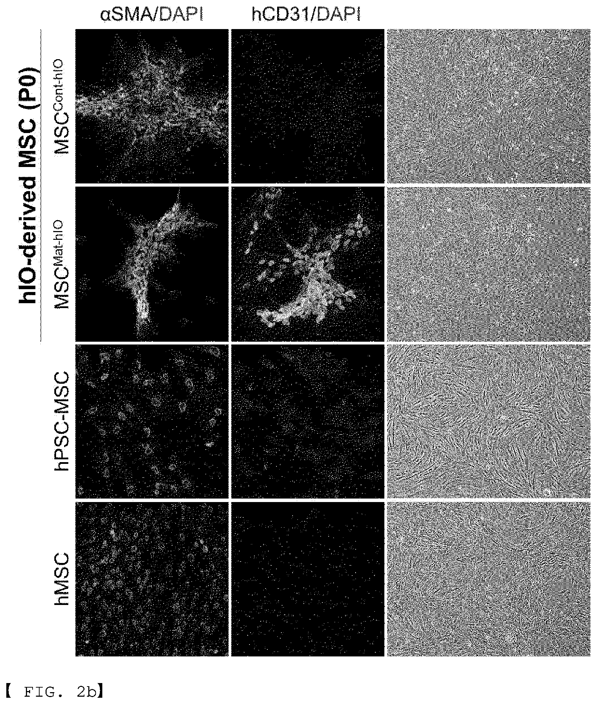

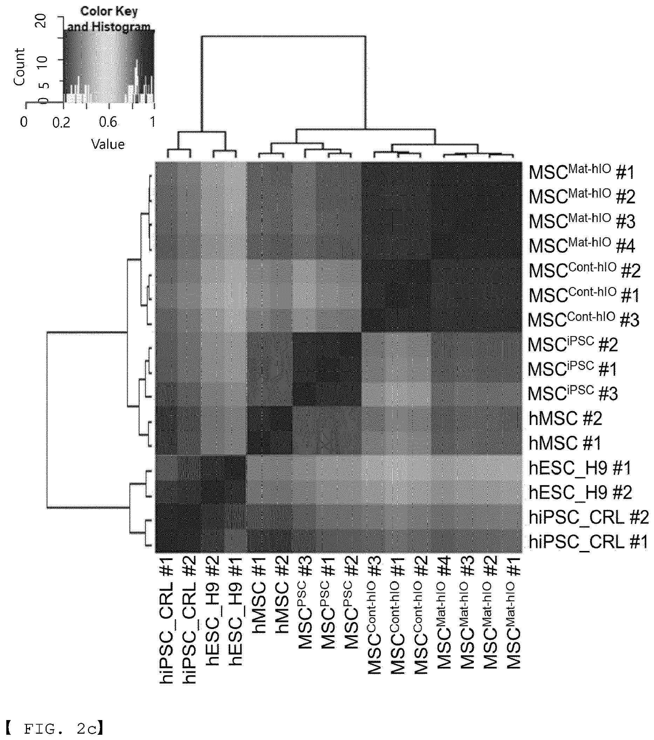

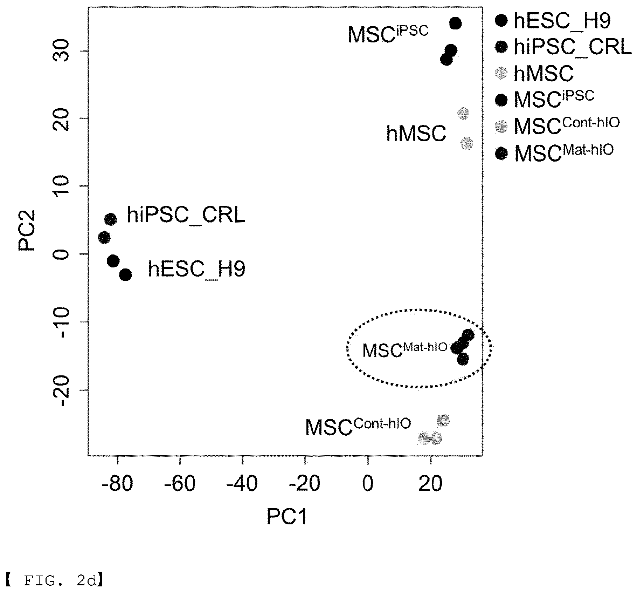

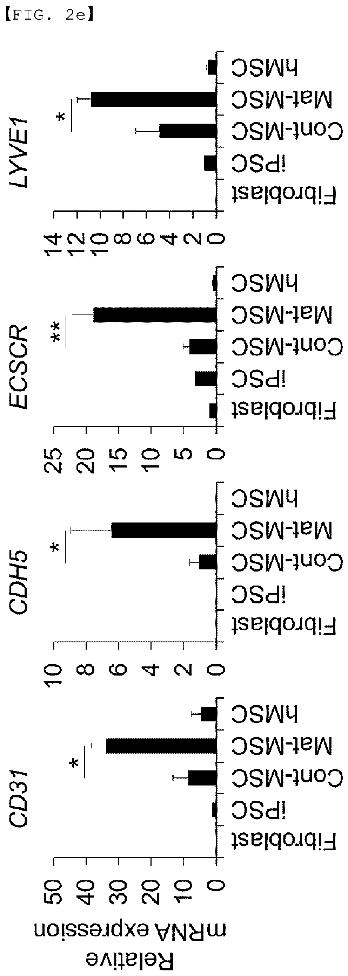

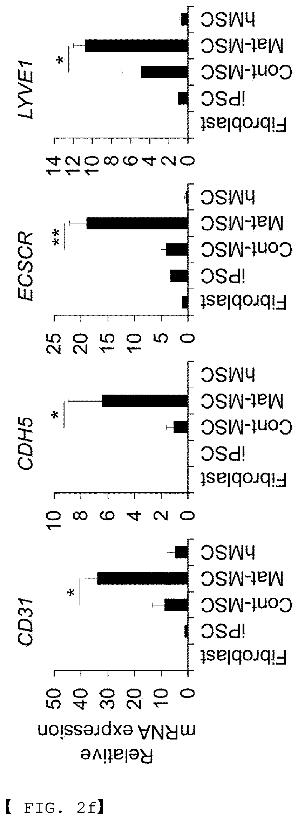

To closely examine the characteristics of the isolated mesenchymal stem cells, pluripotent stem cells (hESC-H9, and hiPSC-CRL), commercially available human mesenchymal stem cells (hMSC) and mesenchymal stem cells differentiated from induced pluripotent stem cells (MSC^{iPSC}) were used as control groups. Expression of αSMA, a marker of smooth muscle cell or pericyte, was confirmed in all cells through immunofluorescence staining. However, in mature intestinal organoid-derived mesenchymal stromal cells, expression of the human vascular endothelial cell marker hCD31⁺ was observed, along with αSMA (FIG. 2a). For genome information analysis, RNA sequencing was performed to select differential genes between groups through DEG analysis while showing similarities between samples (FIG. 2b). MDS plot confirmed the difference between Mat-hIO-derived MSCs and Cont-hIO-derived MSCs (FIG. 2c), and GOterm analysis of EnrichR identified endothelial cell type characteristics (FIG. 2d). The difference between the two groups was validated by qPCR to confirm the expression of angiogenic factors (CD31, CDH5, ECSCR, and LYVE1), and by ELISA to detect the vascular secretory factor VEGF, revealing a novel characteristic of Mat-hIO-derived MSCs (FIGS. 2e and 2f).

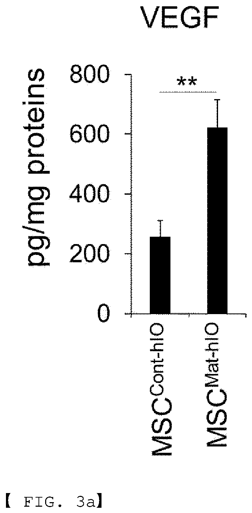

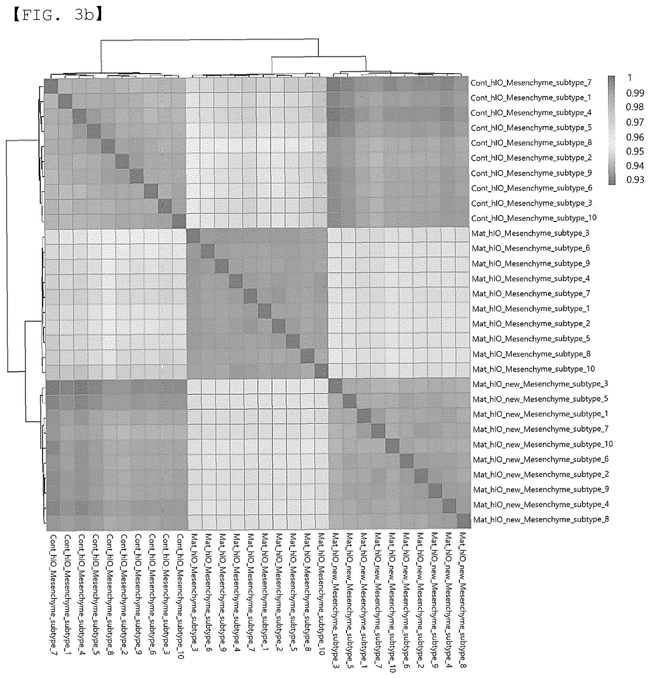

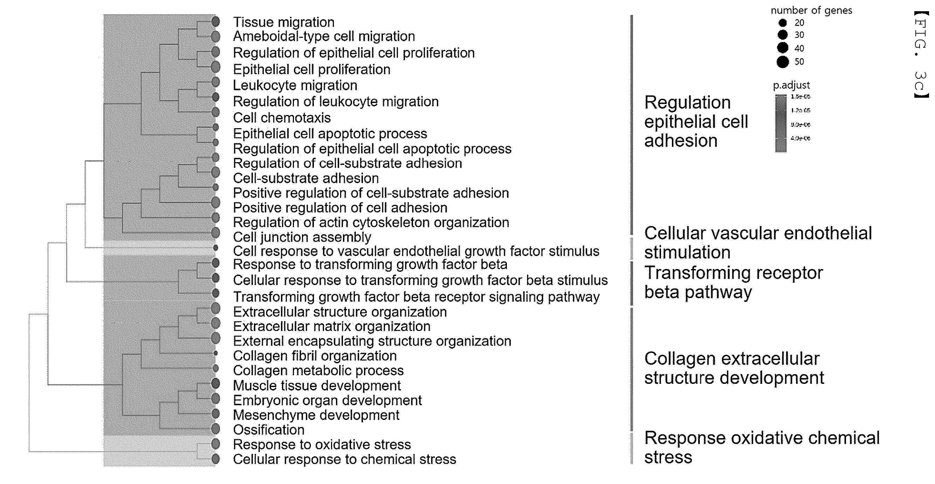

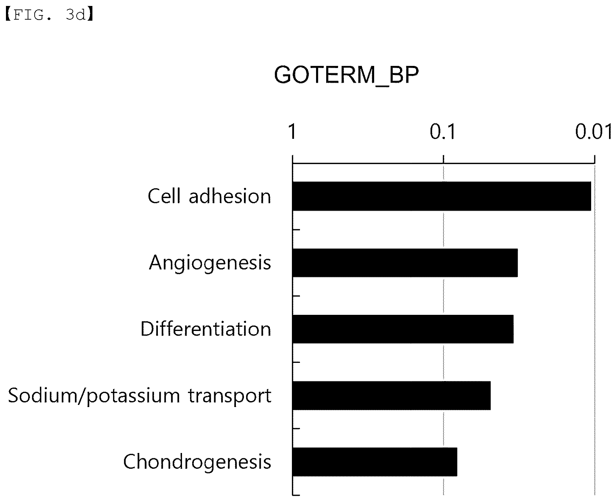

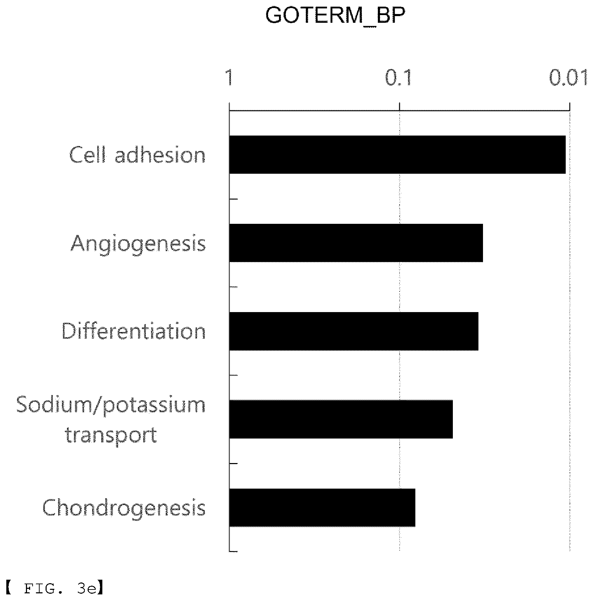

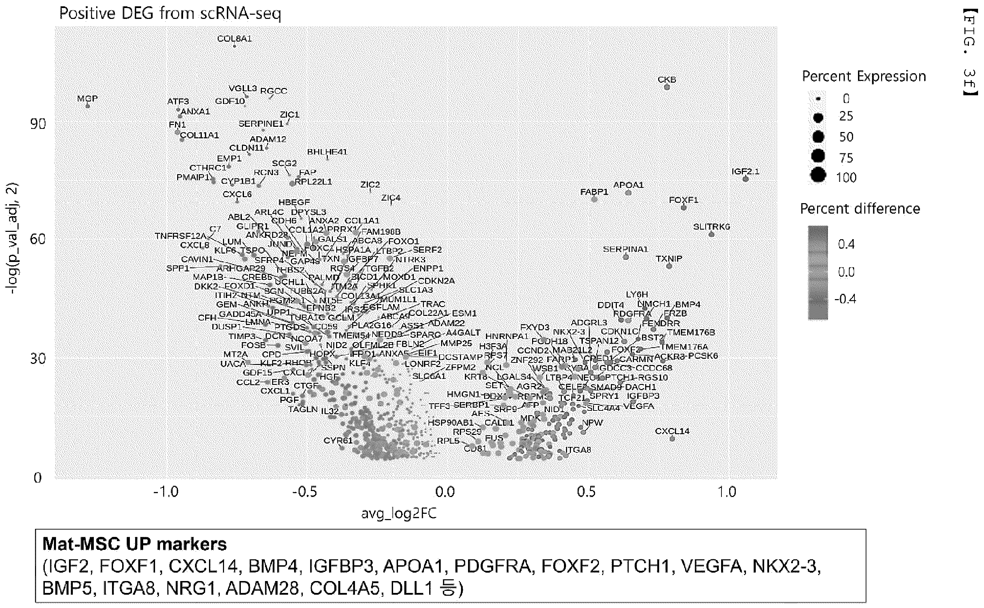

These results confirm that Mat-hIO-derived MSCs have different characteristics from immature organoid-derived MSCs, commercially available human MSCs, and MSCs differentiated from induced pluripotent stem cells. For a closer analysis at the gene level, single cell RNAseq was performed to analyze one kind of Cont-hIO and two kinds of Mat-hIO, identifying that the Mesenchyme cluster was composed of a total of seven groups (8_0, 8_1, 8_2, 8_3, 8_4, 8_5, and 8_6), where the remaining groups except for 8_0 and 8_6 confirmed the mesenchymal cell characteristics of Mat-hIO (FIG. 3a). Differentially Expressed Genes (DEGs) were selected from the Mesenchyme group of Mat-hIO compared to Cont-hIO, and the up-regulated Gene ontology biological process (GOBP) included terms related to Epithelial cell adhesion or cellular vascular endothelial stimulation. In addition, GOTERM_BP analysis of DEGs in DAVID (https://david.ncifcrf.gov/summary.jsp) also confirmed that cell adhesion, angiogenesis, differentiation, mesenchyme development, etc., were increased in Mat-hIO compared to Cont-hIO (FIGS. 3b to 3e). In addition, the volcano plot of DEGs in the mesenchymal stromal cell cluster of Mat-hIO showed that the up-regulated genes in the mesenchymal stromal cells of Mat-hIO included IGF2, FOXF1, CXCL14, BMP4, IGFBP3, APOA1, PDGFRA, FOXF2, PTCH1, VEGFA, NKX2-3, BMP5, ITGA8, NRG1, ADAM28, COL4A5, and DLL1, as representative examples (FIG. 3f). The detailed gene list is shown in Table 3.

**[Table 3]**

| | **p_val** | **avg_log2FC** |
|---|---|---|
| **IGF2.1** | 9.66E-28 | 1.059351744 |
| **FOXF1** | 1.50E-25 | 0.839701803 |
| **CXCL14** | 7.19E-08 | 0.798390905 |
| **BMP4** | 4.03E-17 | 0.781237745 |
| **IGFBP3** | 9.76E-11 | 0.659753907 |
| **APOA1** | 1.09E-26 | 0.642983424 |
| **PDGFRA** | 6.20E-17 | 0.642415099 |
| **FOXF2** | 1.60E-14 | 0.56758628 |
| **PTCH1** | 1.54E-12 | 0.551034596 |
| **VEGFA** | 1.33E-10 | 0.491855898 |
| **NKX2-3** | 1.03E-13 | 0.487365795 |
| **BMP 5** | 9.30E-09 | 0.473039399 |
| **COL9A1** | 2.67E-08 | 0.432872887 |
| **ITGA8** | 1.39E-06 | 0.419933776 |
| **NRG1** | 6.00E-08 | 0.415831905 |
| **ADAM28** | 1.17E-08 | 0.414366444 |
| **COL4A5** | 2.14E-06 | 0.397617201 |
| **DLL1** | 8.21E-08 | 0.381424539 |

### Example 2. Comparison of immature and mature hIO-derived mesenchymal stromal cell factors

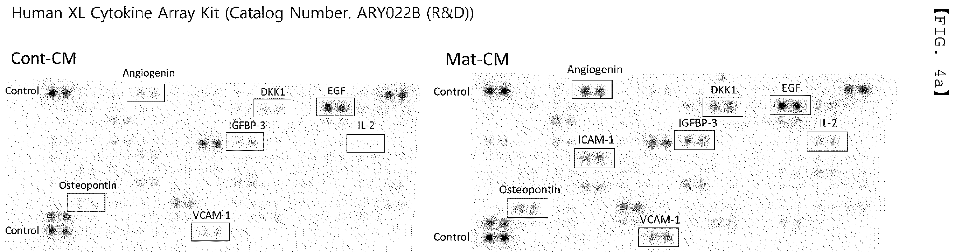

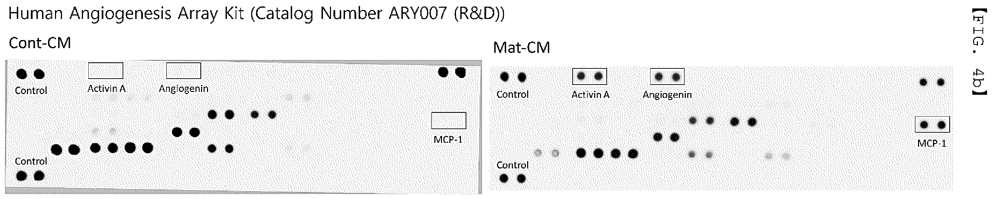

Based on the scRNAseq analysis results, the differences in gene levels (DEGs) between Mat-hIO-derived MSCs and Cont-hIO-derived MSCs were confirmed, and to identify secreted protein factors, Cont-MSC and Mat-MSC cultures were collected, and cytokines and vascular-related factors were identified using an Array kit. Angiogenin, DKK1, EGF, Osteopontin, ICAM-1, IGFBP-3, and VCAM-1 were confirmed to increase through a cytokine array kit (Human XL Cytokine Array kit). Angiogenin, Activin A, and MCP-1 were confirmed to significantly increase through a vascular factor array kit (Human Angiogenesis Array kit) (FIG. 4).

### Example 3. Change in ISC morphology depending on mature hIO-derived mesenchymal stromal cell-specific factors

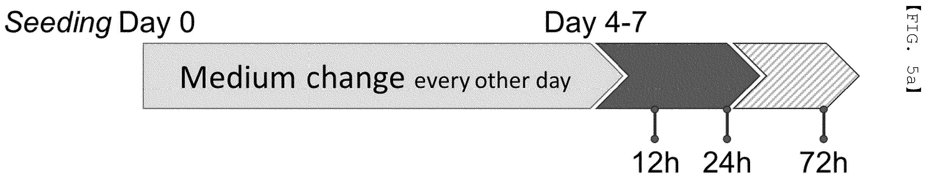

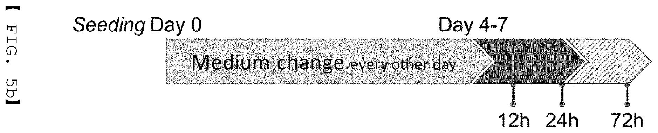

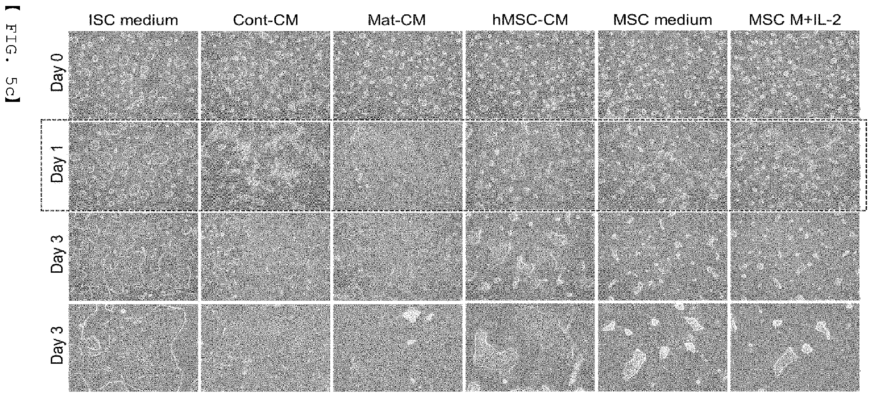

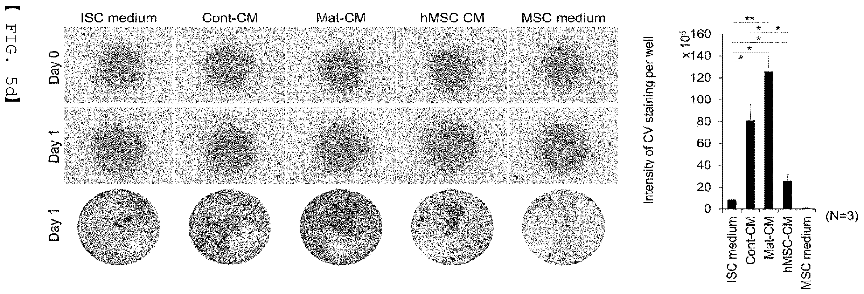

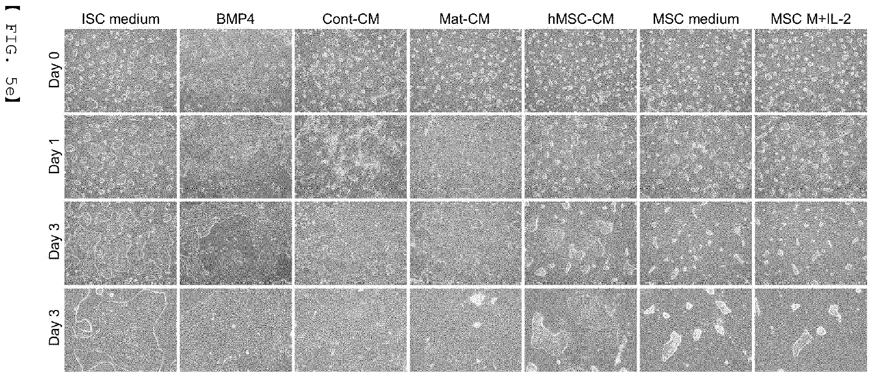

Based on the results of scRNAseq analysis, factors affecting intestinal stem cells (ISCs) were identified from differences in gene levels of Cont-hIO-derived MSCs versus Mat-hIO-derived MSCs. In addition, actual changes in ISC morphology were observed by treatment with culture media of mesenchymal stromal cells (Cont-CM, Mat-CM, and hMSC-CM). In the culture medium of hIO-derived mesenchymal stromal cells, changes in cell morphology and distribution were clearly observed on day 3, and in particular, Mat-CM induced rapid changes on day 1. CV staining showed on day 1 that Mat-CMs showed not only the morphological changes of cells but also a rapid increase in distribution compared to Cont-CM (FIGS. 5a to 5c). In order to identify the factor that particularly affects ISC among the secreted factors, growth factor (BMP4) that showed a significant increase at the gene level was treated, and as a result, the cell characteristics when treated with BMP4 were confirmed to be similar to those when treated with culture medium of hIO-derived mesenchymal stromal cells (FIG. 5d). In addition, Cont-CM+ BMP4 co-treatment showed an increase in cell distribution and faster changes in morphology compared to Cont-CM single treatment (FIG. 5e).

### Example 4. Change in ISC cell metabolism depending on mature hIO-derived mesenchymal stromal cell-specific factors

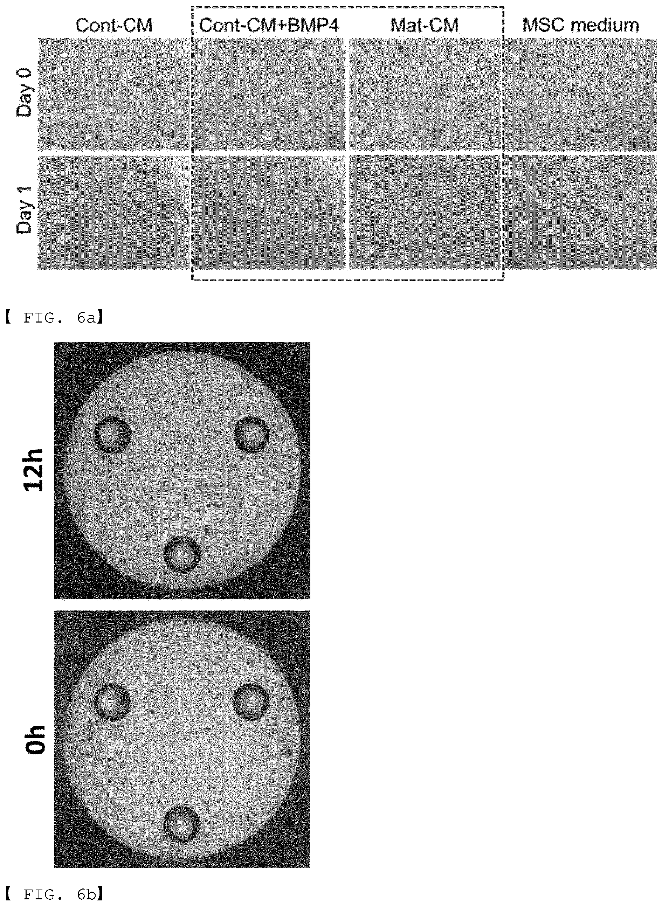

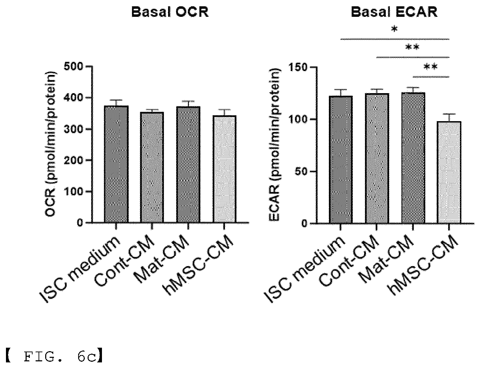

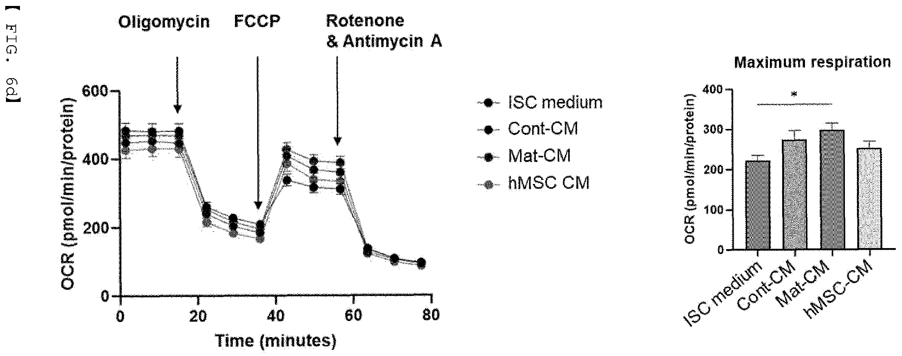

Basal oxygen consumption rate (Basal OCR) and extracellular acidic rate (Basal ECAR) showed no difference between ISCs cultured in ISC medium and ISCs cultured in Cont-CM and Mat-CM, but maximal respiration was enhanced 1.36-fold in the group treated with mature hIO-derived mesenchymal stromal cell culture medium (Mat-CM) compared to ISC medium (FIGS. 6a to 6c). It was confirmed that the glycolytic capacity and glycolytic reserve were improved by 1.18 times and 1.72 times, respectively, in the Mat-CM group (FIG. 6d). This means that ISCs were converted into cells (differentiated cells) capable of synthesizing large amounts of biogenic unit substances and ATP.

### Example 5. Tubule formation of HUVEC depending on mature hIO-derived mesenchymal stromal cell-specific factors

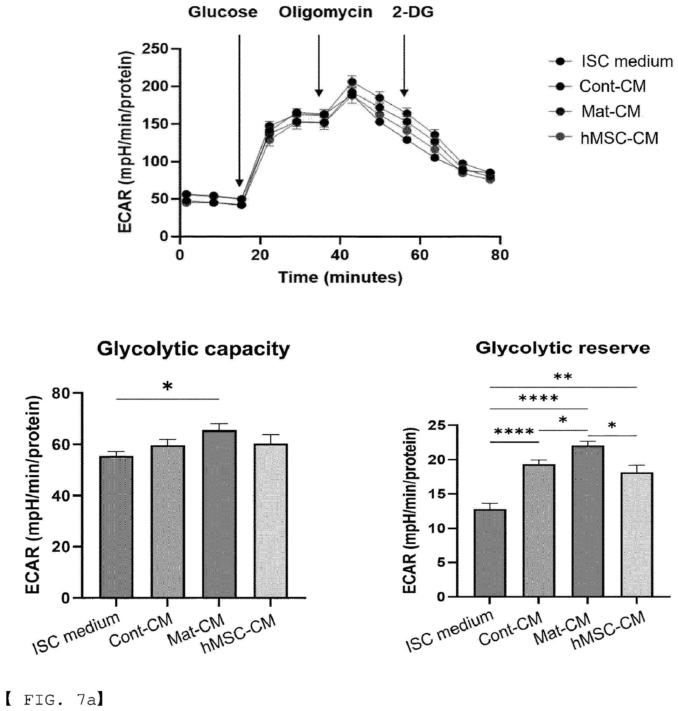

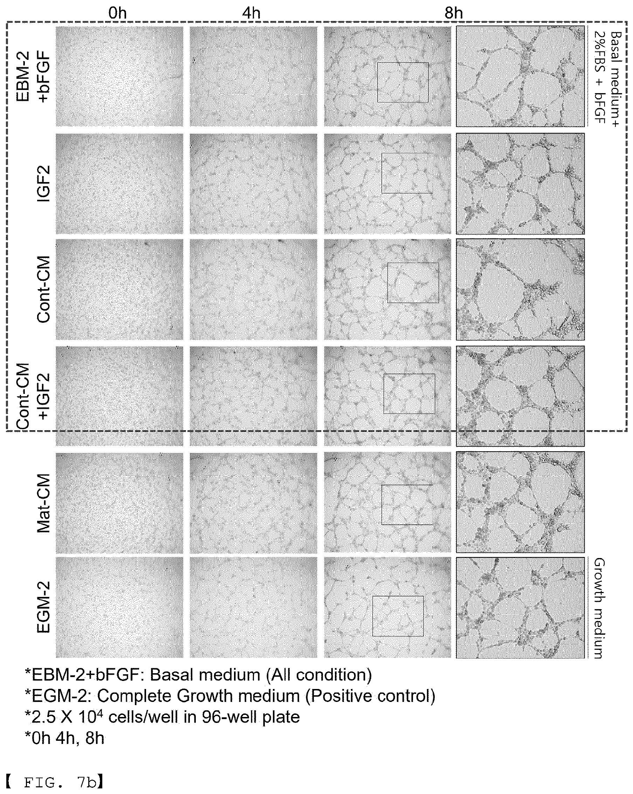

Based on the results of scRNAseq analysis, a tubule formation assay was performed by treating a factor (IGF2) that affects endothelial cells (HUVEC) at the gene level difference between Mat-hIO-derived MSCs and Cont-hIO-derived MSCs. When seeded at 25,000 cells per well and observed for 4 hours and 8 hours, respectively, IGF2 treatment showed rapid tubule formation starting at 4 hours compared to EBM-2+bFGF (basal medium), with a higher rate of complete tubule formation at 8 hours (FIG. 7a). To confirm the efficacy of IGF2 factor, IGF2 was added to Cont-CM and compared. It was verified that IGF2-added medium formed complete tubules similarly to the Mat-CM treatment than the Cont-CM single treatment, and the formation was better than the EGM-2 medium of HUVEC cells (FIG. 7a). When quantitative evaluation was performed using ImageJ, it was verified that the number of junctions and segments significantly increased in Cont-CM+IGF2 and Mat-CM compared to Cont-CM (FIG. 7b). Compared to EBM-2+IGF2 (Basal medium), total length was significantly increased in Cont-CM, Cont-CM+IGF2, and Mat-CM.

### Example 6. Wound healing (migration) of HUVEC depending on mature hIO-derived mesenchymal stromal cell-specific factors

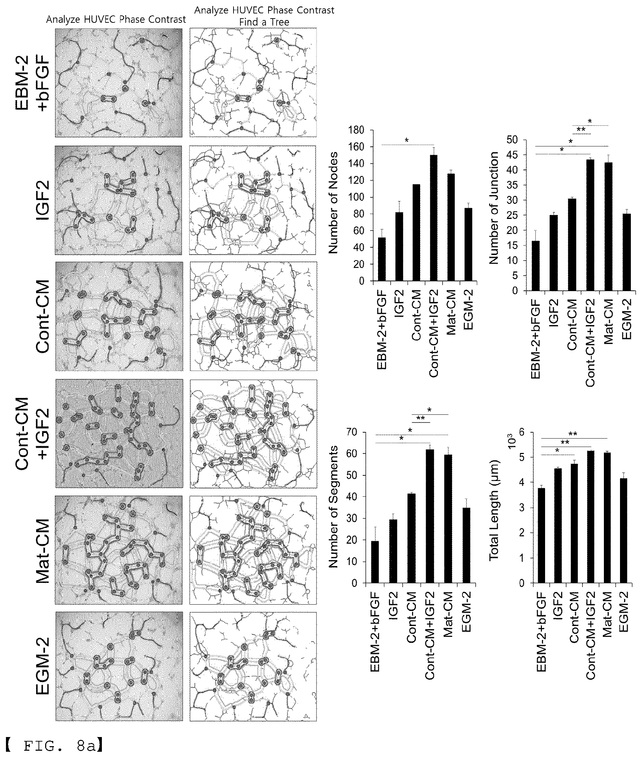

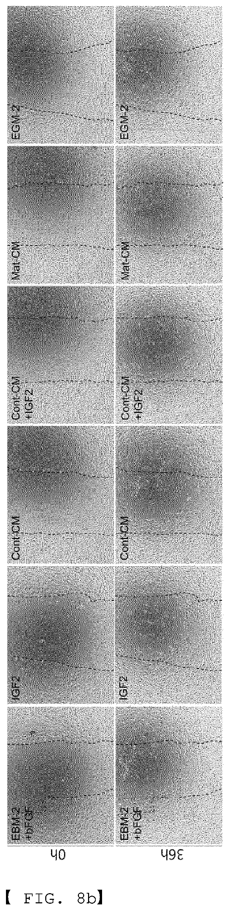

Based on the results of scRNAseq analysis, a wound healing assay was performed by treating a factor (IGF2) that affects endothelial cells (HUVEC) at the gene level difference between Mat-hIO-derived MSCs and Cont-hIO-derived MSCs. When observed at 0 hour and 36 hour after scratching using a 200 µl tip, the wound size was significantly reduced in Cont-CM+IGF2 and Mat-CM compared to EBM-2+bFGF (Basal medium) or Cont-CM (FIG. 8a). Quantitative evaluation was performed using ImageJ to verify the increase in the difference in area (FIG. 8b).

### Example 7. Verification of transplantation efficacy of mature hIO-derived mesenchymal stromal cells

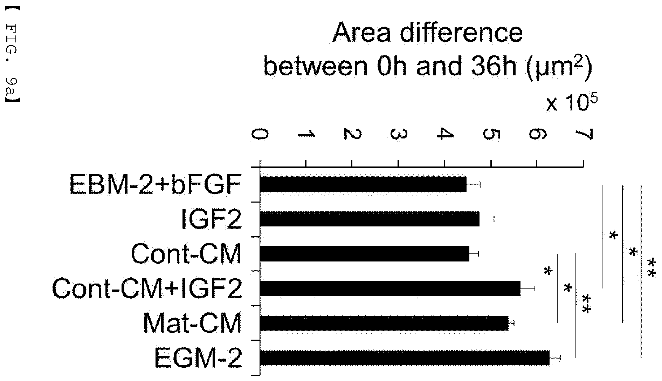

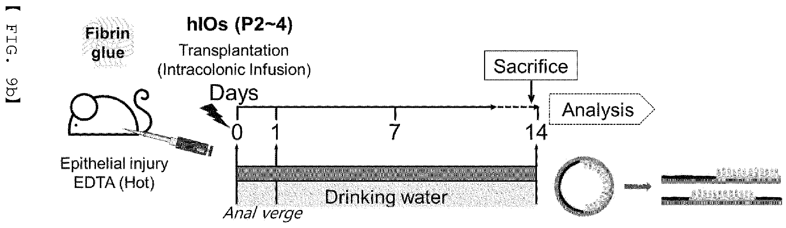

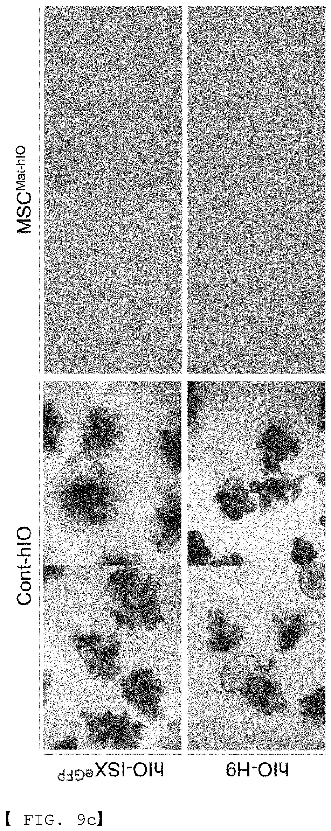

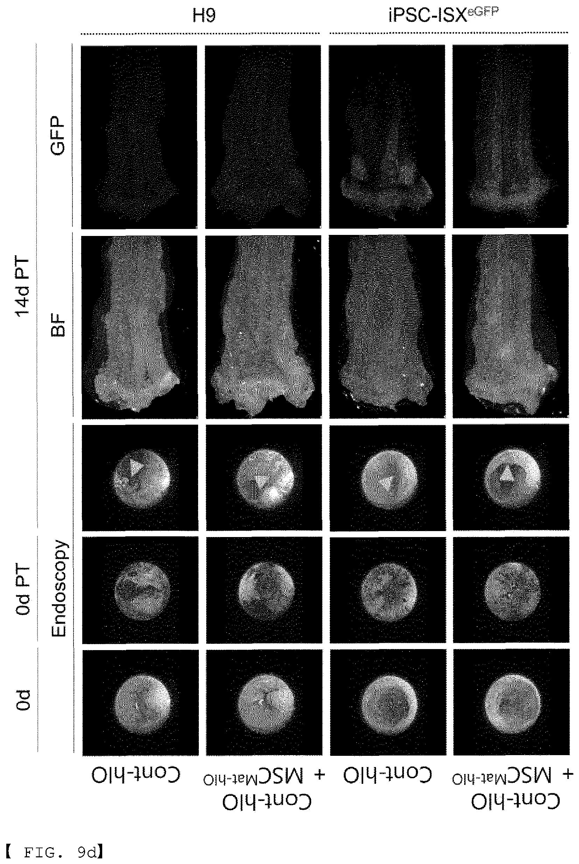

The expression of vascular endothelial factors by Mat-hIO-derived MSCs may aid in engraftment upon transplantation. Regarding this, the transplantation efficacy of MSC^{Mat-hIO} was investigated through co-transplantation of MSC^{Mat-hIO} and Cont-hIO derived from immature or mature intestinal organoids (FIGS. 9a and 9b). Cont-hIO single transplantation and co-transplantation with MSCMat-hIO were performed in an intestinal epithelial injury model of immunodeficient mice, and the degree of epithelial damage and cell engraftment were confirmed through colonoscopy and dissecting fluorescence microscopy. The Cont-hIO single transplantation group showed some bleeding and fragile intestinal mucosa, but the co-transplantation group showed faster recovery of epithelial injury at 2 weeks and a higher engraftment rate of reporter organoids compared to the single transplantation group. Histological analysis (H&E) and functional analysis (AB-PAS) showed that the epithelial structure was restored and that the mature goblet cells actively secreted mucin (FIGS. 9c and 9d).

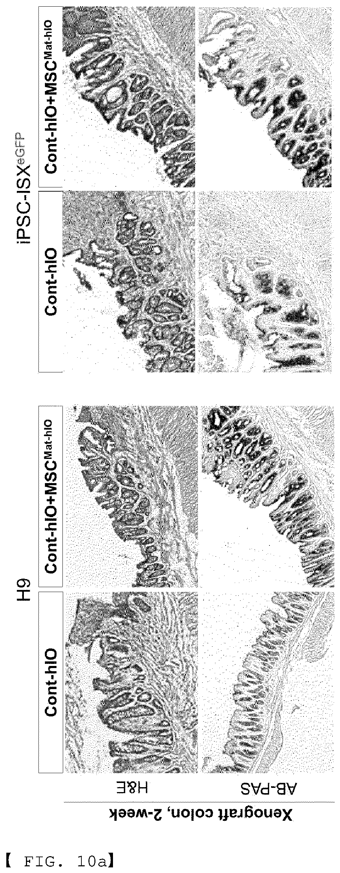

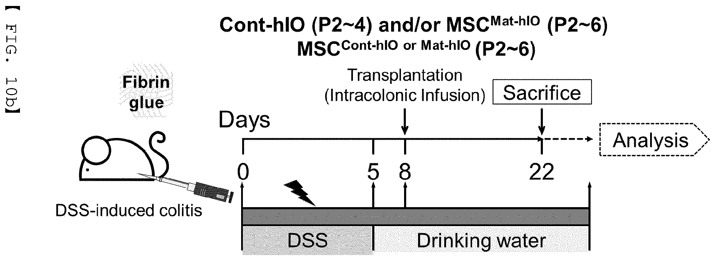

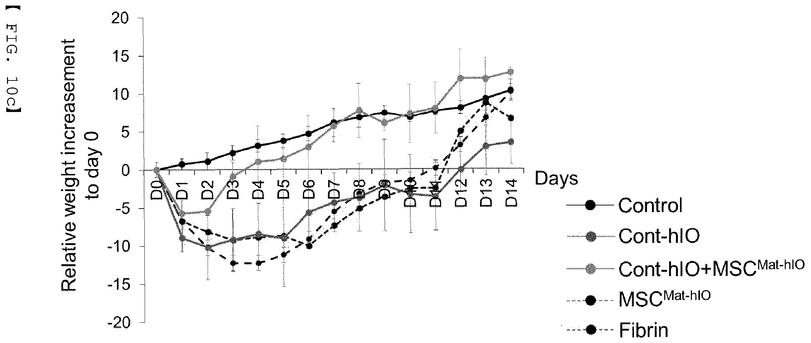

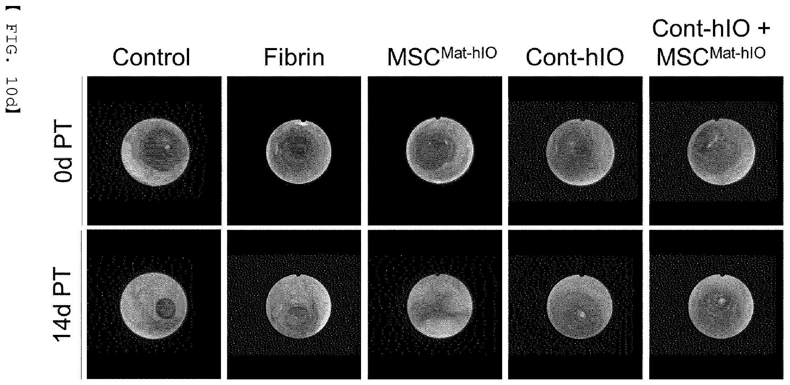

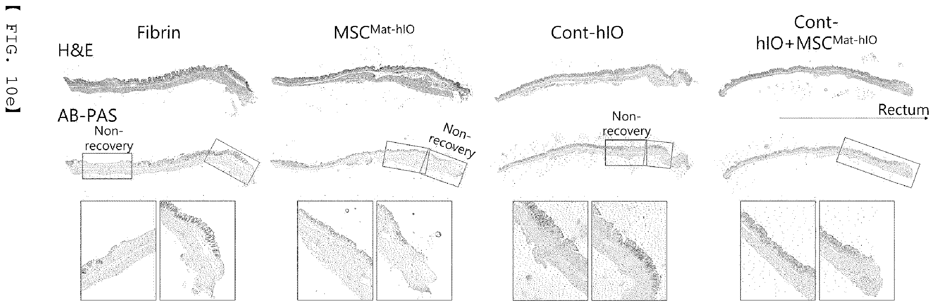

MSC^{Cont-hIO}, Cont-hIO single transplantation, and Cont-hIO+ MSC^{Cont-hIO} co-transplantation groups were compared in a DSS-induced inflammatory bowel disease model (FIG. 10a). The weight gain in the co-transplantation group was rapidly increased starting from day 3 (FIG. 10b). In addition, colonoscopy showed that there was no redness and inflammation in the co-transplantation group at week 2 (FIG. 10c). The co-transplantation group showed a decrease in intestinal epithelial injury and regeneration throughout the intestinal epithelium through histological analysis, and a significant recovery of Mucin functionality through AB-PAS staining (FIG. 10d). In addition, the crypt depth of the damaged (injured) region was also confirmed to be significantly increased in the co-transplantation group (FIG. 10e).

This confirmed that MSC^{Mat-hIO} contributed to the healing of intestinal epithelial injury more effectively than Cont-hIO single transplantation group.

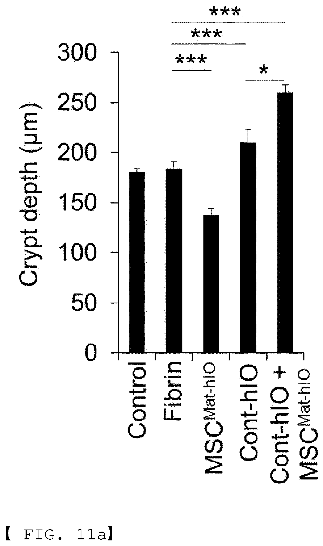

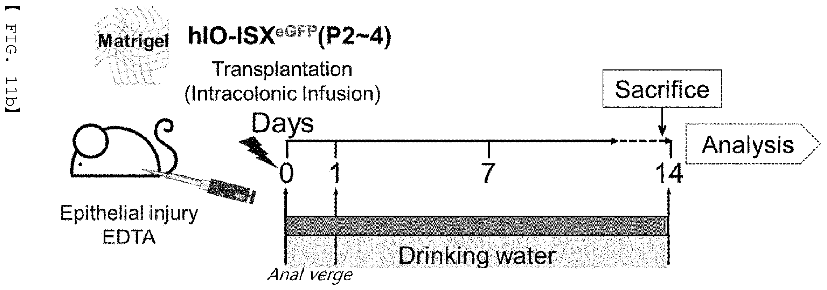

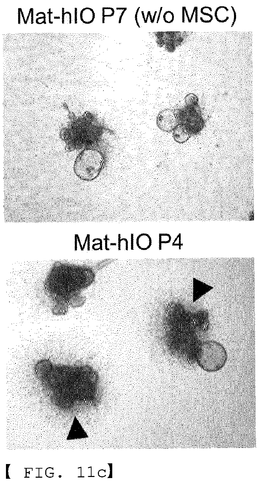

Further, it was shown that the recovery rate of epithelial injury after transplantation was different depending on the presence or absence of MSCs even in the same Mat-hIO (FIG. 11). Specifically, Mat-hIO passage culture confirmed that the group transplanted with P7, where MSC^{Mat-hIO} was barely present, showed a decrease in the crypt depth of the damaged (injured) region compared to Mat-hIO (P4).

Considering these results, mesenchymal stromal cells (MSCMat-hIO) present around mature intestinal organoids were identified as playing an important role in the engraftment and tissue improvement of intestinal organoids.

### Example 8. Universal application of mature hIO-derived mesenchymal stromal cells

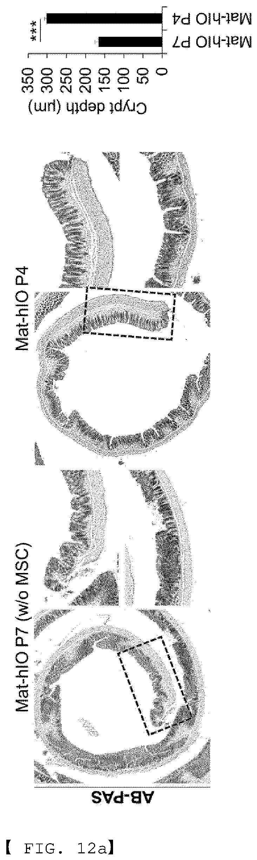

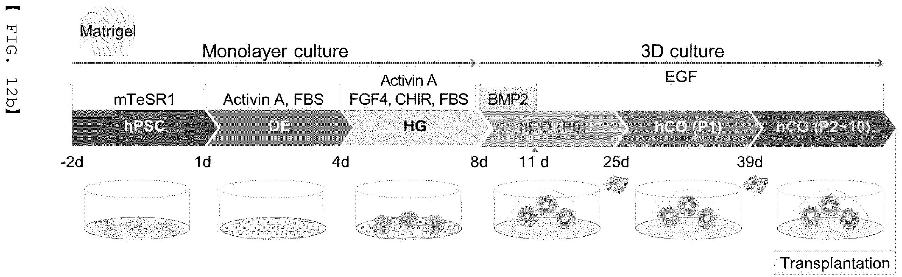

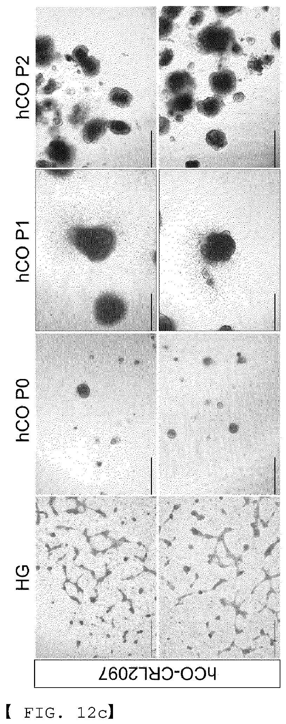

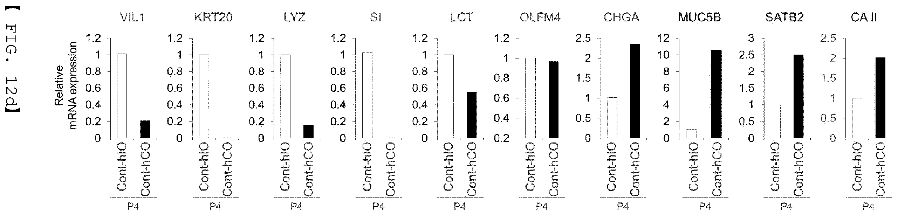

To evaluate the universal application of MSC^{Mat-hIO}, previously published induced pluripotent stem cell-derived colon organoids were produced (FIG. 12a). Compared to conventional intestinal organoids (Cont-hIO), the produced colon organoids (hCO) are characterized by fewer budding structures and reduced expression of representative small intestine markers (VIL1, KRT20, LYZ, SI, and LCT). On the other hand, some increase in CHGA, an enteroendocrine marker, and increased expression of MUC5B, SATB2, and CAII, which are representative characteristics of hCO, were identified (FIGS. 12b and 12c). The hCO and MSC^{Mat-hIO} produced in this way were transplanted into immunodeficient mice with DSS-induced inflammatory bowel disease to verify their efficacy as regenerative therapeutics for inflammatory diseases (FIG. 12d). Examination of intestinal inflammation level via colonoscopy showed that the fibrin control group and the hCO single transplantation group still had bleeding spots, whereas the hCO+MSC^{Mat-hIO} transplantation group and the MSC^{Mat-hIO} single transplantation group had recovered intestinal inflammation (FIG. 12e). Finally, histopathological examination using H&E and intestinal function test using AB-PAS showed that the crypt depth was significantly improved and the mucin layer was well formed in the hCO+MSC^{Mat-hIO} transplantation group (FIG. 12f).

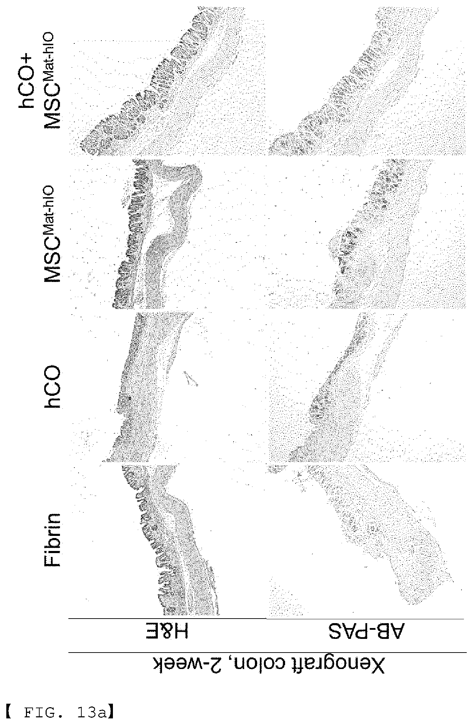

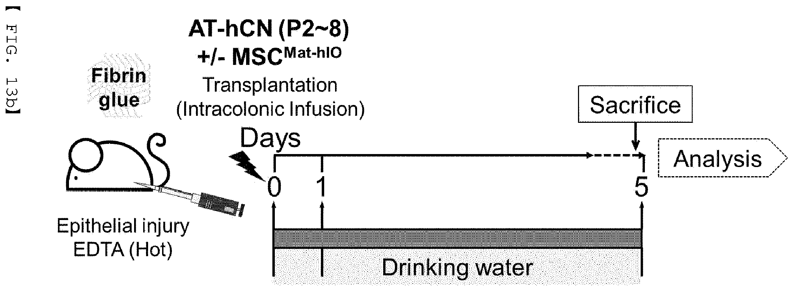

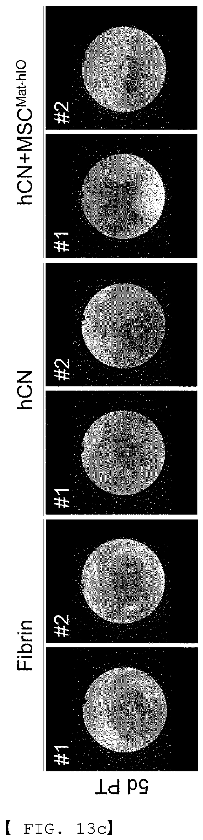

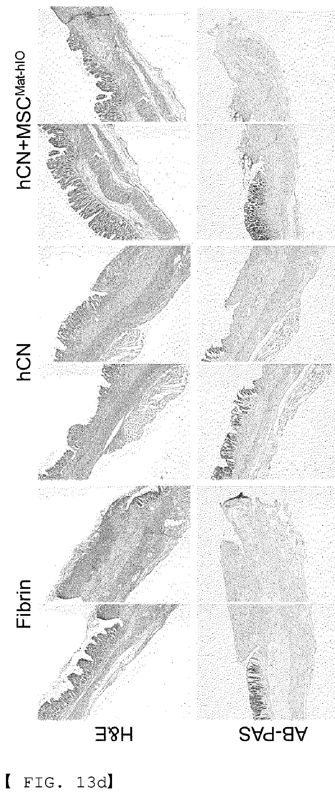

Human adult stem cell-derived colon organoids (hCN) single transplantation and MSC^{Mat-hIO} co-transplantation were performed in immunodeficient mice with EDTA-induced intestinal epithelial injury and the mice were observed at day 5 after transplantation (FIG. 13a). To confirm the effect of MSCs on early engraftment, colonoscopy was performed on day 5 after transplantation and showed that the co-transplantation group had a significant reduction in inflammation caused by epithelial injury and the degree of injury (FIG. 13b). Histological analysis also confirmed that epithelial loss due to rectal wounds still remained in the Fibrin and hCN groups, but the hCN+MSC^{Mat-hIO} transplantation group showed rapid epithelial recovery and functional mucin secretion (FIG. 13c). Immunofluorescence staining with a human-specific antibody (hECAD) directly confirmed the distribution of human cells, confirming a significant increase relative to nuclei (DAPI) in the co-transplantation group compared to the control group and the single transplantation group (FIG. 13d).

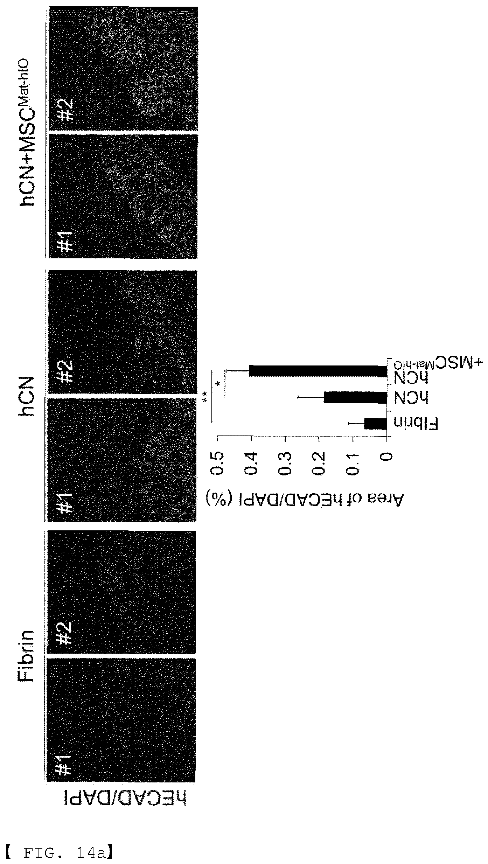

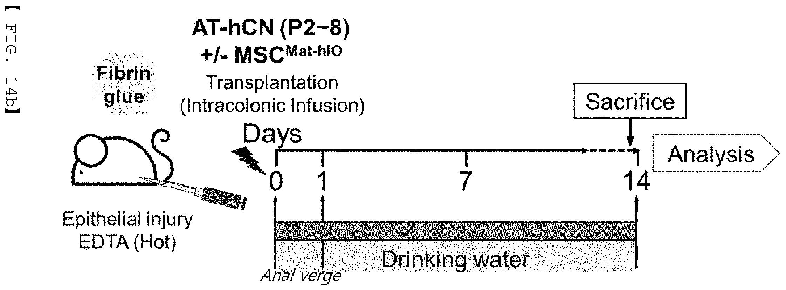

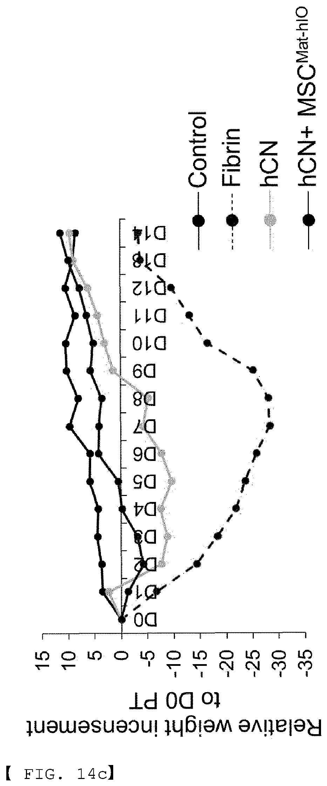

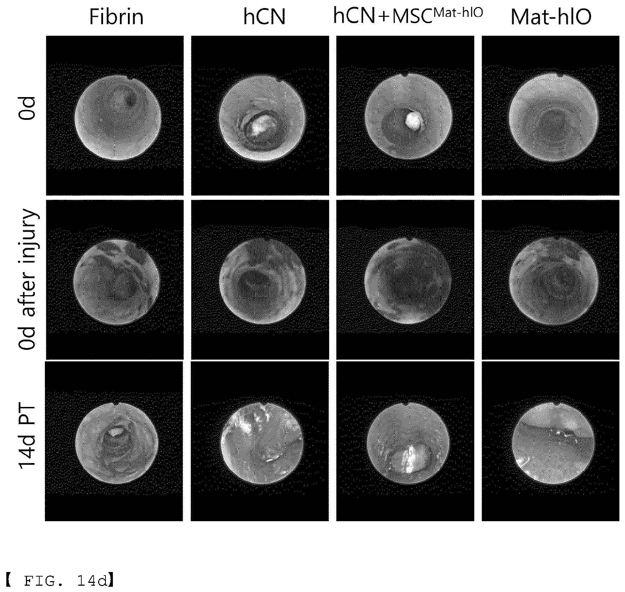

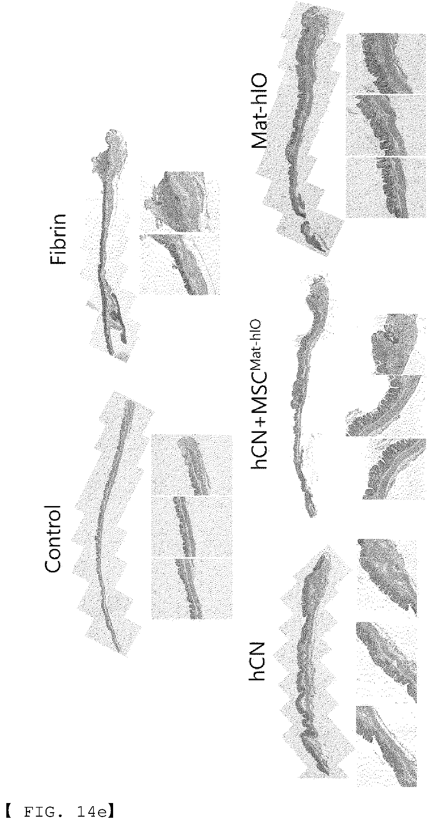

In the above intestinal epithelial injury model, the Fibrin, hCN single transplantation, and hCN+MSC^{Mat-hIO} co-transplantation groups were compared at week 2, and the results showed that there was no significant difference in body weight and colonoscopy at week 2, histological analysis showed that the intestinal epithelial structure in the rectal area was recovered, and AB-PAS staining showed that the co-transplantation group had more active mucin secretion (FIG. 14).

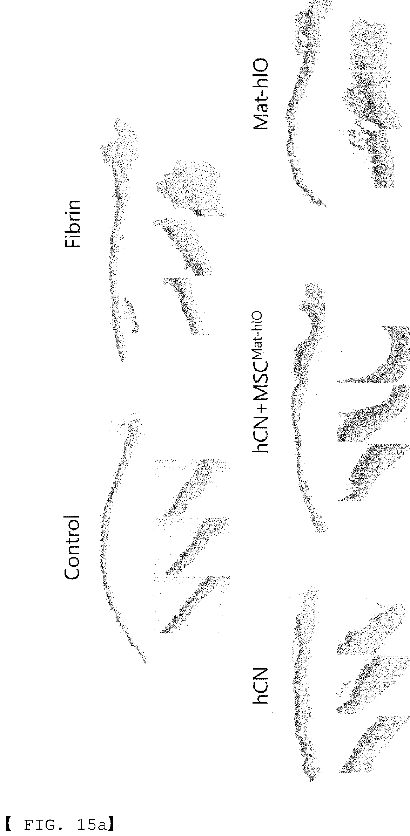

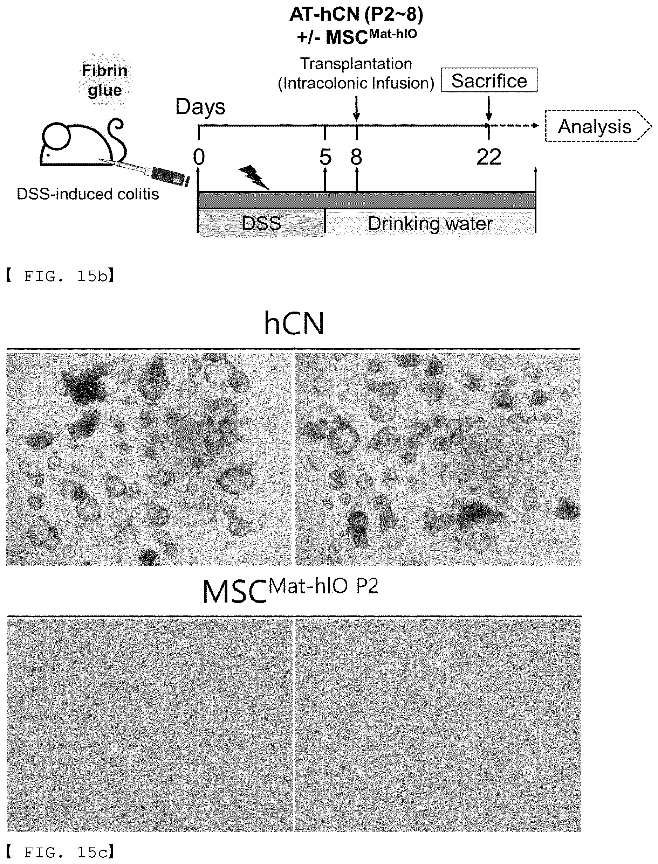

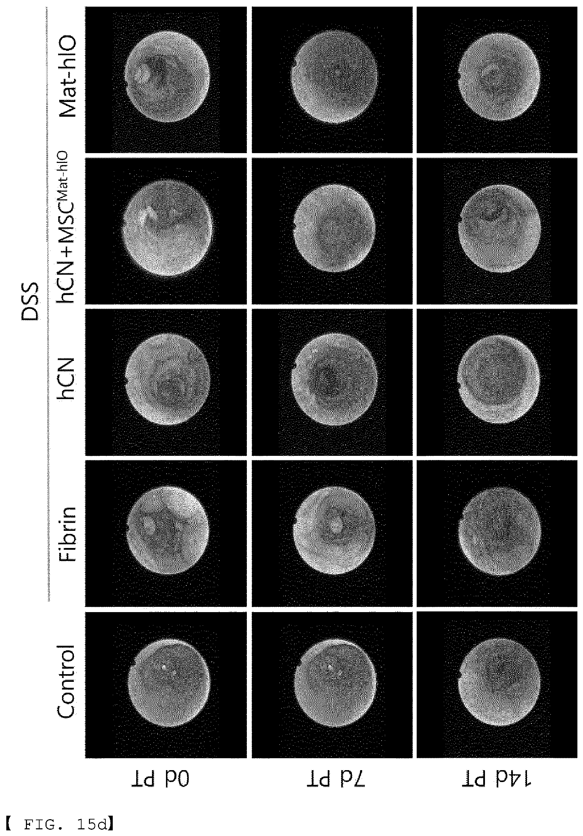

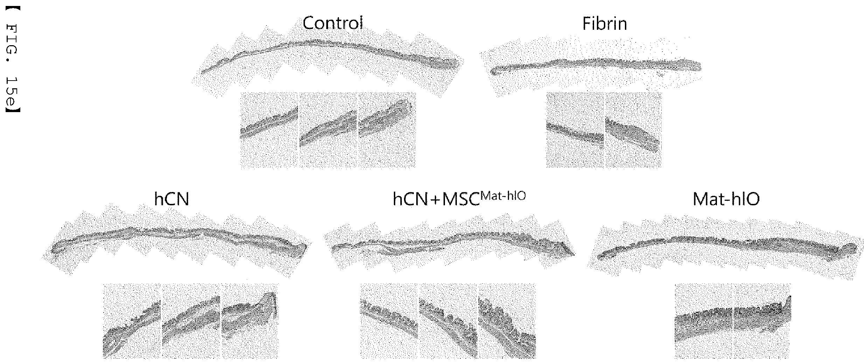

In the DSS-induced inflammatory bowel disease model, colonoscopy was performed at week 2 in the Fibrin, hCN single transplantation and MSC^{Mat-hIO} co-transplantation groups, and the results showed that the inflammation level and the degree of redness were reduced in both single transplantation and co-transplantation groups, and histological analysis confirmed that similar levels of engraftment and regeneration occurred throughout the intestine, indicating evenly secreted mucin (FIG. 15).

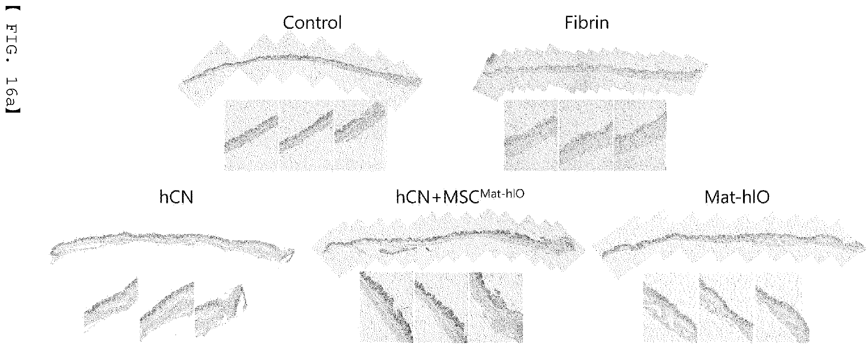

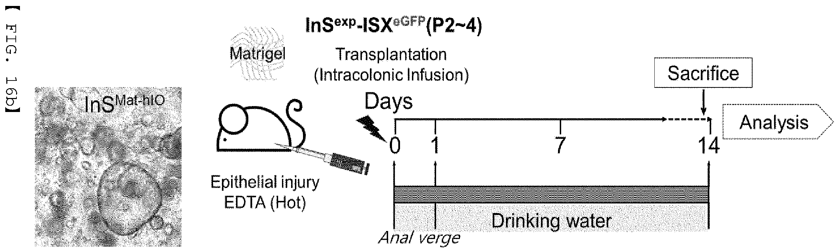

In the EDTA-induced intestinal epithelial damage immunodeficient mouse model, 3D intestinal stem cell (InS^{hIO}) single transplantation and MSC^{Mat-hIO} co-transplantation were compared (FIG. 16a). As a result, as in the previous results, compared to the single transplantation group, the co-transplantation group showed faster recovery of intestinal crypt depth and intestinal epithelial structure, and almost normal mucin secretion of mature goblet cells, as confirmed in both Swiss-roll sections and longitudinal sections (FIG. 16b).

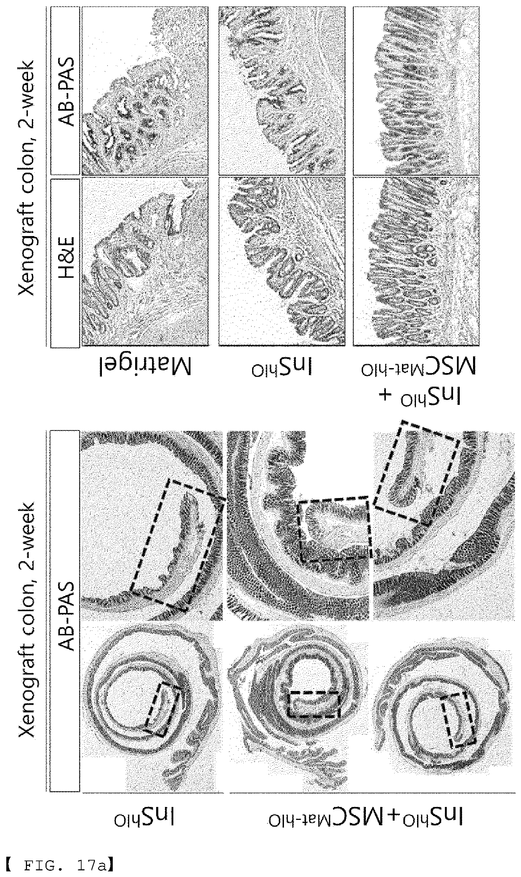

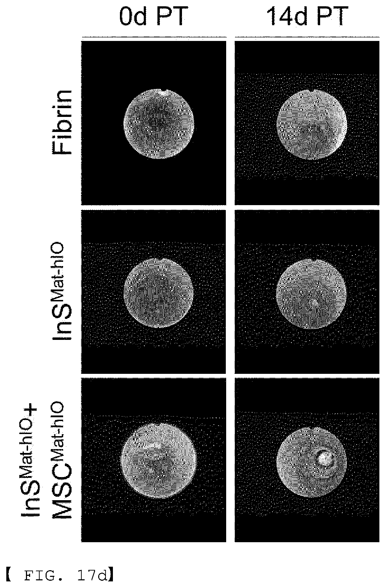

In addition, in the inflammatory bowel disease model, the InS^{Mat-hIO}+MSC^{Mat-hIO} co-transplantation group showed faster recovery above the normal control group 7 days after transplantation through body weight changes compared to the InS^{Mat-hIO} single transplantation group, and colonoscopy showed that the inflammation level of the intestinal epithelium was significantly reduced to normal levels (FIGS. 17a to 17c). Histological analysis also showed that the co-transplantation group had recovered epithelial structure throughout the intestine and smooth secretion of mucin compared to the control group and the single transplantation groups (FIG. 17d).

### Example 9. Confirmation of characteristics of mature hIO

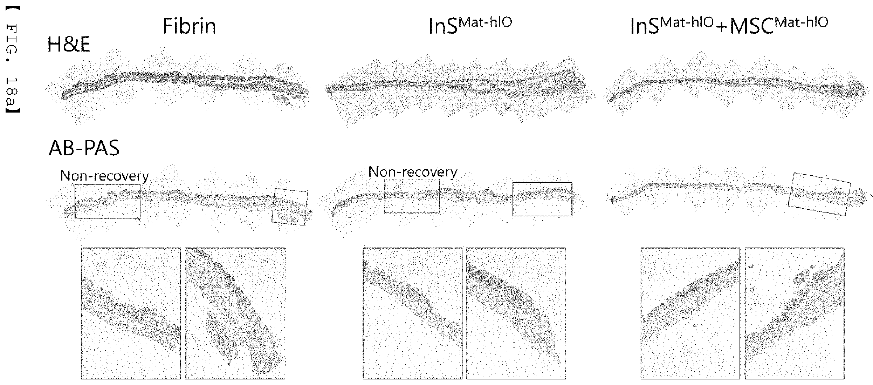

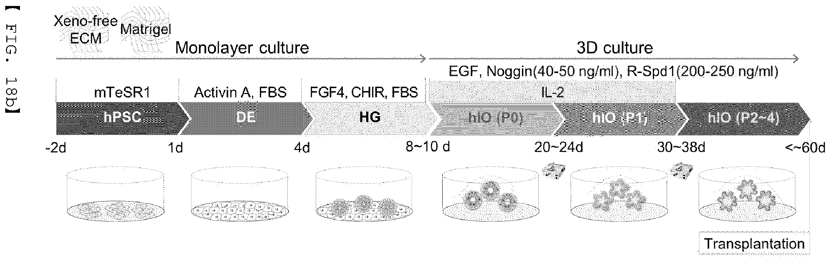

As described above, hIOs were produced from hPSCs containing hESC lines or non-integrating-hiPSC lines, using the hIO differentiation protocol. Then, the culture process of mature and immature organoids produced by treatment with 1 ng/ml rhIL-2 from P0 was shown as a schematic diagram (FIG. 18a). Embryonic stem cell- or induced pluripotent stem cell-derived immature and high-performance (mature) intestinal organoids were produced and cell characteristics thereof were observed (FIG. 18b), and expression levels of small intestine-specific genes were compared with those of conventional mature intestinal organoids.

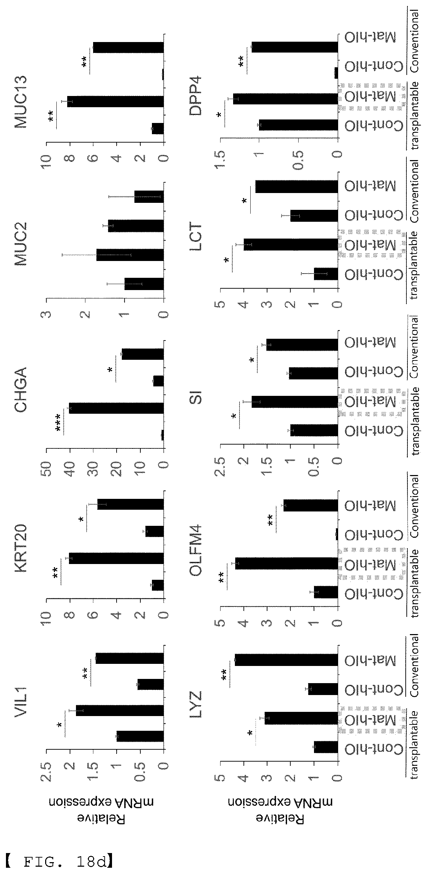

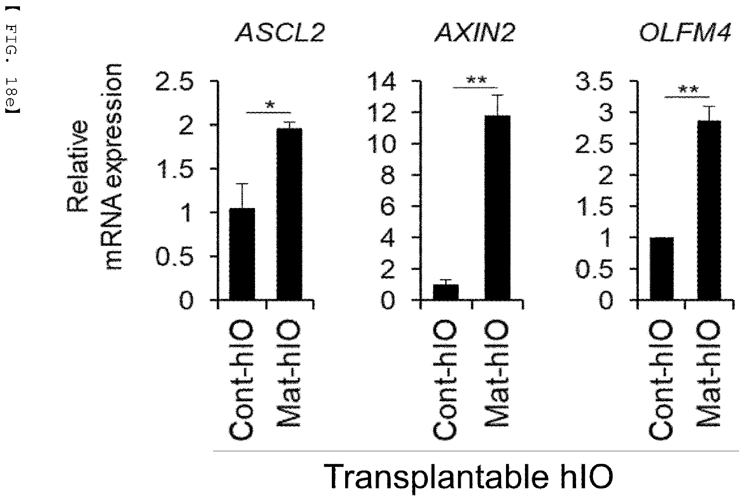

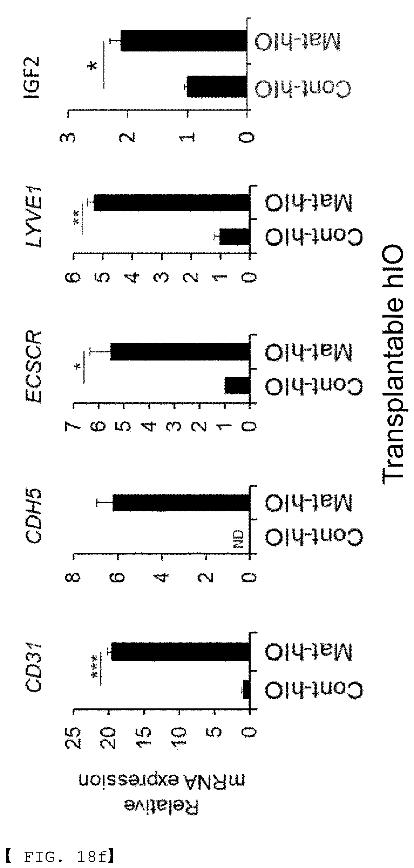

The results showed high expression levels of intestinal cell type-specific markers (VIL1, KRT20, CHGA, MUC2, and LYZ) and intestinal maturation markers (MUC13, OLFM4, SI, LCT, and DPP4 and LCT) (FIG. 18c). In addition, it was confirmed that intestinal stem cell-specific markers (ASCL2, AXIN2, LGR5, and OLFM4) and vascular endothelial-related factors (CD31, CDH5, ECSCR, LYVE1, and IGF2) were highly expressed in high-performance intestinal organoids for transplantation compared to immature organoids (FIGS. 18d and 18e), and that these markers were also highly expressed in high-performance intestinal organoids for transplantation compared to conventional mature organoids (FIG. 18f).

### Example 10. Confirmation of maintenance of characteristics of mature hIO when applying xeno-free ECM

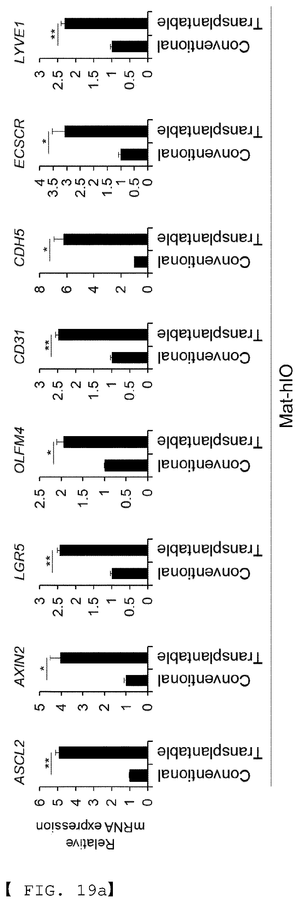

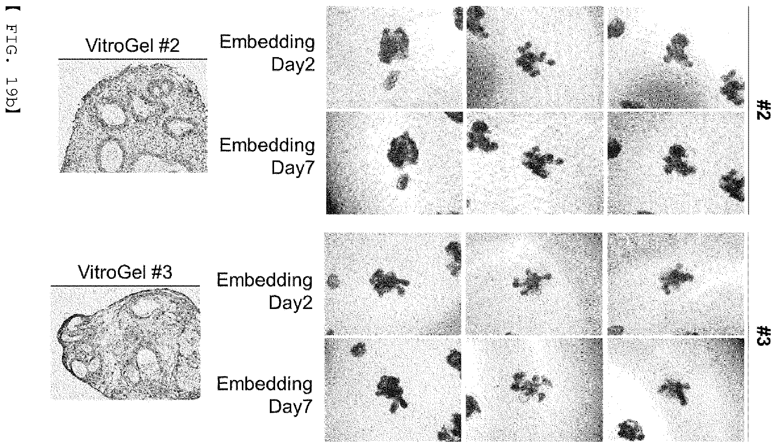

The differentiation protocol presented above was used to perform differentiation from embryonic stem cells and induced pluripotent stem cells into endoderm (DE) and hindgut (HG) on dishes coated with Matrigel and ECMatrix^{™}-511, and it was confirmed that the structure of the intestinal epithelium was maintained in Vitrogel #2 and #3 types, which were proposed to replace Matrigel for 3D culture (FIG. 19a). Matrigel, Vitrogel #2, and Vitrogel #3 showed some marker-specific differences in the expression of small intestine-specific markers (CDX2, VIL1, LGR5, ASCL2, LYZ, MUC2, and MUC13), but the expression of all markers remained higher than that of undifferentiated pluripotent stem cells, confirming that the differentiation into intestinal organoids using clinically applicable ECMatrix^{™}-511 and Vitrogel, is the same as that using Matrigel.

### Example 11. Comparison of post-transplantation characteristics between immature and high-performance mature hIOs via subrenal transplantation

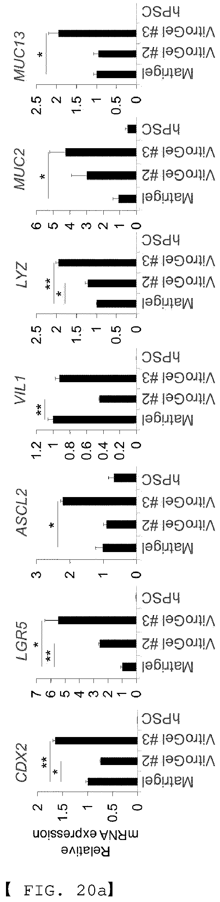

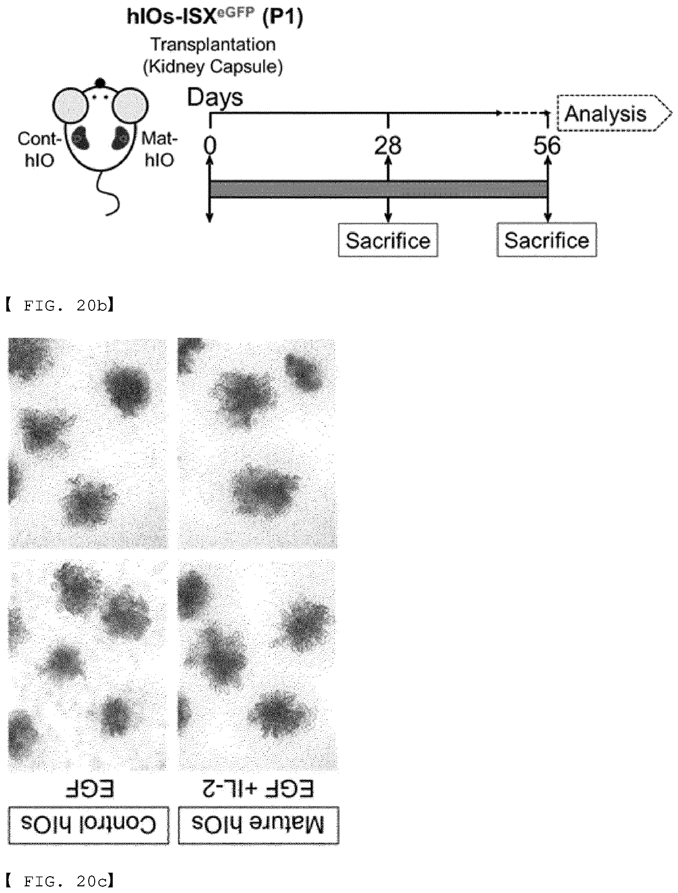

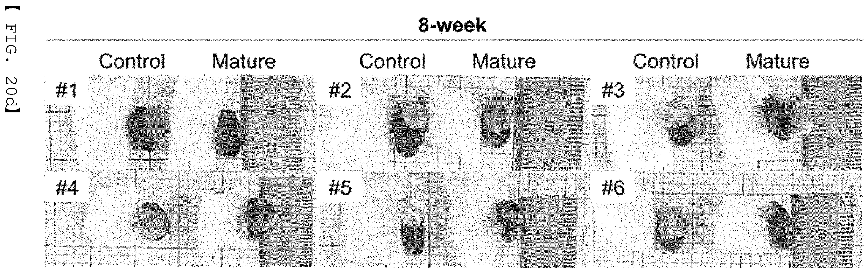

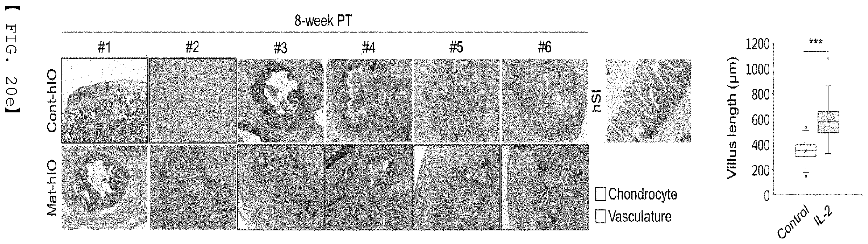

To minimize differences between mice after transplantation, Cont-hIO and Mat-hIO were transplanted into both kidneys of the same immunodeficient mouse and observed after 4 and 8 weeks, respectively (FIGS. 20a and 20b). After 8 weeks, the xenografts from both kidneys were excised and observed, and prominent vascularization was confirmed around the mature organoid xenografts compared to the immature organoids (FIG. 20c). This was supported by histological analysis using H&E, which showed that the blood vessels formed around the transplanted intestinal organoids and the villus length of the intestinal organoid structure developed similarly to that of adult human small intestine tissue (FIG. 20d). The expression of vascular markers (hVE-Cad, PECAM-1, and VEGF) using immunofluorescent staining confirmed the vascularization and structure around the xenograft organoid tissue and validated the development of the villus structure (hECAD) and mesenchymal stromal cells (αSMA) of the intestinal organoids in vivo (FIG. 20e). The above results showed that vascularization was significantly enhanced and the development of the Villus structure (hECAD) of intestinal organoids and mesenchymal stromal cells and pericytes (αSMA) was also excellent by the peristromal cell layer present around mature intestinal organoid.

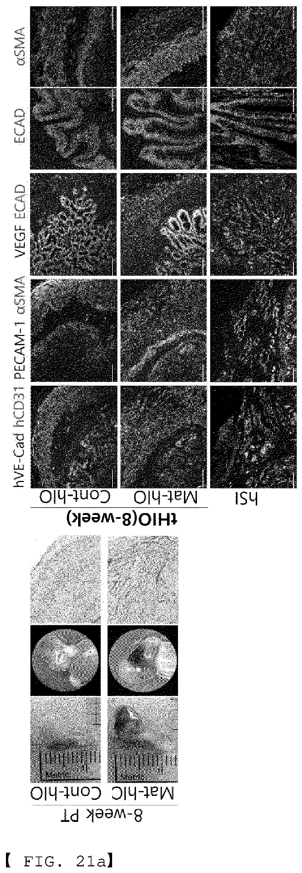

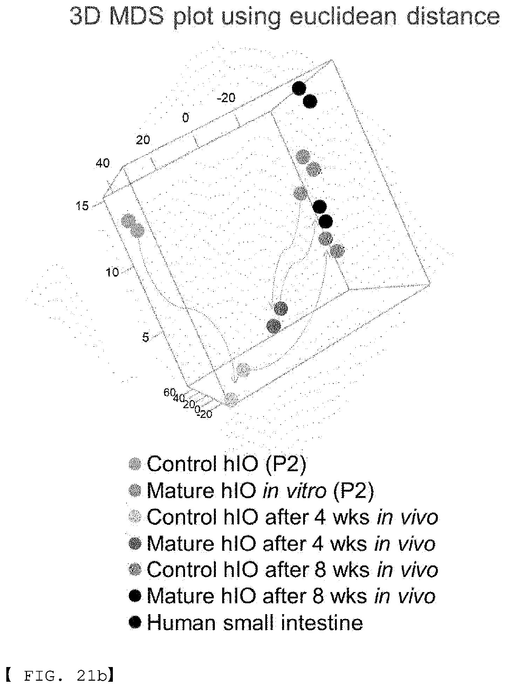

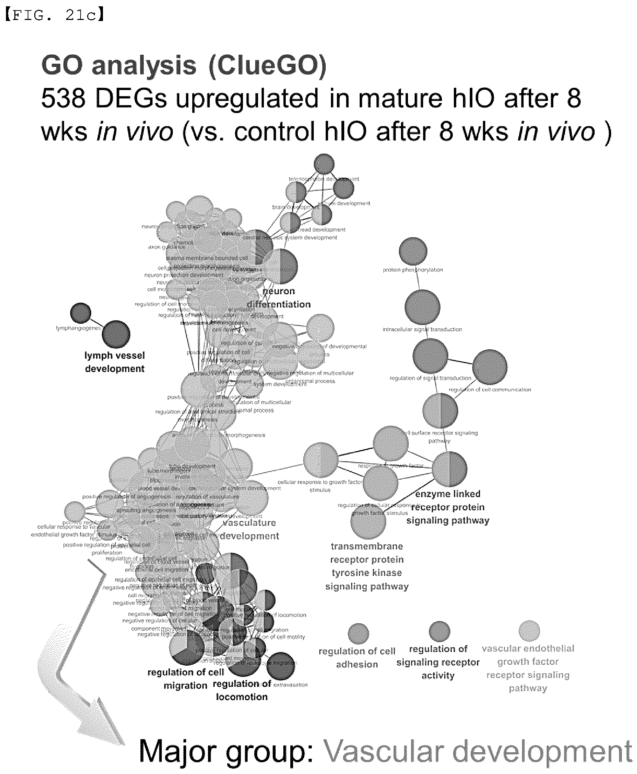

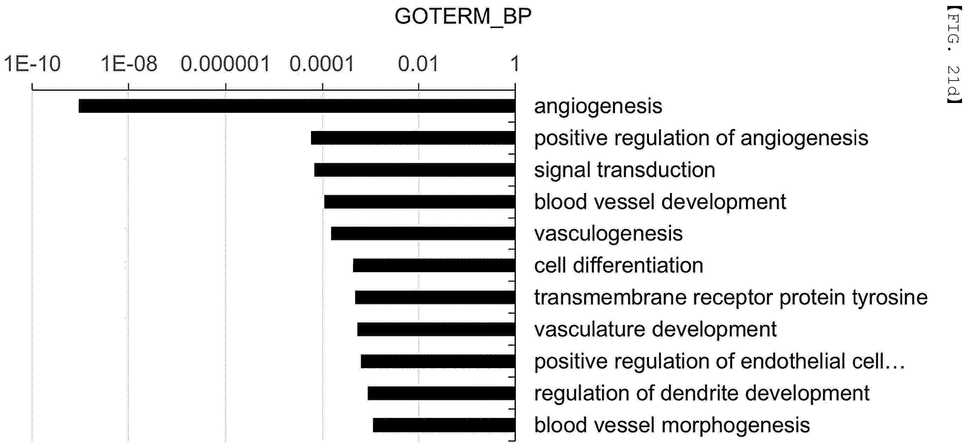

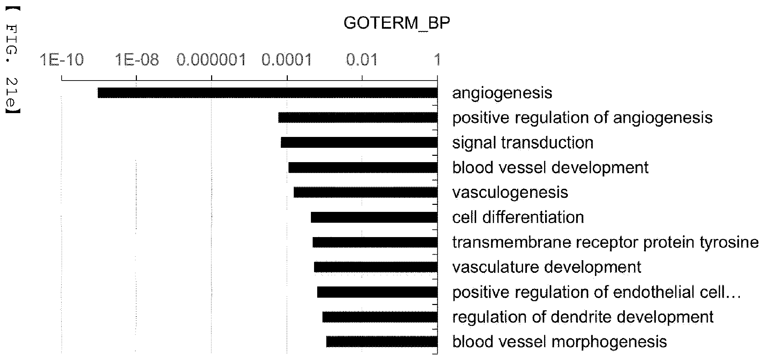

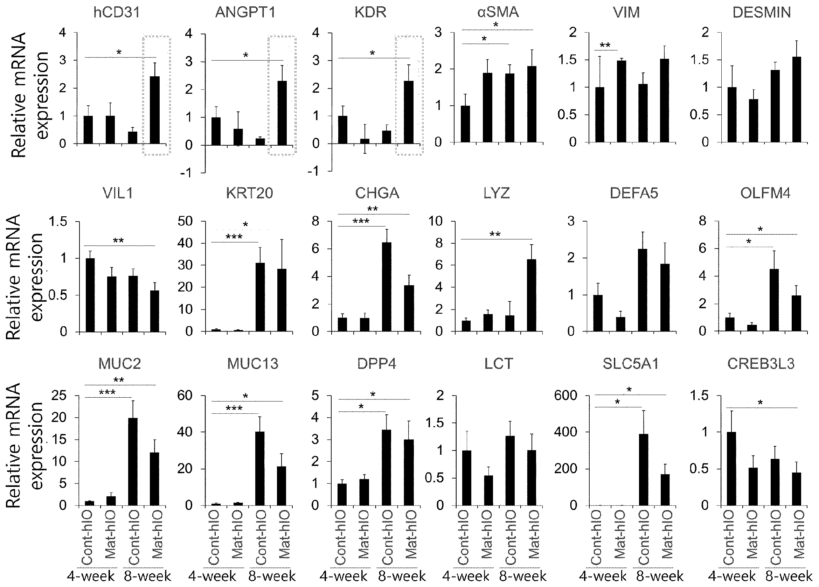

Further, genomic analysis via RNAseq confirmed the previously known maturation pathway of intestinal organoids upon subrenal transplantation by comparing immature intestinal organoids, 4- and 8-week xenograft intestinal organoids, ex vivo mature intestinal organoids, and human small intestinal tissue (FIG. 21a). In particular, GO analysis of 538 DEGs that were different between the Cont-hIO transplantation group and the Mat-hIO transplantation group 8 weeks after transplantation identified that vascular development was the major group (FIGS. 21b to 21d). In the RNAseq genome analysis results, qPCR showed that vascular markers (hCD31, ANGPT1, and KDR) were increased in the 8-week Mat-hIO xenograft group, and the mesenchymal stromal cell marker and pericyte marker (αSMA) was increased in the 4-week Mat-hIO xenograft group, as compared to Cont-hIO (FIG. 21e).

## Claims

1. A mesenchymal stromal cell derived from a stromal cell layer around a mature intestinal organoid, exhibiting at least one characteristic selected from the following:
(a) an enhanced expression level of hCD31+ compared to adult-derived mesenchymal stem cells, pluripotent stem cell-derived mesenchymal stem cells and/or immature intestinal organoid-derived mesenchymal stromal cells;
(b) enhanced expression levels of factors related to vascular development, mesenchyme development, epithelial cell proliferation, and/or muscle tissue development, compared to adult-derived mesenchymal stem cells, pluripotent stem cell-derived mesenchymal stem cells, and/or immature intestinal organoid-derived mesenchymal stromal cells;
(c) enhanced expression levels of endothelial cell types, compared to adult-derived mesenchymal stem cells, pluripotent stem cell-derived mesenchymal stem cells, and/or immature intestinal organoid-derived mesenchymal stromal cells;
(d) enhanced expression levels of endothelial cell characteristic factors on gene ontology (GO) term enrichment, compared to adult-derived mesenchymal stem cells, pluripotent stem cell-derived mesenchymal stem cells, and/or immature intestinal organoid-derived mesenchymal stromal cells;
(e) enhanced expression levels of angiogenic factors, vascular endothelial-related factors and/or vascular secretory factors, compared to adult-derived mesenchymal stem cells, pluripotent stem cell-derived mesenchymal stem cells, and/or immature intestinal organoid-derived mesenchymal stromal cells; and
(f) maintained expression levels of any one or more selected from the group consisting of αSMA, VIM and DESMIN at the time of subculture.

2. The mesenchymal stromal cell derived from a stromal cell layer around a mature intestinal organoid of claim 1, wherein the mesenchymal stromal cell derived from the stromal cell layer around the mature intestinal organoid exhibits
an enhanced expression level of hCD31+ compared to adult-derived mesenchymal stem cells, pluripotent stem cell-derived mesenchymal stem cells and/or immature intestinal organoid-derived mesenchymal stromal cells; and
enhanced expression levels of angiogenic factors, vascular endothelial-related factors and/or vascular secretory factors, compared to adult-derived mesenchymal stem cells, pluripotent stem cell-derived mesenchymal stem cells, and/or immature intestinal organoid-derived mesenchymal stromal cells.

3. The mesenchymal stromal cell derived from a stromal cell layer around a mature intestinal organoid of claim 2, wherein
the mesenchymal stromal cell derived from the stromal cell layer around the mature intestinal organoid further exhibits
enhanced expression levels of factors related to vascular development, mesenchyme development, epithelial cell proliferation, and muscle tissue development, compared to adult-derived mesenchymal stem cells, pluripotent stem cell-derived mesenchymal stem cells, and immature intestinal organoid-derived mesenchymal stromal cells.

4. The mesenchymal stromal cell derived from a stromal cell layer around a mature intestinal organoid of claim 1, wherein
the angiogenic factor, and/or vascular endothelial-related factor is any one or more selected from the group consisting of hCD31, CDH5, ECSCR, LYVE1, IGF2, VEGF, ANGPT1, and KDR.

5. The mesenchymal stromal cell derived from a stromal cell layer around a mature intestinal organoid of claim 1, wherein
the vascular secretory factor includes Angiogenin, DKK1, EGF, Osteopontin, ICAM-1, IGFBP-3, VEGF, VCAM-1, MCP-1, and Activin A.

6. The mesenchymal stromal cell derived from a stromal cell layer around a mature intestinal organoid of claim 1, wherein
the immature intestinal organoid-derived mesenchymal stromal cell is a mesenchymal stromal cell derived from an intestinal organoid that do not contain, as a medium component, any one or more cytokines selected from the group consisting of IL-2, IL-22, IL-6, IL-1β, IL-11, EGF, OSM and IL-10, NRG-1, or colivelin, an activator of STAT3 and mTOR signaling pathway.

7. The mesenchymal stromal cell derived from a stromal cell layer around a mature intestinal organoid of claim 1, wherein
the mature intestinal organoid contains, as a medium component, any one or more cytokines selected from the group consisting of IL-2, IL-22, IL-6, IL-1β, IL-11, EGF, OSM and IL-10, NRG-1, or colivelin, an activator of STAT3 and mTOR signaling pathway, in differentiation from a hindgut into the intestinal organoid.

8. The mesenchymal stromal cell derived from a stromal cell layer around a mature intestinal organoid of claim 7, wherein the cytokine is IL-2.

9. The mesenchymal stromal cell derived from a stromal cell layer around a mature intestinal organoid of claim 1, wherein
the mesenchymal stromal cell derived from the stromal cell layer around the mature intestinal organoid is cultured in medium containing B27, EGF, or both.

10. A method for culturing a mesenchymal stromal cell derived from a stromal cell layer around a mature intestinal organoid differentiated from a pluripotent stem cell (PSC), comprising the following steps:
(a) isolating a mature intestinal organoid into single cells or small cell clumps;
(b) seeding the isolated single cells or the small cell clumps in a culture medium coated with any one selected from the group consisting of gelatin, fibrin, and collagen I; and
(c) obtaining only cells that are adhered and cultured from the seeded cells.

11. The method of claim 10, further comprising, after Step (c), (d) subculturing the adhered and cultured cells.

12. The method of claim 10, wherein the culture medium in Step (b) is a culture medium containing B27, EGF, IL-2 or any combination thereof.

13. The method of claim 10, wherein the mature intestinal organoid is produced through the following steps:
1) differentiating pluripotent stem cells into definitive endoderm cells by treatment with any one or more selected from the group consisting of Nodal, Activin A, Activin B, BMP4, and Wnt3a;
2) differentiating the definitive endoderm cells into a hindgut by treatment with FGF and Wnt ligands; and
3) maturing the intestinal organoid by treating the hindgut with a BMP inhibitor, a Wnt agonist, a receptor tyrosine kinase ligand, and cytokine secreted by T-lymphocyte.

14. The method of claim 13, wherein
Step 1) comprises treating embryonic stem cells or induced pluripotent stem cells with Activin A to perform differentiation into definitive endoderm cells.

15. The method of claim 13, wherein
Step 2) comprises differentiating the definitive endoderm cells into a hindgut by treatment with FGF and CHIR99021.

16. The method of claim 13, wherein
Step 3) comprises maturing the intestinal organoid by treating the hindgut with Noggin as a BMP inhibitor, any one or more Wnt agonists selected from the group consisting of R-spondin 1, R-spondin 2, R-spondin 3, and R-spondin 4, an epidermal growth factor (EGF) as a receptor tyrosine kinase ligand, and any one or more cytokines selected from the group consisting of IL-2, IL-22, IL-6, IL-1β, IL-11, EGF, OSM, and IL-10 as cytokines secreted by T-lymphocytes, NRG-1, or colivelin, an activator of STAT3 and mTOR signaling pathway.

17. The method of claim 16, wherein
the cytokine secreted by T-lymphocyte is IL-2.

18. A mesenchymal stromal cell derived from a stromal cell layer around a mature intestinal organoid, produced by the method for culturing the mesenchymal stromal cell derived from the stromal cell layer around the mature intestinal organoid differentiated from the pluripotent stem cell (PSC) according to claim 10.

19. A pharmaceutical composition for preventing or treating an intestinal disease, comprising: the mesenchymal stromal cell derived from the stromal cell layer around the mature intestinal organoid according to any one of claims 1 to 9 and 18.

20. A pharmaceutical composition for assisting intestinal transplantation, comprising: the mesenchymal stromal cell derived from the stromal cell layer around the mature intestinal organoid according to any one of claims 1 to 9 and 18.

21. A pharmaceutical composition for preventing or treating an intestinal disease, comprising: the mesenchymal stromal cell derived from the stromal cell layer around the mature intestinal organoid according to any one of claims 1 to 9 and 18; and a small intestinal organoid, a large intestinal organoid, or an intestinal stem cell.

22. The pharmaceutical composition of claim 19, wherein the intestinal disease is any one or more selected from the group consisting of intestinal leakage syndrome, short bowel syndrome, irritable bowel syndrome, Crohn's disease, ulcerative colitis, intestinal Behcet's disease, infectious enteritis, ischemic bowel disease, and radiation enteritis.

23. The pharmaceutical composition of claim 21, wherein the intestinal disease is any one or more selected from the group consisting of intestinal leakage syndrome, short bowel syndrome, irritable bowel syndrome, Crohn's disease, ulcerative colitis, intestinal Behcet's disease, infectious enteritis, ischemic bowel disease, and radiation enteritis.

24. The pharmaceutical composition of claim 19, further comprising:
any one or more biodegradable supports selected from the group consisting of fibrin, collagen, gelatin, chitosan, alginate, hyaluronic acid, dextran, polylactic acid, poly(glycolic acid) (PGA), poly(lacticcoglycolic acid) (PLGA), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide) copolymers, copolymers thereof, and mixtures thereof.

25. The pharmaceutical composition of claim 20, further comprising:
any one or more biodegradable supports selected from the group consisting of fibrin, collagen, gelatin, chitosan, alginate, hyaluronic acid, dextran, polylactic acid, poly(glycolic acid) (PGA), poly(lacticcoglycolic acid) (PLGA), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide) copolymers, copolymers thereof, and mixtures thereof.

26. The pharmaceutical composition of claim 21, further comprising:
any one or more biodegradable supports selected from the group consisting of fibrin, collagen, gelatin, chitosan, alginate, hyaluronic acid, dextran, polylactic acid, poly(glycolic acid) (PGA), poly(lacticcoglycolic acid) (PLGA), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide) copolymers, copolymers thereof, and mixtures thereof.
